(19) Europäisches Patentamt
European Patent Office
Office européen des brevets

(11) EP 4 663 134 A1

(12) # EUROPEAN PATENT APPLICATION
published in accordance with Art. 153(4) EPC

(43) Date of publication:
**17.12.2025 Bulletin 2025/51**

(21) Application number: **24753461.3**

(22) Date of filing: **09.02.2024**

(51) International Patent Classification (IPC):
***A61B 10/00*** *(2006.01)* ***A61B 3/113*** *(2006.01)*
***A61B 5/00*** *(2006.01)* ***A61B 5/11*** *(2006.01)*
***A61B 5/107*** *(2006.01)* ***G06T 7/00*** *(2017.01)*
***G16H 50/20*** *(2018.01)*

(52) Cooperative Patent Classification (CPC):
**A61B 3/113; A61B 5/00; A61B 5/107; A61B 5/11; A61B 10/00; G06T 7/00; G16H 50/20**

(86) International application number:
**PCT/JP2024/004673**

(87) International publication number:
**WO 2024/167018 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.02.2023 JP 2023019491**

(71) Applicant: **PUBLIC UNIVERSITY CORPORATION YOKOHAMA CITY UNIVERSITY**
**Yokohama-shi, Kanagawa 236-0027 (JP)**

(72) Inventors:
- **OKADA, Kozo**
  **Yokohama-shi, Kanagawa 232-0024 (JP)**
- **ISHIHARA, Keiichiro**
  **Yokohama-shi, Kanagawa 236-0004 (JP)**
- **KOBAYASHI, Yusuke**
  **Yokohama-shi, Kanagawa 236-0004 (JP)**

(74) Representative: **Berggren Oy**
**P.O. Box 16**
**Fabianinkatu 21**
**00101 Helsinki (FI)**

(54) **HEART FAILURE ESTIMATION DEVICE, HEART FAILURE ESTIMATION SYSTEM, HEART FAILURE ESTIMATION METHOD, AND HEART FAILURE ESTIMATION PROGRAM**

(57) A heart failure estimation device acquires an image or video obtained by imaging the face of a patient. The heart failure estimation device extracts at least one feature parameter from the image or video. The heart failure estimation device estimates a degree of heart failure of the patient on the basis of the at least one feature parameter.

EP 4 663 134 A1

FIG.1

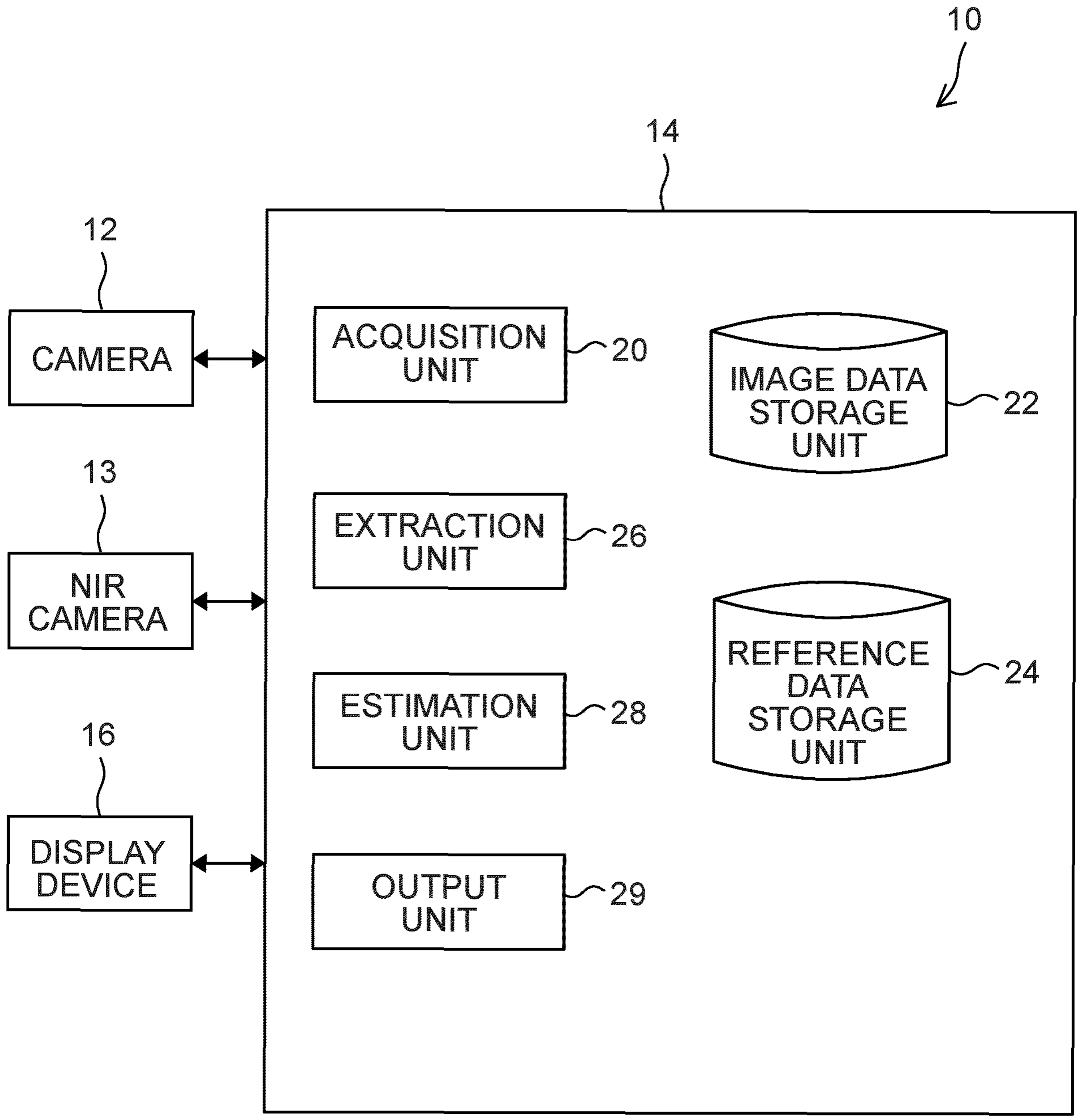
10
14
12
CAMERA
ACQUISITION UNIT
20
IMAGE DATA STORAGE UNIT
22
13
NIR CAMERA
EXTRACTION UNIT
26
REFERENCE DATA STORAGE UNIT
24
ESTIMATION UNIT
28
16
DISPLAY DEVICE
OUTPUT UNIT
29

## Description

TECHNICAL FIELD

The present disclosure relates to a heart failure estimation device, a heart failure estimation system, a heart failure estimation method, and a heart failure estimation program.

BACKGROUND ART

Heretofore, a system that automatically monitors congestive heart failure (CHF) of a patient has been known (for example, see Japanese Patent No. 5,281,002). This system receives signals generated by non-invasively monitoring the breathing and cardiac physiology of a patient, and automatically monitors CHF.

A heart failure progression judging system that judges the progression of heart failure in a patient is also known (for example, see Japanese Patent No. 6,901,042). Furthermore, a heart failure estimation system is known that may reduce the work of a healthcare worker in determining a stage of heart failure (for example, see Japanese Patent No. 6,893,002).

SUMMARY OF INVENTION

TECHNICAL PROBLEM

An object of the present disclosure is to provide a heart failure estimation device, a heart failure estimation system, a heart failure estimation method and a heart failure estimation program that may estimate a degree of heart failure of a patient from a still image or video image of a facial region of the patient.

SOLUTION TO PROBLEM

In order to achieve the object described above, a heart failure estimation device according to the present disclosure includes: an acquisition unit that acquires an image or video obtained by imaging the face of a patient; an extraction unit that extracts at least one feature parameter from the image or video acquired by the acquisition unit; an estimation unit that estimates a degree of heart failure of the patient on the basis of the at least one feature parameter extracted by the extraction unit; and an output unit that outputs a result estimated by the estimation unit.

ADVANTAGEOUS EFFECTS OF INVENTION

According to the present disclosure, an effect is provided in that a degree of heart failure of a patient may be estimated from an image or video of a facial region of the patient.

BRIEF DESCRIPTION OF DRAWINGS

Fig. 1 is a diagram showing an example of schematic structure of a heart failure estimation system according to a first exemplary embodiment.

Fig. 2 is a diagram for describing feature parameters.

Fig. 3 is a diagram for describing feature parameters.

Fig. 4 is a diagram for describing feature parameters.

Fig. 5 is a diagram for describing feature parameters.

Fig. 6-1A and Fig. 6-1B are diagrams for describing feature parameters.

Fig. 6-2 is a diagram for describing feature parameters.

Fig. 6-3 is a diagram for describing feature parameters.

Fig. 6-4 is a diagram for describing feature parameters.

Fig. 7 is a diagram for describing feature parameters.

Fig. 8 is a diagram for describing feature parameters.

Fig. 9 is a diagram for describing feature parameters.

Fig. 10 is a diagram for describing feature parameters.

Fig. 11-1 is a diagram for describing feature parameters.

Fig. 11-2 is a diagram for describing feature parameters.

Fig. 12 is a diagram for describing feature parameters.

Fig. 13 is a diagram for describing feature parameters.

Fig. 14 is a diagram schematically showing an example of a usage state of the heart failure estimation system according to the first exemplary embodiment.

Fig. 15 is a diagram showing an example of a computer constituting a heart failure estimation device.

Fig. 16 is a diagram for describing processing executed by the heart failure estimation system according to the present exemplary embodiment.

Fig. 17 is a diagram showing an example of schematic structures of a heart failure estimation system according to a second exemplary embodiment.

Fig. 18 is a diagram schematically showing an example of a usage state of the heart failure estimation system according to the second exemplary embodiment.

Fig. 19 is a diagram schematically showing an example of a usage state of the heart failure estimation system according to the second exemplary embodiment.

Fig. 20 is a diagram for describing Examples.

Fig. 21 is a diagram for describing the Examples.

Fig. 22-1 is a diagram for describing the Examples.

Fig. 22-2 is a diagram for describing the Examples.

Fig. 22-3 is a diagram for describing the Examples.

Fig. 22-4 is a diagram for describing the Examples.

Fig. 23 is a diagram for describing the Examples.

Fig. 24 is a diagram for describing the Examples.

Fig. 25 is a diagram for describing the Examples.

Fig. 26 is a diagram for describing the Examples.

Fig. 27-1 is a diagram for describing the Examples.

Fig. 27-2 is a diagram for describing the Examples.

Fig. 27-3 is a diagram for describing the Examples.

Fig. 27-4 is a diagram for describing the Examples.

Fig. 27-5 is a diagram for describing the Examples.

Fig. 27-6 is a diagram for describing the Examples.

Fig. 27-7 is a diagram for describing the Examples.

Fig. 27-8 is a diagram for describing the Examples.

Fig. 27-9 is a diagram for describing the Examples.

Fig. 27-10 is a diagram for describing the Examples.

Fig. 27-11 is a diagram for describing the Examples.

Fig. 28 is a diagram for describing a variant example.

DETAILED DESCRIPTION

Below, exemplary embodiments of the present invention are described in detail with reference to the attached drawings.

= Heart failure estimation system according to first exemplary embodiment =

Fig. 1 shows a heart failure estimation system 10 according to a first exemplary embodiment. As shown in Fig. 1, the heart failure estimation system 10 according to the first exemplary embodiment is equipped with a camera 12, a near-infrared (NIR) camera 13, a heart failure estimation device 14 and a display device 16.

The heart failure estimation system 10 may estimate a cardiac condition of a patient on the basis of an image of a facial region of the patient imaged by the camera 12 or a video of the facial region of the patient. The present exemplary embodiment describes an example in which, as the cardiac condition of the patient, the heart failure estimation system 10 calculates a degree of heart failure of the patient and estimates whether or not the patient has heart failure on the basis of this degree. The cardiac condition of the patient is not limited to a degree of heart failure of the patient but may be a cardiac load condition of the patient, a pulmonary congestion condition of the patient, a fluid retention condition of the patient or the like. This is described more specifically below.

As shown in Fig. 1, functionally, the heart failure estimation device 14 includes an acquisition unit 20, an image data storage unit 22, a reference data storage unit 24, an extraction unit 26, an estimation unit 28 and an output unit 29. The heart failure estimation device 14 is realized by a computer, as described below.

The acquisition unit 20 acquires a still image of the facial region of the patient or a video image of the facial region of the patient. In the present exemplary embodiment, an example is described in which the acquisition unit 20 acquires a video of the facial region of the patient. Images of the facial region of the patient at respective times may be acquired from the video of the facial region of the patient. The patient is a subject for whom the presence or absence of heart failure is to be estimated. The acquisition unit 20 stores the video of the facial region of the patient into the image data storage unit 22.

The image data storage unit 22 stores videos of the patient that are acquired by the acquisition unit 20.

The reference data storage unit 24 stores one or more feature parameters extracted from images of reference patients or videos of reference patients who are already known to have or not have heart failure (referred to below simply as reference data). The reference data is data of people whose degree of heart failure has been diagnosed previously. The reference data storage unit 24 may store derived data based on these data.

The reference data storage unit 24 stores a computation model for using the one or more feature parameters extracted from the images or videos to compute the degree of heart failure of the patient. This computation model is, for example, a statistical model or a machine learning model. For example, when a regression model is used as a statistical model, a regression equation of the regression model and coefficient values of the regression model are stored in the reference data storage unit 24 as the computation model. As another example, when a machine learning model is employed, the machine learning model that is a combination of structural formulas of the machine learning model and acquisition of trained parameters of the machine learning model is stored in the reference data storage unit 24 as the computation model. The statistical model or machine learning model acquires the coefficients or parameters in advance on the basis of training data collected beforehand. These computation models can be used when computing degrees of heart failure of users.

Rather than employing a statistical model, a machine learning model or the like, a degree of similarity between feature parameters extracted from the image or video obtained from the patient and reference data or feature parameters extracted from the reference data may be used to compute the degree of heart failure of the patient. The present exemplary embodiment describes an example in which a computation model for computing degrees of heart failure of users and feature parameters are used to compute the degree of heart failure of a user.

The extraction unit 26 reads video data stored in the image data storage unit 22. The extraction unit 26 performs various kinds of processing on the video data and extracts one or more feature parameters from the video data.

More specifically, the extraction unit 26 extracts the positions of points in the face from an image included in the video data by applying a previously known technique based on machine learning to the image. The positions of these points in the face are extracted to serve as feature points. On the basis of the results of extraction of the feature points, the extraction unit 26 performs detection of the outline of the face, eyes, mouth and so on of the patient.

For example, the extraction unit 26 uses a previously known technique called FACEMESH (https://google.github.io/mediapipe/solutions/face_mesh.html) or DLIB (http://dlib.net/) to extract the feature points from the image of the facial region of the patient and detect the outline of the face, eyes, mouth and so on of the patient. The extraction of feature points may be applied to any of monochrome, color and near-infrared still images and videos. In the case of a video, the feature points are extracted from an image of a single frame constituting the video.

The extraction unit 26 then generates one or more feature parameters on the basis of the plural feature points extracted from the image of the facial region of the patient. These feature parameters are described below.

The estimation unit 28 estimates a degree of heart failure of the patient on the basis of the one or more feature parameters extracted by the extraction unit 26.

For example, on the basis of the one or more feature parameters extracted by the extraction unit 26 and a trained model stored in the reference data storage unit 24, the estimation unit 28 inputs the one or more feature parameters into the trained model and, as a result, acquires a score expressing the degree of heart failure of the patient that is outputted from the trained model. The estimation unit 28 then estimates the degree of heart failure of the patient on the basis of this score.

As an alternative example, on the basis of the one or more feature parameters extracted by the extraction unit 26 and a statistical model stored in the reference data storage unit 24, the estimation unit 28 inputs the one or more statistical parameters as explanatory variables of the statistical model and, as a result, acquires a response variable obtained from the statistical model to serve as a score expressing the degree of heart failure of the patient. The estimation unit 28 then estimates the degree of heart failure of the patient on the basis of this score.

Feature parameters that can be employed in the present exemplary embodiment are described below.

- 1. Parameter relating to shape of the face of the patient -

The points depicted in Fig. 2 and Fig. 3 are an example of the feature points extracted from an image of the facial region of a patient. As shown in Fig. 2 and Fig. 3, the feature points are extracted from respective points of the face of the patient (in the illustrated example, 468 feature points are extracted). The respective feature points have three-dimensional coordinates. An index for identifying the feature points (an identification number) is assigned to each feature point.

In the present exemplary embodiment, one or more feature parameters are generated on the basis of the plural feature points. The feature parameters of the present exemplary embodiment include at least one of a parameter relating to shape of the face of the patient, a parameter relating to complexion of the patient, a parameter relating to an eye of the patient, a parameter relating to the mouth of the patient, a parameter expressing a result of near-infrared analysis of water content of the face of the patient, and a parameter expressing three-dimensional shape data of the face of the patient.

A parameter relating to shape of the face of the patient that is extracted in the present exemplary embodiment is at least one of the following parameters.

(1) Parameter expressing an area of the face

Fig. 2 is a diagram for describing a parameter expressing the area of the face of the patient. As illustrated in Fig. 2, the present exemplary embodiment uses a region encircled by feature points disposed at outline portions of the head of the patient as a parameter expressing the area of the face. Because detection of the outline of the head of the patient may be affected by the hairline, an area "Area_brow" of the face in a region below the eyebrows of the patient can be detected as a feature parameter. A sum of the area Area_brow of the face in the region below the eyebrows of the patient and an area of the face in a region above the eyebrows of the patient can be utilized as a parameter "Area_all" expressing the area of the face.

(2) Parameter expressing a horizontal direction width of the face

Fig. 3 is a diagram for describing a parameter expressing a horizontal direction width of the face of the patient. As shown in Fig. 3, the present exemplary embodiment utilizes a width of the face corresponding to a distance between feature points disposed at the outline portions of the head of the patient as the parameter expressing the horizontal direction width of the face (below referred to simply as the width of the face). In the example shown in Fig. 3, a width WO5_29, a width W06_28, a width W07_27, a width W08_26, a width W09_25, a width W10_24, a width W11_23, a width W12_22, a width W13_21, a width W14_20, a width W15_19 and a width W16_18 of regions below the eyes are illustrated. The digits are indexes for identifying the feature points. The size of the face of the patient within an imaging angle differs each time an image is captured. In order to correct for this, in the present exemplary embodiment, each of the above-mentioned widths is standardized by being divided by a distance "reference(w)" between a feature point indicating the inner corner of the left eye and a feature point indicating the inner corner of the right eye. Thus, widths of the face that differ between images are standardized and the parameter expressing the width of the face can be extracted. In the present exemplary embodiment, an example is described in which widths of the face are standardized by the distance reference(w) between the feature point indicating the inner corner of the left eye and the feature point indicating the inner corner of the right eye, but this is not limiting. For example, a marker that serves as reference points may be attached to the face of the patient. Because the size (width) of the marker is already known, widths of the face may be standardized in accordance with the size (width) of the marker.

(3) Parameter expressing a vertical direction pitch of the face

The present exemplary embodiment utilizes a pitch direction inclination of the head of the patient as the parameter (Pitch) expressing a vertical direction pitch of the face. More specifically, on the basis of three-dimensional coordinates of the above-mentioned feature points, a parameter expressing the vertical direction pitch of the face is calculated from depth direction coordinate information of the feature points. For example, a depth direction coordinate of a feature point disposed in the upper half of the face of the patient is compared with a depth direction coordinate of a feature point disposed in the lower half of the face of the patient, and when the feature point disposed in the upper half of the face is further away from the camera 12 than the feature point disposed in the lower half of the face, the parameter expressing the vertical direction pitch of the face is calculated as an inclination of the head of the patient in a direction with the chin projecting forward. Alternatively, when the feature point disposed in the lower half of the face is further away from the camera 12 than the feature point disposed in the upper half of the face, the parameter expressing the vertical direction pitch of the face is calculated as an inclination of the head of the patient in a direction with the chin being drawn back.

(4) Parameter of a vector expressing features of the face

The present exemplary embodiment employs a vector expressing features of the face as a parameter. For example, an image of the face is inputted to a previously known machine learning model, and as a result a vector expressing features of the face (below referred to simply as a face vector) is outputted from the machine learning model. The present exemplary embodiment uses the face vector obtained in this manner as a parameter. The present exemplary embodiment utilizes a previously known technology called FACEANET (https://github.com/davidsandberg/FACENET). Therefore, the present exemplary embodiment uses a 512-dimension face vector as the parameter. The face vector manifests features, positions and the like of points of the face.

(5) Parameter expressing change of a face vector (embdiff)

The present exemplary embodiment employs a parameter embdiff that expresses a change in the face vector. The parameter expressing the change of the face vector is at least one of the following parameters.

(5-1) Feature parameter expressing distance between a face vector obtained on the estimation date and a face vector obtained on a previous date (embdiff_day)

For example, a new feature parameter embdiff_day is generated that is a distance between the face vector obtained from an image of the patient on an estimation day D2 and the face vector obtained from an image of the patient on a previous date D1 prior to the estimation date D2. Fig. 4 is a diagram for describing the new feature parameter embdiff_day. As illustrated by the example in Fig. 4, an image of the facial region of a patient imaged on June 27 is inputted into a convolution neural network (CNN) used by the above-mentioned FACEANET, and a 512-dimension face vector for June 27 is extracted. As is further illustrated in Fig. 4, an image of the facial region of the patient imaged on June 28 is inputted into the CNN and a 512-dimension face vector for June 28 is extracted. In this way, face vectors for respective dates are obtained as illustrated in Fig. 4. A distance between the face vector obtained from the image of the patient on June 28, which is an estimation date, and the face vector obtained from the image of the patient on June 27 prior to the estimation date is calculated. More specifically, the distance between the 512-dimension face vector for June 27 and the 512-dimension face vector for June 28 is calculated as a feature parameter for June 28 (6/28 embdiff_day). The feature parameter is calculated for respective dates in the same way.

(5-2) Feature parameter expressing distance between a face vector obtained on the estimation date and a face vector for a remission period or a progression period (embdiff_dod or embdiff_doa)

For example, a feature parameter embdiff_dod or embdiff_doa is generated that is a distance between the face vector obtained from an image of the patient on an estimation date and the face vector in a remission period or a progression period.

More specifically, a new feature parameter embdiff_dod can be generated that is the distance between the face vector obtained from the image of the patient on the estimation date D1 and the face vector obtained from an image of the patient on a date Dx on which the patient is in remission and leaves the hospital. With this parameter, the current condition of the patient is estimated by reference to the face vector of the face of the patient in a remission period. In the parameter label, "dod" is an abbreviation of "date of discharge".

Fig. 5 is a diagram for describing this new feature parameter embdiff_dod. As illustrated by the example in Fig. 5, an image of the facial region of a patient imaged on June 27 is inputted into the CNN, and the 512-dimension face vector for June 27 is extracted. As is further illustrated in Fig. 5, the image of the facial region of the patient imaged on June 28 is inputted into the CNN and the 512-dimension face vector for June 28 is extracted. In this way, the face vectors for respective dates are obtained as illustrated in Fig. 5. A distance between the face vector obtained from the image of the patient on June 28, which is an estimation date, and the face vector obtained from an image of the patient on July 9, which is the date the patient is discharged, is calculated. More specifically, the distance between the 512-dimension face vector for June 27 and the 512-dimension face vector for July 9 is calculated as a feature parameter for June 27 (6/27 embdiff_dod). The feature parameter is calculated for respective dates in the same way.

Alternatively, a new feature parameter embdiff_doa is generated that is the distance between the face vector obtained from the image of the patient on the estimation date D1 and the face vector obtained from an image of the patient on a date Dx on which the patient is in progression and goes into the hospital. With this parameter, the current condition of the patient is estimated by reference to the face vector of the face of the patient in a progression period. In the parameter label, "doa" is an abbreviation of "date of admission".

Fig. 6-1A and Fig. 6-1B are diagrams showing examples of trends in embdiff_dod and embdiff_doa. Fig. 6-1A and Fig. 6-1B are diagrams depicting trends in the values of embdiff_dod and embdiff_doa when a patient goes into remission due to medical treatment of heart failure. These trends in embdiff_dod and embdiff_doa depict changes in an actual patient. The facial images shown below the graphs are depicted schematically in consideration of privacy.

Fig. 6-1A uses a facial image in a remission period as the reference. The vertical axis (y axis) of the graph shown in the upper part of Fig. 6-1A indicates values of embdiff_dod and the horizontal graph (x axis) indicates dates. The dates indicated by the x axis of the graph in Fig. 6-1A run from Day 1 (a progression period) to Day 12 (a remission period) for that patient. Facial images of the patient corresponding with the respective dates are illustrated in the lower part of Fig. 6-1A. Day 1 in the lower part of Fig. 6-1A corresponds with the heart failure progression period and Day 12 corresponds with the heart failure remission period. As shown in the lower part of Fig. 6-1A, it can be seen that a degree of edema of the patient decreases from Day 1 to Day 12. As is shown in Fig. 6-1A, the difference (distance) is greatest between the face vector obtained from the facial image for Day 1, when the heart failure had progressed furthest, and the face vector obtained from the facial image in the remission period that is the reference. The difference between the face vectors decreases from Day 1 in the progression period to Day 12 in the remission period as a result of medical treatment of the heart failure. Therefore, it can be seen that embdiff_dod changes in accordance with the condition of heart failure, and the condition of the patient steadily improves. Thus, assigning embdiff_dod to the vertical axis (y axis) and assigning date or time to the horizontal axis (x axis) enables evaluations from degrees of change in embdiff_dod (calculated by, for example, dividing a difference between embdiff_dod on a particular date and embdiff_dod on a different date by the number of days between) in combination with disease states (for example, a speed of progression or remission of the heart failure and the like).

Fig. 6-1B uses a facial image in the progression period as the reference. The vertical axis (y axis) of the graph shown in the upper part of Fig. 6-1B indicates values of embdiff_doa, and the horizontal graph (x axis) indicates dates. The dates indicated by the x axis of the graph in Fig. 6-1B run from Day 1 (the progression period) to Day 12 (the remission period) for that patient. As is shown in Fig. 6-1B, the difference (distance) is smallest between the face vector obtained from the facial image for Day 1, when the heart failure had progressed furthest, and the face vector obtained from the facial image in the progression period that is the reference. The difference between the face vectors increases from Day 1 in the progression period to Day 12 in the remission period as a result of the medical treatment of the heart failure. Because the face vector for each date becomes further from the face vector in the period of progression as the days pass, similarly to Fig. 6-1A, it can be seen that embdiff_doa changes in accordance with the condition of heart failure. This enables evaluations from degrees of change in embdiff_doa in combination with disease states (for example, a speed of progression or remission of the heart failure and the like).

The image of the patient that is the reference is acquired in advance by imaging the face of the patient in a progression period, a remission period or a period of stability. More specifically, the image of the patient that is to be the reference is acquired in advance by imaging the face of the patient when the patient is transported to the hospital, when the patient visits the hospital for an initial examination, at a time of hospital admission, at a time of hospital discharge, when some change occurs, or in a period while the patient is staying at home. The difference between a face vector obtained from the image of the patient on a respective date and a face vector obtained from the reference image, which is a heart failure state, is calculated to serve as a feature parameter. Thus, by utilizing differences between the face vector obtained from the reference image and face vectors obtained from images captured on estimation dates, a time series of states of the patient may be observed. By taking account of chronological changes between the face vector obtained from the reference image and the face vectors obtained from the images captured on estimation dates, "differences in the features of the faces of different patients" may be corrected for, and the accuracy of estimating heart failure states may be improved. Moreover, accuracy of a machine learning model may be improved by collecting large quantities of data, and even when states over time cannot be compared, a state of heart failure may be estimated from a single image capture.

When a patient is in a heart failure state, the body of the patient may not be able to properly excrete bodily fluids (specifically, water in the body). As a result, edema occurs in the body of the patient. The shape, outline, area, width and the like of the face are altered by this swelling. When swelling occurs in the face of a patient, the face of the patient may even be identified as the face of a different person. Accordingly, the heart failure estimation system 10 of the present exemplary embodiment extracts parameters relating to the shape of the face of a patient from images of the facial region of the patient, and estimates heart failure of the patient on the basis of these parameters. In addition, because the parameters described above and below are calculated as continuous variables, a speed of progression or remission of heart failure may be estimated from the magnitude of a change in values of a parameter.

- 2. Parameter relating to complexion of the patient -

A parameter relating to the complexion of a patient is utilized in the present exemplary embodiment. The parameter relating to the complexion of the patient that is extracted in the present exemplary embodiment is at least one of the following parameters.

(1) Parameter expressing hue of the face of the patient

The present exemplary embodiment employs a parameter expressing a hue of the face of a patient, which is extracted from an image of the facial region of the patient.

(2) Parameter expressing green component of the face of the patient (Green)

The present exemplary embodiment employs a parameter expressing a green component of the cheeks, the area between the eyebrows, or the nose in the face of a patient. The region of the cheeks, area between the eyebrows, or nose can be identified on the basis of positions of the above-mentioned feature points.

Complexion changes mainly in accordance with amounts of melanin and amounts of hemoglobin. Darkening of the skin when tanned by the sun is due to an increase in melanin. Meanwhile, hemoglobin is a protein that carries oxygen and is known to have a light absorption peak in the vicinity of 550 nm in the visible light range. The vicinity of 550 nm corresponds with a green component of the three primary colors of visible light. When hemoglobin in the body decreases due to bleeding, anemia or the like, reflected light in the vicinity of 550 nm increases, causing redness of the face to decrease, which is apparent to the human eye as the face looking pale. The upper graph in Fig. 6-2 shows a relationship between hemoglobin concentrations in the blood and values of the green component in the cheeks (Green) obtained from images of a particular case (G-HF017). The horizontal axis of the graph indicates values of hemoglobin concentration in the blood of the patient (Hb, g/dl), which are obtained from blood tests, and the vertical axis indicates values of the green component obtained from facial images of the patient. The graph illustrates that as the hemoglobin concentration in the blood rises, absorption by the hemoglobin of light with wavelength components corresponding to green colors increases and values of the green component obtained from facial images decrease.

The lower graph in Fig. 6-2 shows a relationship between hemoglobin concentrations in the blood and values of hue of a lower eyelid (Hue) obtained from images of the particular case (G-HF017). The horizontal axis of the graph indicates values of hemoglobin concentration in the blood of the patient (Hb, g/dl), which are obtained from blood tests, and the vertical axis indicates values (Hue) of the hue obtained from facial images of the patient. The graph illustrates that as the hemoglobin concentration in the blood rises, values of hue obtained from facial images decrease, changing in a direction of increasing redness of the complexion. The hue values represent hue angles in the L*a*b color space, with the direction approaching zero representing increasing redness.

The graph in Fig. 6-3 shows relationships between hemoglobin concentrations in the blood and values of hue of lower eyelids (Hue) that are obtained from images of plural cases. The horizontal axis of the graph in Fig. 6-3 indicates values of hemoglobin concentration in the blood of the patients (Hb, g/dl), which are obtained from blood tests, and the vertical axis indicates values (Hue) of the hue obtained from facial images of the patients. As mentioned above, complexion changes mainly in accordance with amounts of melanin and amounts of hemoglobin. Because melanin is increased by exposure to ultraviolet rays from sunlight, amounts of melanin differ between individuals. There are also individual differences in hemoglobin values between cases. The graph in Fig. 6-3 shows differences in hue resulting from individual differences caused by melanin and hemoglobin amounts. However, when changes within the respective cases are examined, as long as melanin amounts do not change greatly, changes in complexion are principally due to changes in hemoglobin amounts. In particular, changes in hemoglobin concentrations in the blood may be perceived by measurements of changes in complexion in environments with little opportunity for exposure to ultraviolet light, such as when staying in the hospital and the like.

When a patient has a heart failure condition, the concentration of hemoglobin generally decreases due to an increase in water retention in the body, leading to anemia. In addition, when a patient has a heart failure condition, blood is not adequately supplied to the whole body and redistribution of the blood occurs. That is, in order to assure blood supplies to vital organs (for example, the brain and the viscera), blood vessels constrict in organs that do not have particularly high priority in regard to maintaining life, such as skin, muscles and the like, and their blood supplies are limited. When blood vessels constrict and blood supplies are limited, amounts of blood distributed to those organs are smaller and supplies of hemoglobin contained in that blood decrease. As a result, the face of the patient becomes pale, similarly to anemia. Accordingly, the heart failure estimation system 10 according to the present exemplary embodiment extracts a parameter relating to complexion of the patient from an image of the facial region of the patient and estimates heart failure of the patient on the basis of this parameter. In order to acquire the parameter expressing the complexion, a marker for standardizing complexion may be attached to the face of the patient or the like. Because the color of the marker is already known, complexions may be standardized in accordance with the color of the marker.

The upper graph in Fig. 6-4 shows trends over time, in association with in-patient treatment of heart failure, of hemoglobin concentrations in the blood and hues (Hue) of the lower eyelid obtained from images of a particular case (G-HF017). The horizontal axis of this graph indicates days passed from the date of hospital admission. Toward the right, treatment proceeds and the heart failure goes into remission. The vertical axis at the left side indicates values of hemoglobin concentration in the blood of the patient (Hb, g/dl), which are obtained from blood tests, and the vertical axis at the right side indicates values of hue obtained from facial images of the patient (Hue). Looking at the six days after hospital admission due to heart failure progression, water amounts in blood vessels decrease in a period of emergency treatment due to water being eliminated by a diuretic, and the graph shows a temporary rise in hemoglobin concentrations in the blood, shifting to stable values from the seventh day onward. The graph of hue values shows a decrease from hospital admission, corresponding to the changes in hemoglobin concentrations in the blood (decreasing hue corresponding to an increase in redness of the complexion), subsequently shifting to stable values. In particular, from the seventh day onward the hemoglobin concentrations that temporarily rose in the emergency period fall again and stabilize. In spite of the decrease in hemoglobin concentrations, hue values proceed to stabilize rather than increasing. This is thought to be a result of the heart failure condition improving, blood flows to the skin that were restricted by redistribution improving (increasing), and absolute amounts of hemoglobin in the vicinity of the face increasing.

The lower graph in Fig. 6-4 shows a relationship between NYHA functional categories and hue values of the lower eyelid obtained from images of the particular case (G-HF017). The horizontal axis of the graph indicates values of a symptom index (NYHA functional classification) when data of the case evaluated by a cardiologist are acquired. The vertical axis indicates values of hue obtained from facial images of the patient (Hue). Heart failure treatment causes the heart failure condition to go into remission and the NYHA functional category decreases (the symptoms recede). Similarly, hue values decrease (redness increases), reflecting the rise in hemoglobin concentrations and increase in blood flow amounts (absolute values of hemoglobin) caused by the improvement in the heart failure condition, and this demonstrates a strong correlation (R=0.93) between the degree of heart failure symptoms and the hue of the face.

- 3. Parameter relating to an eye of the patient -

The present exemplary embodiment employs a parameter relating to the eyes of the patient. The parameter relating to the eyes of the patient that is extracted in the present exemplary embodiment is at least one of the parameters in Table 1 below.

Table 1

| Feature parameter | Label |
|---|---|
| Parameter expressing aspect ratio of an eye of the patient | EAR |

(continued)

| Feature parameter | Label |
|---|---|
| Parameter expressing difference between the aspect ratios EAR of the left and right eyes of the patient | diffLR |
| Parameter expressing a duration an eye is closed in a single blink | width |
| Parameter expressing a proportion of a unit time that an eye is closed | fcnt |
| Parameter expressing an interval between blinks | b_intv |
| Parameter expressing a number of blinks per unit time | Close |
| Parameter expressing speed when an eye closes | grad_close |
| Parameter expressing speed when an eye opens | grad_open |
| Parameter expressing length of time an eye is closed | area |
| Parameter expressing average speed during opening and closing of an eye | grad_avg |

Fig. 7 to Fig. 9 are diagrams for describing the parameters listed in Table 1 above.

(1) Parameter expressing aspect ratio of an eye of the patient (EAR: Eye Aspect Ratio)

As shown in Fig. 7, for example, feature points $P_1$, $P_2$, $P_3$, $P_4$, $P_5$ and $P_6$ in the vicinity of the eye are extracted by the processing mentioned above. Hence, the parameter EAR expressing the aspect ratio of the eye of the patient is calculated in accordance with distances between the feature points in the vicinity of the eye as depicted in Fig. 7. More specifically, the parameter EAR expressing the aspect ratio of the eye of the patient is calculated by the following expression.

$$\mathrm{ERA} = \frac{\|P_2 - P_6\| + \|P_3 - P_5\|}{2\|P_1 - P_4\|}$$

(2) Parameter expressing difference between the aspect ratios EAR of the left and right eyes of the patient (diffLR)

The parameter diffLR expressing the difference between the aspect ratios EAR of the left and right eyes of the patient is calculated in accordance with feature points in the vicinities of the eyes as depicted in Fig. 7.

(3) Parameter expressing duration an eye is closed in a single blink (width)

The parameter expressing a duration an eye is closed when a patient blinks is calculated, for example, as illustrated in Fig. 8 in accordance with the feature points in the vicinity of the eye as depicted in Fig. 7. Fig. 8 shows an example in which values of the parameter EAR expressing the aspect ratio of the eye of the patient at respective times are set to 100% in an open eye state, according to maximum likelihood estimation, and set to 0% when the value of EAR is zero. Accordingly, values on the vertical axis (y axis) in Fig. 7 indicate degrees of opening of the eye (%) calculated from the parameter EAR expressing the aspect ratio of the eye of the patient. For example, the parameter expressing the duration that the eye is closed in a single blink (width) is a duration W80width in which degrees of opening of the eye are 80% and a duration W20width in which degrees of opening of the eye are 20%.

(4) Parameter expressing a proportion of a unit time that an eye is closed (fcnt)

The parameter fcnt expressing the proportion of a unit time that an eye is closed is calculated in accordance with the feature points in the vicinity of the eye as depicted in Fig. 7. For example, a sum of durations in which the degree of opening of the eye (%) is 80% or less in a predetermined duration is divided by the predetermined duration. This divided value is calculated to serve as a parameter W80fcnt expressing the proportion of unit time that the eye is closed. A sum of durations in which the degree of opening of the eye (%) is 20% or less in a predetermined duration is also divided by the predetermined duration, and this divided value is calculated to serve as a parameter W20fcnt expressing the proportion of unit time that the eye is closed.

(5) Parameter expressing an interval between blinks (b_inv)

The parameter b_intv expressing an interval between blinks is calculated in accordance with the feature points in the vicinity of the eye as depicted in Fig. 7. For example, a duration from a time when the degree of opening of the eye (%) falls below 20% until a time when the parameter EAR expressing the aspect ratio of the eye of the patient next falls below 20% is calculated to serve as the parameter b_inv expressing the interval between blinks. An average b_intv_avg of the intervals b_inv between blinks by the patient can also be calculated.

(6) Parameter expressing a number of blinks per unit time (Close)

The parameter Close expressing the number of blinks per unit time is calculated in accordance with the feature points in the vicinity of the eye as depicted in Fig. 7. For example, a number of times the degree of opening of the eye falls below 20% in a predetermined duration is divided by the predetermined duration. This divided value is calculated to serve as the parameter Close expressing the number of blinks per unit time.

(7) Parameter expressing speed when an eye closes (grad_close)

The parameter grad_close expressing a speed when an eye closes is calculated in accordance with the feature points in the vicinity of the eye as depicted in Fig. 7. Values on the vertical axis (y axis) in Fig. 9, similarly to Fig. 8, indicate degrees of opening of the eye (%) calculated from the parameter EAR expressing the aspect ratio of the eye of the patient. The speed when the eye closes corresponds to a falling slope (grad_close) of the degree of opening of the eye, as illustrated in Fig. 9. A parameter grad_close_avg expressing an average of speeds grad_close when the eye closes is then calculated by averaging the falling slopes grad_close of the degree of opening of the eye.

(8) Parameter expressing speed when an eye opens (grad_open)

The parameter grad_open expressing a speed when an eye opens is calculated in accordance with the feature points in the vicinity of the eye as depicted in Fig. 7. The speed when the eye opens corresponds to a rising slope (grad_open) of the degree of opening of the eye, as illustrated in Fig. 9. A parameter grad_open_avg expressing an average of speeds grad_open when the eye opens is then calculated by averaging the rising slopes grad_open of the degree of opening of the eye.

(9) Parameter expressing length of time an eye is closed (area)

The parameter area expressing a length of time an eye is closed is calculated in accordance with the feature points in the vicinity of the eye as depicted in Fig. 7. For example, as illustrated in Fig. 9, the area of a region defined by a length of time the degree of opening of the eye is 80% or less, the parameter grad_close expressing a speed when the eye closes, and the parameter grad_open expressing a speed when the eye opens may be calculated to serve as the parameter expressing the length of time the eye is closed (area). Alternatively, simply the length of time the degree of opening of the eye is 80% or less may be calculated to serve as the parameter expressing the length of time the eye is closed (area).

(10) Parameter expressing average speed during opening and closing of an eye (grad_avg)

The parameter grad_avg expressing an average speed during opening and closing of the eye is calculated in accordance with the feature points in the vicinity of the eye as depicted in Fig. 7. For example, the parameter grad_avg expressing the average speed during opening and closing of the eye is calculated on the basis of the parameter grad_close expressing a speed when the eye closes and the parameter grad_open expressing a speed when the eye opens.

When a patient has a heart failure condition, edema occurs in the face of the patient. When this happens, water retention is likely to occur in regions in the face with relative free space (that is, looser regions in the face). For example, the eyelids are usually a region with free space. When water is retained in this region, the eyelids swell, opening the eyes is more difficult, and the eyes are smaller (more specifically, opening of the eyes decreases and the aspect ratio changes). When the patient has the heart failure condition, the patient feels weariness, as a result of which continuous opening of the eyes is difficult. Because of these changes, durations that the eyes of the patient are closed are longer, numbers of blinks are greater, and times taken in blinking are longer. The person with the heart failure condition blinks slowly compared to the momentary blinking of a healthy person. Moreover, when the person with the heart failure condition faces forward with the eyelids swollen and the eyes being difficult to open, the orientation of the face inevitably tends to pitch upward (more specifically, in an attitude such that the chin projects forward). For these reasons, the heart failure estimation system 10 according to the present exemplary embodiment extracts a parameter relating to the eyes of a patient from an image of the facial region of the patient and estimates heart failure of the patient on the basis of this parameter.

- 4. Parameter relating to the mouth of the patient -

The present exemplary embodiment employs a parameter relating to the mouth of a patient. The parameter relating to the mouth of the patient that is extracted in the present exemplary embodiment is at least one of the parameters in Table 2 below.

Table 2

| Feature parameter | Label |
|---|---|
| Parameter expressing aspect ratio of the mouth of the patient | m_OUTLINE |
| Parameter expressing degree of opening of the mouth of the patient | m_OPEN |
| Parameter expressing the left corner of the mouth | m_gradL |
| Parameter expressing the right corner of the mouth | m_gradR |

Fig. 10 is diagrams for describing the parameters listed in Table 2 above. As shown in Fig. 10, for example, feature points in the vicinity of the mouth are extracted by the processing mentioned above. The digits in the drawing are indexes for identifying the feature points in the vicinity of the mouth.

(1) Parameter expressing aspect ratio of the mouth of the patient (m_OUTLINE)

The parameter m_OUTLINE expressing the aspect ratio of the mouth of the patient is calculated in accordance with the feature points in the vicinity of the mouth as depicted in Fig. 10. For example, in the example shown in Fig. 10, the average of the length of the line joining feature point 37 with feature point 84, the length of the line joining feature point 0 with feature point 17 and the length of the line joining feature point 267 with feature point 314 is divided by the length of the line joining feature point 61 with feature point 291 to calculate the value of m_OUTLINE.

(2) Parameter expressing degree of opening of the mouth of the patient (m_OPEN)

The parameter m_OPEN expressing the degree of opening of the mouth of the patient is calculated in accordance with the feature points in the vicinity of the mouth as depicted in Fig. 10. For example, in the example shown in Fig. 10, the average of the length of the line joining feature point 82 with feature point 87, the length of the line joining feature point 13 with feature point 14 and the length of the line joining feature point 312 with feature point 317 is calculated as the value of m_OPEN.

(3) Parameter expressing the left corner of the mouth (m_gradL)

The parameter m_gradL expressing the left corner of the mouth of the patient is calculated in accordance with the feature points in the vicinity of the mouth as depicted in Fig. 10. For example, in the example shown in Fig. 10, an angle formed between a straight line (the broken line) specified in the left-and-right direction of the mouth and a straight line indicating the corner of the mouth at the left side, which is specified by a feature point in the vicinity of the left corner of the mouth, is calculated to serve as the parameter m_gradL expressing the left corner of the mouth. In the upper part of Fig. 10, the line joining feature point 0 with feature point 17 serves as a reference line in the vertical direction, and a line (the broken line shown in the lower part of Fig. 10) that crosses this reference line at a feature point in the vicinity of an upper end region of the lower lip serves as a horizontal reference line.

(4) Parameter expressing the right corner of the mouth (m_gradR)

The parameter m_gradR expressing the right corner of the mouth of the patient is calculated in accordance with the feature points in the vicinity of the mouth as depicted in Fig. 10. For example, in the example shown in Fig. 10, an angle formed between the straight line (the broken line) specified in the left-and-right direction of the mouth and a straight line indicating the corner of the mouth at the right side, which is specified by a feature point in the vicinity of the right corner of the mouth, is calculated to serve as the parameter m_gradR expressing the right corner of the mouth.

As described above, when a patient has a heart failure condition, the patient must raise heavy eyelids in which swelling has occurred in order to open their eyes. Therefore, corresponding effort is required for the patient with the heart failure condition to open their eyelids. Moreover, when the person with the heart failure condition faces forward with the eyelids swollen and the eyes being difficult to open, the orientation of the face inevitably tends to pitch upward. With the heart failure condition, water is retained in the cheeks in vicinities of the corners of the mouth, is pulled down by gravity, and may drag the corners of the mouth downward. As a result of these effects, when the patient has the heart failure condition, the effort associated with opening the eyes produces a "gritted teeth" expression, the inclination of the face pitches upward, and the edema of the cheeks feels heavier and deforms or displaces the corners of the mouth downward. Similarly to the eyelids, the thickness, shape and the like of the lips are changed by the occurrence of edema. For these reasons, the heart failure estimation system 10 according to the present exemplary embodiment extracts a parameter relating to the mouth of the patient from an image of the facial region of the patient and estimates heart failure of the patient on the basis of this parameter.

- 5. Parameter expressing a result of near-infrared analysis of water content of the face of the patient -

The present exemplary embodiment employs a parameter expressing results of analysis of water content by near-infrared illumination of the face of a patient. Water is known to have a spectral characteristic with a light absorption peak in the near-infrared wavelength region. When light including near-infrared radiation is illuminated onto the face of a patient and dispersed light is measured, how much of the light is absorbed by water within the skin, and thus water content, may be measured. More specifically, a parameter expressing a water content contained in the face of the patient is calculated on the basis of an image captured by the NIR camera 13. In specific terms, the parameter expressing the water content contained in the face of the patient is calculated by using near-infrared spectroscopy to analyze the image captured by the NIR camera 13. The effects of inconsistencies in illumination appearing in this image may be eliminated by using differential absorption imaging, which is already known. For example, when considering an absorption peak of 980 nm in a wavelength range of 700 nm to 1100 mm, a wavelength band at 700 nm, whose absorbency by water is much lower than the absorbency at 980 nm and changes with illuminated light intensity, may be removed. Camera output signals in the respective wavelength bands contain noise components due to dark currents in imaging elements. Therefore, actual light intensities may be obtained by subtracting camera output signals that are captured when the camera is shielded. For example, a parameter $A_{diff}$ that is calculated by the following expression is calculated to serve as the parameter expressing a result of analysis of water content. In the expression, $I_{980}$ is a camera output signal strength for the 980 nm wavelength band when the face of the patient is imaged, $Idark_{980}$ is a camera output signal strength for the 980 nm wavelength band when the camera is shielded, $I_{700}$ is a camera output signal strength for the 700 nm wavelength band when the face of the patient is imaged, and $Idark_{700}$ is a camera output signal strength for the 700 nm wavelength band when the camera is shielded. The above description of wavelength bands used in imaging is an example. A nearby wavelength band and another water absorption peak wavelength such as, for example, 1450 nm or the like may be used.

$$
\begin{aligned}
A_{diff} &= \log_{10}(1/(I_{980} - Idark_{980})) - \log_{10}(1/(I_{700} - Idark_{700})) \\
&= \log_{10}((I_{700} - Idark_{700})/(I_{980} - Idark_{980}))
\end{aligned}
$$

A region of the face on which the analysis of water content is performed is divided into, for example, the regions shown in Fig. 11-1 according to the aforementioned feature points in the face of the patient, and the parameter expressing water content is calculated for the respective regions. The regions in the face in Fig. 11-1 are specified on the basis of feature points detected by the processing mentioned above. Results of analysis of regions of the whole of the face confirm a strong correlation between changes in the parameter expressing water content and levels of heart failure progression, particularly for the regions of the upper and lower eyelids of a patient and regions of the cheeks of the patient shown in Fig. 11-1.

As described above, the outline of the face of a patient with a heart failure condition is larger due to edema in the face. However, patients with heart failure conditions include some cases in which edema of the face is not particularly strong, in which cases it may be hard to discern changes in the outline of the face of the patient. These changes can be seen in a progressive stage of heart failure but it is commonly difficult to discern changes in the outline of the face when heart failure progression eases. On the other hand, as heart failure progression becomes more severe, water content in the body increases and changes in the outline of the face are very notable.

In essence, water retention often manifests in the forward direction and lateral directions of the face. However, in some patients retention of water manifests notably only in the forward direction of the face (that is, as a shape projecting toward the front). Water retention that manifests in the lateral direction of the face may be evaluated by extracting the outline of the face in two dimensions. However, when water retention manifests strongly in the forward direction of the face, estimating the degree of heart failure by using the above-mentioned feature parameter relating to the outline of the face may be difficult.

Accordingly, the heart failure estimation system 10 according to the present exemplary embodiment extracts a parameter relating to water content obtained by near-infrared analysis and estimates the heart failure condition of the patient on the basis of this parameter. Accordingly, water content in regions being examined in the face may be measured. That is, when water retention occurs in the face, the water retention may be perceived even when the water retention manifests in the horizontal direction of the face, when the water retention manifests in the forward direction of the face, and when the water retention is of an extent that does not cause enough effect to change the outline of the face. In some cases of heart failure it may be difficult to judge a heart failure condition from the outline of the face and the like in practice. However, it has been verified that water contents obtained by near-infrared analysis change and decrease due to treatment even in these cases, and morphological changes of the face and complementary actions have been verified. The melanin and hemoglobin that principally affect the color of human skin have low absorbencies in the wavelength range from 700 nm to 1100 nm that is used for analysis of water content by near-infrared illumination as mentioned above. Therefore, near-infrared analysis of water content is unlikely to be affected by differences in the color of skin associated with ethnicity. Moreover, components in this wavelength range are very low or not included in room lighting, which is principally fluorescent lights, LEDs and the like, so room lighting does not affect the analysis. In order to acquire the parameter expressing a result of near-infrared analysis of water content, a marker for standardizing signal levels may be attached to the face of the patient or the like, or may be disposed near the face. Because reflectivity of the marker is already known, signal levels may be standardized.

- 6. Parameter expressing three-dimensional shape data of the face of the patient

The present exemplary embodiment employs a parameter expressing three-dimensional shape data of the face of a patient. More specifically, the parameter expressing three-dimensional shape data of the face of the patient is calculated on the basis of an image captured by a depth camera (not shown in the drawings).

Of the feature parameters described above, when calculating a parameter relating to the shape of the face of the patient, a parameter relating to an eye of the patient or a parameter relating to the mouth of the patient, feature points detected by FACEMESH or the like may simply be projected onto a two-dimensional plane to enable calculations of distances and areas between the feature points. On the other hand, when feature points measured by a camera or estimated by FACEMESH or the like have a depth direction (Z), distances and areas between the feature points may be calculated in three-dimensional space. An example of detecting facial feature points in a three-dimensional space is illustrated in Fig. 11-2.

The feature parameters described above are utilized in the present exemplary embodiment. Fig. 12 and Fig. 13 show tables of the above-mentioned feature parameters.

The above-mentioned feature parameters may be utilized as numerical values, and degrees of heart failure may also be evaluated as numerical values. In order to calculate degrees of heart failure as objective numerical values, a skill-independent index that overrides the boundaries between specialisms, occupations and the like may be employed. A state of illness may also be evaluated from a degree of rate of change. For example, a patient for whom the numerical value representing the degree of heart failure increases by three over one day, from 2 to 5, can be judged to have an illness that is deteriorating more rapidly than a patient for whom the numerical value expressing the degree of heart failure increases by one over one day, from 1 to 2. Thus, how quickly a patient is deteriorating or improving may be evaluated as well as whether the state of the patient is simply bad or good.

The output unit 29 outputs the estimation result estimated by the estimation unit 28. The output unit 29 may output the score representing the degree of heart failuure itself as the estimation result.

The display device 16 displays the estimation result outputted from the output unit 29.

A clinical practitioner or user operating the heart failure estimation device 14 checks the estimation result outputted from the display device 16 and checks the possibility that the patient has heart failure.

The heart failure estimation system 10 according to the present exemplary embodiment is expected to be used, for example, in a situation as illustrated in Fig. 14.

In the example in Fig. 14, a healthcare worker H such as a doctor or the like holds a tablet terminal, which is an example of the heart failure estimation system 10. The healthcare worker H uses a camera (not shown in the drawings) provided at the tablet terminal to collect image data of a patient U. On the basis of the image data of the patient U, the tablet terminal estimates whether or not the patient U has heart failure and outputs an estimation result to a display unit (not shown in the drawings). The healthcare worker H refers to the estimation result displayed at the display unit (not shown in the drawings) of the tablet terminal and the healthcare worker H judges a degree of heart failure of the patient U.

The heart failure estimation device 14 may be realized by, for example, a computer 50 as illustrated in Fig. 15. The computer 50 is provided with a CPU 51, a memory 52 that serves as a temporary memory region, and a nonvolatile memory section 53. The computer 50 is further provided with an input/output interface (I/F) 54 to which external equipment, output devices and the like are connected, and a read/write (R/W) section 55 that controls reading and writing of data at a recording medium. The computer 50 is also provided with a network interface 56 that is connected to a network such as the Internet or the like. The CPU 51, memory 52, memory section 53, input/output interface 54, read/write section 55 and network interface 56 are connected to one another via a bus 57.

The memory section 53 may be realized by a hard disk drive (HDD), solid-state drive (SSD), Flash memory or the like. A program for causing functioning of the computer 50 is memorized at the memory section 53, which serves as a memory medium. The CPU 51 reads the program from the memory section 53, loads the program into the memory 52, and sequentially executes processes of the program.

- Operation of the heart failure estimation system according to the first exemplary embodiment -

Now, concrete operations of the heart failure estimation system 10 according to the first exemplary embodiment are described. The heart failure estimation device 14 of the heart failure estimation system 10 executes the processing shown in Fig. 16.

First, in step S100, the acquisition unit 20 acquires a video of a patient captured by the camera 12. The acquisition unit 20 then stores the video of the patient into the image data storage unit 22. An acquisition unit may acquire a video of a patient captured by the NIR camera 13 and store this video into the image data storage unit 22.

In step S102, the extraction unit 26 reads the video stored in the image data storage unit in step S100 and extracts one or more feature parameters from images constituting the video at respective times.

In step S104, the estimation unit 28 estimates a degree of heart failure of the patient on the basis of the one or more feature parameters extracted in step S102 and the computation model stored in the reference data storage unit 24.

In step S106, the estimation unit 28 outputs the degree of heart failure of the patient estimated in step S104 as a result.

The output unit 29 outputs the estimation result from the estimation unit 28. The display device 16 displays the estimation result outputted from the output unit 29. A healthcare worker or user operating the heart failure estimation device 14 checks the estimation result outputted from the display device 16 and the healthcare worker or user checks the degree of heart failure.

As described above, the heart failure estimation device 14 of the heart failure estimation system 10 according to the first exemplary embodiment extracts at least one feature parameter from an image or video obtained by imaging the face of a patient. The heart failure estimation device 14 then estimates a degree of heart failure of the patient on the basis of the extracted at least one feature parameter. Thus, the degree of heart failure of the patient may be estimated from an image or video of the facial region of the patient.

In many patients, heart failure is not simply a poor outcome. Patients with heart failure are intractable chronic patients who go through cycles of remission and progression, and rates of recurrence after leaving the hospital are high. However, even when progression starts, recurrence and progression may be prevented if prompt medical treatment is possible. Even when a patient has left the hospital, prompt discovery of heart failure progression is important for heart failure management. BNP (blood tests), chest X-rays, and echocardiography are widely used for quantitative evaluation of heart failure, but are mainly conducted within the hospital and interpreting their results requires specialists. Frequent testing is impractical because of invasiveness, testing costs, and expertise. Alternative conventional technologies include non-invasive monitoring systems based on wearable or portable sensors of periodic changes in respiration of the patient, heart rate changes, electrocardiograms and the like. However, to acquire estimations of heart failure conditions, each of these technologies requires information collection by installation or attachment of equipment, implantation in the body, or long-term continuous recording for data acquisition. Consequently, there are problems such as effects from artefacts (artificial and mechanical errors) that are caused by body movements, posture changes, contact failures and the like, restrictions on posture, physical burdens due to data acquisition, and so forth. Therefore, no at-home or remote technology is currently available that may overcome the boundaries between specialisms, occupations and the like and identify heart failure disease states (for example, promptly revealing progression or the like) simply, quickly and non-invasively without contact.

Previously, with the aim of quickly perceiving signs of the progression of heart failure at home, there have been efforts to remotely monitor body weight or vital signs. However, these have not been successful in promptly uncovering heart failure progression. Reasons include the heart failure patient internally using various medicines that influence blood activity (for example, medicines that lower blood pressure or reduce the heart rate and medicines that cause water to be excreted out of the body), and the difficulty of perceiving changes in vital signs (for example, blood pressure, pulse and the like). With complex conditions and frailty (frailty here meaning a condition in which muscle amounts or fat amounts decrease and the patient loses weight), there are many patients whose muscle or fat amounts decrease and are replaced by progressive retention of liquid such as phlegm and the like without body weight increasing. Therefore, particularly in a living situation in which access to doctors is limited, it may not be possible for a patient, healthcare worker or the like to notice heart failure progression before the heart failure becomes serious.

In everyday clinical practice, poor health may be observed from changes in the facial expression of a patient even without changes in vital signs, body weight or the like. It is not unusual to notice heart failure progression and, conversely, to verify the efficacy of medical treatment from improvements in the expression of a patient.

The heart failure estimation system according to the present exemplary embodiment has been made in consideration of the circumstances described above and estimates a degree of heart failure of a patient on the basis of one or more feature parameters extracted from an image or video obtained by imaging the face of the patient. Therefore, the degree of heart failure of the patient may be estimated from an image or video of the facial region of the patient.

Thus, information expressing a change in the facial region of a patient is useful for promptly discovering heart failure progression. However, healthcare workers perceiving these changes have individual differences in diagnostic skills, and it is not easy to verbalize and teach these skills. Therefore, if a change can be considered as an objective digital indicator as in the present exemplary embodiment, a new, skill-independent indicator based on evidence with broad general applicability may be widely utilized for heart failure diagnostics without particular specialists, professionals, individuals, locations and the like being selected.

Data that is utilized in the present exemplary embodiment is an image or video of the face of the patient being examined, which is very easy to check on a daily basis. The face of the patient is an area that is exposed even when the patient is wearing clothes or the patient is lying on a bed. Therefore, heart failure states may be continuously evaluated by a common indicator that is a feature obtained from the face even if the patient has left the hospital and is at home, is an outpatient at a doctor who is not a cardiologist, or is in a nursing home or the like. Thus, medical treatment before heart failure progresses is possible in the community.

Because quantitative data on stages of heart failure progression may be gathered after a patient leaves the hospital, data from their home or a facility (for example, a nursing care home or a rehabilitation center) on trends in heart failure progression, treatment progress, stages of recovery and trends in recovery that have not previously been clear may be shared. By evaluating these data together with personal health records of conventional indicators such as vital signs, activity levels and so forth and electronic medical records of conventional heart failure indicators (blood testing, chest X-rays, echocardiograms and the like) acquired by hospitals and clinics, the data may be utilized as common indicators along with personal health records and electronic medical records. This may contribute to further understanding of heart failure conditions and to the development of diagnostics and treatments of heart failure. In addition, an indicator calculated in the present exemplary embodiment is a continuous variable, daily changes may be visualized objectively regardless of who is evaluating them, and states of heart failure (for example, speeds of progress and remission) may be evaluated from degrees of change.

When diagnosing heart failure in patients, blood sampling, X-rays and echocardiography require dedicated technologies and particular testing systems (equipment), and have problems with invasiveness, radiation exposure, testing costs and specialization. As a result, repeated testing is difficult. In particular, because X-rays and echocardiography require particular testing equipment, testing in a clinic, the home or the like is difficult. Because testing of blood samples is often outsourced to external bodies, the results cannot be checked in real time. In addition, interpreting the results of these tests and other heart failure evaluation indicators requires specialization. By contrast, according to the heart failure estimation system of the present exemplary embodiment, a patient's condition may be repeatedly acquired simply, without contact and non-invasively. More specifically, imaging a patient with a camera takes a short time, less than a minute on each occasion, and results relating to a heart failure condition can be automatically determined in real time. The indicators used in the present exemplary embodiment show excellent correspondence with widely used heart failure indicators according to heart failure states (for example, NYHA functional classification, BNP values, changes in body weight, pulmonary congestion and pleural fluid levels in chest X-rays, echocardiography results and so forth), and consider facial changes that are backed up by clinical evidence. Therefore, interpretation of the obtained results is simple and explicable. As a result, physical and mental burdens on both patients and healthcare workers are greatly eased, which is an advantage over other heart failure evaluation indicators.

The heart failure estimation system according to the present exemplary embodiment may be combined with a smartphone app, a smartphone face authentication function, a face authentication or camera function of a computer, an online meeting system, an online remote medicine system, a home security system, a smart home, smart domestic electrical equipment, an in-car camera, a working environment monitoring system, a metaverse, a digital twin and so forth, making monitoring of heart failure states even easier. Thus, medical care for heart failure treatment or prevention may be optimized and personalized.

If heart failure can be evaluated by a smartphone app, a user may reduce risks of heart failure occurring or reoccurring, and may evaluate the chronology of their heart failure condition. The heart failure estimation system according to the present exemplary embodiment may be incorporated in the daily life of a patient or user as a part of their regular activities. This may encourage changes in attitude and behavior of the patient or user, leading to a lifestyle free of the burden of heart failure, and enabling use of the heart failure estimation system as a digital medical device, an educational tool and the like.

= Heart failure estimation system according to second exemplary embodiment =

Now, a second exemplary embodiment is described. Structures of a heart failure estimation system according to the second exemplary embodiment that are constituted as structures the same as in the first exemplary embodiment are assigned the same reference symbols and are not described here.

Fig. 17 illustrates a heart failure estimation system 310 according to the second exemplary embodiment. As shown in Fig. 17, the heart failure estimation system 310 is provided with a user terminal 18 and a heart failure estimation device 314. The heart failure estimation device 314 is provided with a communications unit 30.

The heart failure estimation device 314 of the heart failure estimation system 310 estimates a degree of heart failure of a user on the basis of an image or video of the user that is imaged by the camera 12 provided at the user terminal 18.

The heart failure estimation system 310 according to the second exemplary embodiment is expected to be used, for example, in conditions as illustrated in Fig. 18 and Fig. 19.

In the example in Fig. 18, a healthcare worker H in a hospital or the like operates the heart failure estimation device 314, and a user U who is being examined operates the user terminal 18. The user U images an image or video of themself with the camera 12 of the user terminal 18 that is being operated by the user U. The user terminal 18 transmits the image or video to the heart failure estimation device 314 via the network 19 that is the Internet or the like.

The heart failure estimation device 314 receives the image or video of the user U transmitted from the user terminal 18. On the basis of the received image or video, the heart failure estimation device 314 estimates a degree of heart failure of the user U, and outputs an estimation result to a display unit 315 of the heart failure estimation device 314. The healthcare worker H refers to the estimation result displayed at the display unit 315 of the heart failure estimation device 314 and judges the degree of heart failure of the user U.

In the example in Fig. 19, a user U who is being examined images an image or video of themself with the camera 12 of the user terminal 18 that the user U is operating. The user terminal 18 transmits the image or video to the heart failure estimation device 314 via the network 19 that is the Internet or the like. The heart failure estimation device 314 receives the image or video of the user U transmitted from the user terminal 18. On the basis of the received image or video, the heart failure estimation device 314 estimates a degree of heart failure of the user U and transmits an estimation result to the user terminal 18. The user terminal 18 receives the estimation result transmitted from the information processing device 314 and displays the estimation result at a display unit (not shown in the drawings). The user checks the estimation result and verifies their own degree of heart failure.

The heart failure estimation device 314 executes an information processing routine similar to Fig. 16 described above.

The heart failure estimation device 314 may be located in the Cloud and the heart failure estimation system according to the second exemplary embodiment as described above may use the heart failure estimation device 314 located in the Cloud to estimate a degree of heart failure of a user.

Utilizing the heart failure estimation system according to the second exemplary embodiment enables estimation of heart failure of users even outside the hospital. There are many advantages in enabling estimation of heart failure outside the hospital, providing great social value. For example, once a patient has been diagnosed with heart failure, after the patient has been treated in the hospital and discharged from the hospital, there is a high likelihood of the heart failure progressing or reoccurring outside the hospital (for example, at home, in a different hospital the patient is transferred to, or in a nursing home) and of the patient being repeatedly re-admitted. In this kind of situation, if symptoms of worsening heart failure can be detected at as early a stage as possible and prompt action can be taken, there is hope that re-admission or heart failure progression due to the heart failure worsening can be prevented. If medical treatment can be promptly applied, there is a high likelihood that the patient will recover quickly in spite of the worsening heart failure. Furthermore, when examination in the hospital is difficult because of a natural disaster, an infectious disease epidemic or the like, this technology may be applied to early detection and management of serious illnesses. Generally, in order to discover heart failure, monitoring of heart failure states is necessary, including blood pressure and the pulse of a patient, blood sampling, X-rays, echocardiograms and the like. However, continual monitoring of the condition of a patient is difficult outside the hospital and where medical and human resources are limited. By contrast, the heart failure estimation system according to the present exemplary embodiment enables easy and cheap repeated monitoring of heart failure states, non-invasively and without contact, even for non-healthcare workers regardless of specialism or occupation. Evaluation elements used in the present system are constituted by plural indicators that are unlikely to be affected by ethnic differences such as skin color or by age and so forth. Therefore, the present system may be used worldwide regardless of age and gender. In addition, treatment outcomes for hereditary heart disease have improved in recent years and more hereditary heart disease patients are reaching adulthood. For these patients, the heart failure estimation system according to the present exemplary embodiment enables monitoring of heart failure conditions continuously from early childhood to adulthood.

According to the heart failure estimation system of the present exemplary embodiment, a degree of heart failure of a user may be calculated on the basis of a facial image or video of the user. Therefore, for example, even a user outside the hospital-at home, in a nursing home, in an evacuation center or the like-may check their degree of heart failure. By utilizing the heart failure estimation system according to the present exemplary embodiment, doctors other than cardiologists and other healthcare workers (pharmacologists, nurses, physiotherapists and so forth) or nursing home staff may judge heart failure of users. Therefore, a change in the degree of heart failure of a patient may be detected promptly. The heart failure estimation system according to the present exemplary embodiment may identify heart failure conditions conveniently without particular times and places being selected. Therefore, the system may be used for feedback of "teaching data" and the results evaluated in combination with user symptoms, physical examinations, test findings and so forth. Thus, the present heart failure estimation system may be employed as a teaching tool for improving the health literacy of users and enhancing knowledge of heart failure in the many healthcare workers, nursing care workers and the like who are involved in heart failure diagnoses.

EXAMPLES

Now, Examples are described. In the present Examples, each of a parameter expressing the shape of the face of a patient, a parameter expressing a face vector of the patient, a parameter expressing the eyes of the patient and a parameter expressing the mouth of the patient are used to estimate heart failure by a decision tree algorithm known as LightGBM. Fig. 20 shows an example of results of using LIGHTGBM to estimate degrees of heart failure of patients.

The horizontal axis (x axis) of each graph in Fig. 20 indicates values in the NYHA (New York Heart Association) functional classification, which are described in records of examinations by doctors. The vertical axis (y axis) of each graph indicates values in the NYHA functional classification estimated by the trained LIGHTGBM.

The NYHA functional categories are shown below (excerpted from Japanese Circulation Society/Japanese Heart Failure Socity 2017 Guideline on Diagnosis and Treatment of Acute and Chronic Heart Failure).

NYHA I: Presence of cardiac disease. No limitation of physical activity. No significant fatigue, palpitations, breathing difficulty or anginal pain in ordinary physical activity.

NYHA II: Light or moderate limitation of physical activity. No pain when at rest. Ordinary physical activity causes fatigue, palpitations, breathing difficulty or anginal pain.

NYHA III: High limitation of physical activity. No pain when at rest. Effort of ordinary physical activity or less causes fatigue, palpitations, breathing difficulty or anginal pain.

NYHA IV: Unable to carry on any physical activity without discomfort. Symptoms of heart failure and angina even at rest. If any physical activity is undertaken, discomfort increases.

More specifically, the horizontal axis (x axis) of each graph in Fig. 20 indicates values in the NYHA functional classification when case data evaluated by cardiologists are acquired, and the vertical axis (y axis) of each graph indicates estimated values of the NYHA functional classification estimated by the trained LIGHTGBM. The NYHA classification is usually divided into four categories, but heart failure conditions of patients may be evaluated in ordinary clinical practice as NYHA 1-2 and NYHA 2-3 or the like instead of the four distinct categories. For convenience in Fig. 20, NYHA 1-2 is converted to 1.5, NYHA 2-3 is converted to 2.5, and NYHA 3-4 is converted to 3.5 for the analysis. Accordingly, the horizontal axis (x axis) of each graph in Fig. 20 indicates values in increments of 0.5. Meanwhile, the estimated values of NYHA functional classification on the vertical axis (y axis) of each graph in Fig. 20 are outputted from the trained LIGHTGBM as a continuous variable. Accordingly, these values are displayed as numerical values with decimal places.

The left side of Fig. 20 shows results when LIGHTGBM is trained using data excluding a specific patient case (G-HF023) and then NYHA functional classification values of the learning data are estimated using the trained LIGHTGBM. The graph at the right side of Fig. 20 shows results when the trained LIGHTGBM is used on test data that is the data of the specific patient case G-HF023 and NYHA functional classification values are estimated for the specific patient case G-HF023. Fig. 20 also shows respective mean absolute percentage errors (mape).

As shown at the right side of Fig. 20, even though this patient case G-HF023 has a wide range of values, the results have a very high correlation of R=0.99 with progression of the severity of heart failure. Thus, it can be seen that NYHA functional categories may be estimated.

Fig. 21 is a chart showing levels of importance of the obtained functional parameters in the training of LIGHTGBM. It can be seen that many parameters relating to the shapes of the face, mouth and eyes and to near-infrared analysis contribute to the estimation of NYHA functional categories. Because there are individual differences that make changes in feature parameters of individual patients more obvious and suchlike, further improvements in accuracy can be anticipated from applying weightings to the feature parameters of different features of the face.

Fig. 22-1 depicts a statistical model based on multivariate analysis of the NYHA functional classification using data from 37 patients (case numbers GH-F002 to GH-F038, which are described below). This statistical model uses four factors, emdiff_dod, W80fcnt, Pitch and Area_all, to calculate NYHA prediction expressions for predicting NYHA functional categories. The horizontal axis (x axis) of each graph in Fig. 22-1 indicates NYHA functional categories when case data evaluated by cardiologists are acquired, and the vertical axis (y axis) indicates results of NYHA functional categories estimated with the NYHA prediction expressions of the statistical model. The four graphs of the NYHA prediction expressions show results of NYHA predictions (labelled as prediction results in Fig. 22-1) for, from the left, embdiff_dod, W80fcnt, Pitch and Area_all. The correlation diagram below the four graphs shows a result of using the obtained NYHA prediction expressions to check the correlation between actual NYHA values (the x axis) and predicted NYHA values (the y axis) for a case G-HF001 that was not used in creating the NYHA prediction expressions. As shown in this graph, the prediction expressions calculated from the multivariate analysis model may predict NYHA functional categories with a strong correlation of R=0.88.

Fig. 22-2 and Fig. 22-3 show results when LIGHTGBM is additionally trained on water content in the face according to near-infrared analysis, showing a graph depicting the importances of the obtained feature parameters (excluding a parameter relating to complexion) and results of estimating NYHA functional categories using the data of a particular patient case (G-HF017) as test data.

Fig. 22-2 shows the following feature parameters.

| | |
|---|---|
| embdiff_dod | Distance between face vectors in a period of remission |
| UWAMABUTA1_b70 | Water content estimated by near-infrared illumination |
| m_outline_p | Aspect ratio of the mouth |
| m_angle_p | Left and right corners of the mouth |
| w80fcnt | Sum of durations that the degree of opening of an eye is closed to 80% or less as a proportion of unit time |
| m_width_p | Horizontal width of the mouth |
| EAR | Eye aspect ratio |

It can be seen that the parameters relating to the shapes of the face, the eyes and the mouth and the water content parameter broadly contribute to estimations of the NYHA functional categories, and the NYHA functional categories may be estimated with a very high correlation of R=0.97 with progression of the severity of heart failure, similarly to Fig. 20 and Fig. 21. The straight line shown in Fig. 22-4 corresponds with a prediction expression calculated from the statistical model as in the following expression.

NYHA prediction expression = 4.21+0.083 * Area (the face area) - 0.23*EAR (the degree of opening of the eye) + 0.21 *m_outline (the mouth aspect ratio) + 0.00075*m_grad (corner of the mouth) + 0.025*pitch (facial pitch) + 0.42*w80fcnt (duration an eye is closed) - 2.9*w149 (face width) - 5.5*Adiff(water content) + 4.4*embdiff_dod (face vector)

When constructing the above NYHA prediction expression, data from G-HF012 to G-HF060 are used to create a prediction expression from the data, excluding G-HF017. The prediction expression is verified with the data of G-HF017. The results show a strong correlation between actual values and predicted values of NYHA functional classifications (the correlation coefficient R=0.97).

Fig. 23 is a table showing a comparison between subjective evaluations of degrees of heart failure by specialists and estimated values of degrees of heart failure when determination thresholds are consistently specified for the respective feature parameters.

The first, second and third columns from the left of the subjective evaluation columns show results of evaluation by visual examination of the outline of the face, the eyes and the cheeks, focusing on whether or not there is a change between when a patient is admitted to hospital due to heart failure and when the patient is discharged. A change is marked "1" and no change (or no clear change) is marked "0". When a change is seen in any of these columns, the fourth column from the left of the subjective evaluation columns ("logical OR") is marked "1", and is marked "0" when no change is seen.

The left side column of the Area_all columns in Fig. 23 shows change amounts of a parameter expressing area of the face of the patient between hospital admission due to heart failure and hospital discharge, and the right side column shows results of determining whether or not that parameter has changed by a specified threshold value (=0.06). More specifically, when the change amount is at least the specified threshold value, the presence of a change is marked "1", and when the change amount is less than the threshold value, the lack of change is marked "0" (a similar definition applies to embdiff_dod, which is described below).

The left side column of the embdiff_dod columns in Fig. 23 shows results of a parameter expressing change of a face vector of the patient, and the right side column shows results of determining whether or not that parameter has changed by a specified threshold value (=0.29). The EAR column in Fig. 23 is marked "1" when a parameter expressing the aspect ratio of the eye of the patient has increased between hospital admission due to heart failure and hospital discharge, and is marked "0" when the aspect ratio has decreased or is unchanged.

Adiff relation (body weight) and Adiff relation (BNP) in Fig. 23 show results of threshold determination of correlations between a parameter expressing near-infrared analysis results of water content in the face of the patient with body weight of the patient and with BNP values. When a correlation coefficient is at least 0.6, that column is marked "1", and when the correlation coefficient is less than 0.6, that column is marked "0". The detection confidence column shows the sum of results (maximum value 5) of the above threshold determinations (Area_all, embdiff_dod, EAR, Adiff relation (body weight) and Adiff relation (BNP)). Greater values indicate that a change from before to after heart failure treatment was detected from the feature parameters.

As shown in Fig. 23, it is seen that judgment results from subjective evaluations and the feature parameters match up in many of the cases. It is also clear that changes associated with heart failure that are not perceived in the subjective evaluations are sensitively detected by the feature parameters. For example, facial changes associated with heart failure may not be perceived at all in visual examinations in the cases G-HF002, G-HF004, G-HF010, G-HF011, G-HF014, G-HF019, G-HF020, G-HF022, G-HF024 and G-HF025, but meaningful changes are uncovered by plural parameters among Area_all, embdiff_dod, EAR and the Adiff relationships (Fig. 23). In these cases, it is clear that some of the feature parameters show strong correlations (Fig. 24 to 26, described below) with the NYHA functional classification, body weight and BNP (brain natriuretic peptide). Therefore, as well as providing objective support for subjective evaluations of heart failure by cardiologists that are interpreted from visual examinations, the present heart failure estimation system may use many parameters for multifaceted detection of changes that are not perceived in visual examinations. In particular, if accuracy is depicted quantitatively, changes that may be recognized by doctors may be reinforced to 100% by some of the feature parameters. In addition, slight signs may not be discerned by the human eye but produce many false positives if sensitivity is adjusted. These signs may be uncovered by some of the feature amounts instead of being missed.

Fig. 24 to Fig. 26 are tables showing correlations of the feature parameters with the NYHA functional classification, body weight and BNP (brain natriuretic peptide), which are widely used as indicators expressing degrees of heart failure.

Fig. 24 shows correlations between NYHA functional classification and the feature parameters. The leftmost column shows case identification numbers, and the third to eleventh columns from the left list correlations between the respective feature parameters and the NYHA functional classification. The empty cells are for unmeasured parameters. Where relationships with heart failure conditions are theoretically in a suitable direction, the cells are shaded where the correlation is at least moderate (a correlation of at least 0.6 when a positive relationship is seen and a correlation of at most -0.6 when a negative relationship is seen). For example, face width and area are theoretically increased by heart failure progression and are expected to exhibit a positive correlation. Total numbers of the feature parameters that show at least a moderate correlation are listed as "score" in the second column. For example, in case G-HF001 eight parameters-a face vector parameter, a parameter relating to the mouth, two parameters relating to the eyes, the facial pitch, two face width parameters, and a face area parameter-have relationships with high correlations of at least 0.6 with the NYHA functional classification. Thus, the total number of parameters with correlations of at least 0.6, eight, is listed in the score column.

Similarly, Fig. 25 shows total numbers of feature parameters with high correlations with body weight and Fig. 26 shows total numbers of feature parameters with high correlations with BNP. The empty cells show parameters that could not yet be measured and evaluated.

As shown in Fig. 24 to Fig. 26, many of the feature parameters can be seen to have moderate or strong correlations with the NYHA functional classification, body weight and BNP. It should be mentioned that even in cases in which the total number of moderate or higher correlations is small, a strong relationship with one of the feature parameters can be seen. Thus, the present heart failure estimation system that estimates multiple parameters gives results with a reduced risk of overlooking a progression of heart failure. For example, in case G-HF036, the parameters relating to facial shape, the mouth, the eyes, facial pitch, face area and width have lower than moderate correlations with the NYHA functional classification, body weight and BNP, but the near-infrared parameter shows strong correlations with the NYHA functional classification, body weight and BNP. Thus, accurate perception of the heart failure condition is achieved (and a progression of heart failure is not overlooked). Similarly in case G-HF013, the feature parameters relating to the mouth, the eyes, facial pitch, near-infrared, face area and width have lower than moderate correlations with the NYHA functional classification and body weight, but the face vector parameter shows strong correlations with the NYHA functional classification and body weight. Thus, in the present heart failure estimation system the plural feature parameters sensitively reflect heart failure states, and degrees of heart failure may be accurately perceived by the feature parameters working together.

Fig. 27-1 to Fig. 27-3 are diagrams showing correlations between the feature parameters and the NYHA functional classification, body weight and BNP when the results of 30 cases shown in Fig. 24 to Fig. 26 are increased to 60 cases and three-dimensional data of faces, as illustrated in Fig. 11-2, are used to calculate the feature parameters. The empty cells show parameters that could not yet be measured and evaluated. As shown in Fig. 27-1 to Fig. 27-3, correlations between the feature parameters and the NYHA functional classification, body weight and BNP are measured even though the number of cases is increased.

Relationships were confirmed between these feature parameters and degrees of pulmonary congestion and pleural fluid levels in chest X-rays and echocardiography, which are established heart failure evaluation indicators. For example, in case G-HF009 (the upper graphs of Fig. 27-4), as the pulmonary congestion scores and pleural fluid scores indicated on the x axes fall (the lower a score value, the better the pulmonary congestion or pleural fluid level, with zero representing a state in which there is no pulmonary congestion or pleural fluid and the heart failure has been corrected), the NYHA functional classification that is the heart failure symptom indicator shown on the y axes also improves (the lower the category, the milder the symptoms, with category I representing the state in which the heart failure has been corrected and there are no symptoms). Similarly (in the middle graphs and lower graphs of Fig. 27-4), as the pulmonary congestion scores and pleural fluid scores indicated on the x axes fall (improve), the indicator of degrees of opening of the eyes EAR increases (improves) and the facial width decreases (edema improves). These have at least moderate correlations.

Similarly (in Fig. 27-5), as the pulmonary congestion scores and pleural fluid scores fall (improve), the near-infrared indicator Adiff expressing water content increases (the water content decreases) and the indicator of change in the face vector embdiff_dod falls (the heart failure improves).

It is also confirmed that the feature parameters show at least moderate relationships with echocardiography results. For example, Fig. 27-6 shows correlations for case G-HF017 between the indicator of changes in the face vector embdiff_dod and the indicator of degrees of opening of the eyes EAR, which are indicated on the y axes, and an E/A ratio and IVC diameter (inferior vena cava, mm) from echocardiography, which are indicated on the x axes. The E/A ratio is an indicator of enlargement of the heart, which is calculated from a blood flow velocity waveform of the mitral valve (transmitral flow (TMF)). The E wave is an early relaxation wave (E wave) produced by the bicuspid valve opening and an active blood flow from the left atrium to the left ventricle in the early relaxation period. The A wave is an atrial contraction wave (A wave) produced by a blood flow from the left atrium to the left ventricle that is caused by atrial contraction in the late relaxation period. The TMF in middle-aged and older people generally has an E/A ratio of 1 or less, but when heart failure progresses, natural blood flows are inhibited by obstructions in the left ventricle and a proportion of blood flowing in due to atrial contraction increases, as a result of which the A wave increases further (the E/A ratio falls further). In contrast, when the heart failure condition improves due to heart failure treatment, relaxation of the heart improves, the proportion of blood naturally flowing from the left atrium into the left ventricle increases, and the proportional contribution of atrial contraction falls. As a result, the E/A ratio tends to increase. Meanwhile, the IVC diameter indicates a degree of water retention within the body, particularly within blood vessels. The IVC diameter increases when heart failure progresses and decreases when heart failure is in remission. In case G-HF017, the E/A ratio of the TMF increases and the IVC diameter decreases when the heart failure improves. In association therewith, the indicator of change in the face vector embdiff_dod decreases (improves) and the indicator of the degree of opening of the eyes EAR increases (improves). In addition, it is confirmed that feature parameters obtained from images or videos of the face relate with an E/e' ratio obtained by echocardiography (a value in which a mitral valve E wave velocity is divided by a mitral annulus velocity e') and with a tricuspid regurgitation velocity and so forth.

Thus, it is confirmed that the feature parameters obtained from an image or video of a face relate with heart failure symptoms with clinical evidence (the NYHA functional classification), increases in body weight and edema, blood test findings (BNP values), chest X-ray findings and echocardiography findings, which are established heart failure evaluation indicators. The parameters obtained from an image or video of the face may clinically explain the obtained results as specific indicators of heart failure and contribute to building an explicable AI.

Therefore, the present disclosure may provide a heart failure estimation device, a heart failure estimation system, a heart failure estimation method and a heart failure estimation program that may estimate a degree of heart failure of a patient from a still image or video image of a facial region of the patient conveniently and quickly, without contact and non-invasively. The present disclosure may solve the problems of conventional technology, such as requiring equipment installations and technologies necessary for measurement, artefacts associated with body movements, posture and contacts, the physical and economic burdens associated with monitoring, and so forth. According to the present disclosure, a degree of heart failure of a patient may be estimated from an image or video image of the facial region of the patient. Therefore, effects are provided in that making the installation of equipment for measurement unnecessary, avoiding measurement and evaluation errors associated with body movements, posture and contacts, and reducing burdens on the patient may all be achieved at the same time. Moreover, the present disclosure translates subjective evaluations, meaning the feelings and intuitions of experienced doctors, into evaluable indicators that can be objectively evaluated. Therefore, an effect is provided in that changes in condition associated with heart failure that are not perceived by conventional heart failure indicators may be sensitively detected.

The technology of the present disclosure is not limited by the exemplary embodiment described above; numerous modifications and applications are possible within a scope not departing from the gist of the disclosure.

In the exemplary embodiment described above, an example is described in which plural feature parameters are utilized, but this is not limiting. For example, one or more of the plural feature parameters may be used to estimate a degree of heart failure.

A trained model or statistical model that is employed when estimating a degree of heart failure may be a model that is prepared in advance for patients with respective attributes (separated by age, by sex, by disease and the like). For example, a trained model or statistical model for men in their twenties, a trained model or statistical model for women in their twenties, a trained model or statistical model for men in their thirties, a trained model or statistical model for patients with respiratory disease, a trained model or statistical model for patients in dialysis, and so forth can be prepared in advance. For example, when a patient is a man in their twenties, a trained model or statistical model for men in their twenties is used to estimate the degree of heart failure. Thus, degrees of heart failure may be accurately estimated in accordance with the attributes of patients.

A degree of heart failure progression in a patient may be estimated on the basis of chronological data of scores expressing degrees of heart failure or chronological data of one or more feature parameters.

For example, Fig. 27-7 shows an example of a chronological trend of a near-infrared parameter relating to heart failure progression after discharge from the hospital. In the illustrated example, the near-infrared parameter Adiff expressing water content falls greatly due to heart failure treatment between hospital admission and hospital discharge, the body weight falls from 78 kg at admission to 57 kg at discharge, BNP values that are blood sample data expressing degrees of heart failure fall from 1788 pg/ml at admission to 282 pg/ml at discharge, and the NYHA functional classification that is an indicator of heart failure symptoms improves from NYHA IV to NYHA I. However, further progression of the heart failure is seen in the out-patient after discharge, with the body weight rising again to 63 kg (+7 kg), the BNP value rising to 1494 pg/ml, the NYHA functional classification being 1-2, and moderate shortness of breath occurring. The indicator Adiff of water content acquired from facial images in this period rises again, confirming that the progression of heart failure is accurately perceived. Subsequently, further heart failure treatment is applied to the patient as an out-patient, all of the heart failure indicators (body weight, BNP values and NYHA functional classification) improve, and a fall in the water content indicator Adiff is confirmed. Thereafter, the heart failure condition proceeds to be stable, and the water content indicator Adiff similarly confirms the stable trend. In the same case (Fig. 27-8), the face vector parameter embdif_dod and the eye shape parameter EAR show heart failure improvements due to the in-patient treatment, with embdiff_dod (the left side y axis and square marks in Fig. 27-8) falling (the time of hospital discharge is the reference, so differences in the face vector seen between discharge and admission improve) and EAR (the right side y axis in Fig. 27-8 and the round markers) increasing (the degree of opening of the eye improves). When the heart failure progresses outside the hospital, emdiff_dod increases again (differences in the face vector from the time of hospital discharge reappear), and EAR falls (the degree of opening of the eye falls again). Thus, the feature parameters used in the present heart failure estimation system show not just changes in heart failure while in the hospital but also sensitively perceive heart failure changes outside the hospital.

During heart failure progression, enteric edema, loss of appetite and the like occur, a decline in the state of nutrition progresses, and muscle mass and fat quantities often decrease, being replaced with water. Therefore, when water is eliminated by medical treatment, the body weight deteriorates greatly till hospital discharge, causing a state in which "malnutrition" is apparent (the face is narrow and the the cheeks are drawn in). Subsequently, provided the heart failure is managed, water retention is not observed and muscle and fat are recovered, and the state of nutrition improves. The present system may perceive not just changes in heart failure states that reflect changes in water content due to water retention during heart failure progression and water being eliminated during emergency heart failure treatment but also complex changes in heart failure condition, such as improvements in the state of nutrition over a sustained period of heart failure and the like. For example, in the graphs shown in Fig. 27-8 of trends in the parameters expressing changes in the face vector and degrees of opening of the eye (embdiff_dod and EAR), these changes in the period of heart failure progression just after hospital discharge (increases in water retention) are sensitively perceived, and in an acute or sustained period after a subsequent period of remission, reflect improvements in the heart failure condition (a change from the malnutrition state to a "plump" state). Changes occur in the shape of the face that are different from the time of hospital discharge and are sustained. In practice, the aforementioned parameter Adiff expressing water content in the face reflects heart failure remission and subsequent stabilization and transition. It may be confirmed that changes in the shape of the face observed over an acute or sustained period are due not only to the water retention associated with heart failure but are also caused by improvements and changes in the state of nutrition. Thus, cleverly interpreting combinations of the plural feature parameters enables evaluations of not just heart failure states but also the frailty and states of nutrition that are intimately related with heart failure.

Fig. 27-9 is an example of chronological trends in the parameters Adiff and EAR expressing water content in the face. Fig. 27-10 is an example of chronological trends in the parameter Adiff expressing water content in the face and the parameter embdiff_dod expressing changes in the face vector. Fig. 27-11 is an example of chronological trends in the parameter Adiff and a face width w14-9 expressing water content in the face. As can be seen in Fig. 27-9, Fig. 27-10 and Fig. 27-11, chronological trends in the parameter Adiff expressing water content in the face are seen to relate with chronological trends in the respective parameters.

Similarly, as shown in Fig. 28, a graph may be constructed in which the horizontal axis (x axis) shows date and time and the vertical axis (y axis) shows a score expressing degrees of heart failure. In this graph, the smaller a value on the vertical axis (y axis), the more severe (further progressed) the disease. Where the trend is steep, the disease state (speed of progression) is severe (or developing quickly). For example, the scores expressing degrees of heart failure of a patient A in whom the progression of heart failure is small and development is steady are shown by the solid line, and the scores expressing degrees of heart failure in a patient B in whom the disease is rapidly worsening are shown by the broken line.

Chronological graphs may be created in this way and degrees of heart failure progression in patients may be estimated on the basis of these graphs. Alternatively, values of a feature parameter may be plotted on a vertical axis (y axis) and degrees of heart failure of a patient estimated from chronological data. In particular, the parameters employed in the present heart failure estimation system are continuous variables and illness states such as heart failure progression, remission or the like may be estimated from changes within a day or between days. For example, changes in day-by-day values of the parameter embdiff_dod in Fig. 6-1A and Fig. 6-1B are shown chronologically, and how quickly heart failure is progressing or remitting may be perceived from gradients of the trends in Fig. 6-1A and Fig. 6-1B. When this information is taken into account, more appropriate warnings may be given during heart failure progression.

In the exemplary embodiment described above, an example is described in which various kinds of machine learning-FACEMESH, FACENET and LIGHTGBM-are utilized, but this is not limiting. Models are not limited to machine learning models and alternative models may be utilized. For example, other examples of applicable regression models may employ techniques such as Ridge regression, Lasso, Elastic-Net, Least-angle regression (LARS), LARS Lasso, Orthogonal Matching Pursuit (OMP), Bayesian Regression, Logistic regression and Random Forest regression.

In the exemplary embodiment described above, an example is described in which FACEMESH is utilized to extract 468 feature points from a facial image, but this is not limiting. Any number of feature points may be extracted from the facial image.

As an example, the Description of the present Application describes exemplary embodiments in which a program is installed in advance, but the program may be stored and provided on a computer-readable recording medium.

The processing that, in the exemplary embodiment described above, is executed by a CPU reading software (a program) may be executed by various kinds of processor other than a CPU. Examples of processors in these cases include a PLD (programmable logic device) in which a circuit configuration can be modified after manufacturing, such as an FPGA (field-programmable gate array) or the like, a dedicated electronic circuit which is a processor with a circuit configuration that is specially designed to execute specific processing, such as an ASIC (application-specific integrated circuit) or the like, and so forth. A general-purpose graphics processing unit (GPGPU) may also be used as a processor. The processing may be executed by one of these various kinds of processors, and may be executed by a combination of two or more processors of the same or different kinds (for example, plural FPGAs, a combination of a CPU with an FPGA, or the like). Hardware structures of these various kinds of processors are, to be more specific, electronic circuits combining circuit components such as semiconductor components and the like.

In the exemplary embodiment described above, a mode is described in which the program is memorized in advance (installed) at the storage, but this is not limiting. The program may be provided in a mode that is recorded at a non-transitory recording medium such as a CD-ROM (compact disc read-only memory), DVD-ROM (digital versatile disc read-only memory), USB (universal serial bus) memory or the like. Modes are also possible in which the program is downloaded from external equipment via a network.

The processes of the present exemplary embodiment may be configured by a computer, server or the like equipped with a general-purpose arithmetic processing unit and a memory device or the like, and the processes may be executed by a program. This program may be memorized at a memory device, may be recorded on a recording medium such as a magneto-optic disc, an optical disc, a semiconductor memory or the like, and may be provided through a network. Clearly, any other structural elements need not be implemented by a single computer, server or the like but may be distributed and realized at plural computers connected by a network.

- Supplementary Notes -

Supplementary notes on modes of the present disclosure are given below.

- Supplementary note 1 -

A heart failure estimation device includes:

an acquisition unit that acquires an image or video obtained by imaging the face of a patient;
an extraction unit that extracts at least one feature parameter from the image or video acquired by the acquisition unit;
an estimation unit that estimates a degree of heart failure of the patient on the basis of the at least one feature parameter extracted by the extraction unit; and
an output unit that outputs a result estimated by the estimation unit.

- Supplementary note 2 -

In the heart failure estimation device according to supplementary note 1, on the basis of the at least one feature parameter extracted by the extraction unit and a trained model that is trained by machine learning in advance, the estimation unit inputs the at least one feature parameter into the trained model, acquires a score expressing the degree of heart failure of the patient that is outputted from the trained model, and estimates the degree of heart failure on the basis of the score.

- Supplementary note 3 -

In the heart failure estimation device according to supplementary note 1, on the basis of the at least one feature parameter extracted by the extraction unit and a statistical model created in advance, the estimation unit inputs the at least one feature parameter into the statistical model as an explanatory variable, acquires a response variable of the statistical model as a score expressing the degree of heart failure of the patient, and estimates the degree of heart failure on the basis of the score.

- Supplementary note 4 -

In the heart failure estimation device according to any one of supplementary notes 1 to 3, the at least one feature parameter includes at least one of a parameter relating to shape of the face of the patient, a parameter relating to complexion of the patient, a parameter relating to an eye of the patient, a parameter relating to the mouth of the patient, a parameter expressing a result of near-infrared analysis of water content of the face of the patient, and a parameter expressing three-dimensional shape data of the face of the patient.

- Supplementary note 5 -

In the heart failure estimation device according to supplementary note 4, the parameter relating to shape of the face of the patient includes at least one of a parameter expressing an area of the face, a parameter expressing a horizontal direction width of the face, a parameter expressing an inclination of the face, and a parameter expressing a change in a face vector expressing features of the face.

- Supplementary note 6 -

In the heart failure estimation device according to supplementary note 4, the parameter relating to complexion of the patient includes at least one of a parameter expressing hue of the face of the patient, and a parameter expressing greenness of the face of the patient.

- Supplementary note 7 -

In the heart failure estimation device according to supplementary note 4, the parameter relating to an eye of the patient includes at least one of a parameter expressing an aspect ratio of the eye of the patient, a parameter expressing a difference between aspect ratios of the left and right eyes of the patient, a parameter expressing a duration the eye is closed in a single blink, a parameter expressing a proportion of a unit time that the eye is closed, a parameter expressing an interval between blinks, a parameter expressing a number of blinks per unit time, a parameter expressing a speed when the eye closes, a parameter expressing a speed when the eye opens, a parameter expressing a length of time the eye is closed, a parameter expressing an average speed during opening and closing of the eye, and a parameter expressing a vertical direction inclination of the face.

- Supplementary note 8 -

In the heart failure estimation device according to supplementary note 4, the parameter relating to the mouth of the patient includes at least one of a parameter expressing an aspect ratio of the mouth of the patient, a parameter expressing a degree of opening of the mouth of the patient, a parameter expressing the left corner of the mouth, and a parameter expressing the right corner of the mouth.

- Supplementary note 9 -

In the heart failure estimation device according to supplementary note 2, the trained model is a model trained in advance for patients with respective attributes.

- Supplementary note 10 -

In the heart failure estimation device according to supplementary note 3, the statistical model is a statistical model created in advance for patients with respective attributes.

- Supplementary note 11 -

In the heart failure estimation device according to supplementary note 2 or supplementary note 3, the estimation unit estimates a degree of progression of heart failure of the patient on the basis of chronological data of the score or chronological data of the at least one feature parameter.

- Supplementary note 12 -

In the heart failure estimation device according to supplementary note 2 or supplementary note 3, the extraction unit extracts feature points indicating the positions of points in the face from the image or video acquired by the acquisition unit, and extracts the at least one feature parameter on the basis of the feature points.

- Supplementary note 13 -

A heart failure estimation system includes a user terminal provided with a camera, and the heart failure estimation device according to any one of supplementary notes 1 to 3, in which heart failure estimation system:

the user terminal acquires the image or video with the camera and transmits the image or video to the heart failure estimation device;
the acquisition unit of the heart failure estimation device acquires the image or video transmitted from the user terminal; and
the estimation unit of the heart failure estimation device estimates the degree of heart failure of the patient on the basis of the image or video.

- Supplementary note 14 -

In the heart failure estimation device according to any one of supplementary notes 1 to 3, the at least one feature parameter includes a parameter relating to shape of the face of the patient.

- Supplementary note 15 -

In the heart failure estimation device according to any one of supplementary notes 1 to 3, the at least one feature parameter includes a parameter relating to complexion of the patient.

- Supplementary note 16 -

In the heart failure estimation device according to any one of supplementary notes 1 to 3, the at least one feature parameter includes a parameter relating to an eye of the patient.

- Supplementary note 17 -

In the heart failure estimation device according to any one of supplementary notes 1 to 3, the at least one feature parameter includes a parameter relating to the mouth of the patient.

- Supplementary note 18 -

In the heart failure estimation device according to any one of supplementary notes 1 to 3, the at least one feature parameter includes a parameter expressing a result of near-infrared analysis of water content of the face of the patient.

- Supplementary note 19 -

In the heart failure estimation device according to any one of supplementary notes 1 to 3, the at least one feature parameter includes a parameter expressing three-dimensional shape data of the face of the patient.

- Supplementary note 20 -

A heart failure estimation method includes a computer executing processing including:

acquiring an image or video obtained by imaging the face of a patient;
extracting at least one feature parameter from the acquired image or video; and
estimating a degree of heart failure of the patient on the basis of the extracted at least one feature parameter.

- Supplementary note 21 -

A heart failure estimation program causes a computer to execute processing including:

acquiring an image or video obtained by imaging the face of a patient;
extracting at least one feature parameter from the acquired image or video; and
estimating a degree of heart failure of the patient on the basis of the extracted at least one feature parameter.

The disclosures of Japanese Patent Application No. 2023-019491 filed February 10, 2023 are incorporated into the present specification by reference in their entirety. All references, patent applications and technical specifications cited in the present specification are incorporated by reference into the present specification to the same extent as if the individual references, patent applications and technical specifications were specifically and individually recited as being incorporated by reference.

## Claims

**1.** A heart failure estimation device comprising:

an acquisition unit that acquires an image or video obtained by imaging the face of a patient;
an extraction unit that extracts at least one feature parameter from the image or video acquired by the acquisition unit;
an estimation unit that estimates a degree of heart failure of the patient on the basis of the at least one feature parameter extracted by the extraction unit; and
an output unit that outputs a result estimated by the estimation unit.

**2.** The heart failure estimation device according to claim 1, wherein, on the basis of the at least one feature parameter extracted by the extraction unit and a trained model that is trained by machine learning in advance,
the estimation unit inputs the at least one feature parameter into the trained model, acquires a score expressing the degree of heart failure of the patient that is outputted from the trained model, and estimates the degree of heart failure on the basis of the score.

**3.** The heart failure estimation device according to claim 1, wherein, on the basis of the at least one feature parameter extracted by the extraction unit and a statistical model created in advance,
the estimation unit inputs the at least one feature parameter into the statistical model as an explanatory variable, acquires a response variable of the statistical model as a score expressing the degree of heart failure of the patient, and estimates the degree of heart failure on the basis of the score.

**4.** The heart failure estimation device according to any one of claims 1 to 3, wherein the at least one feature parameter includes at least one of

a parameter relating to shape of the face of the patient,
a parameter relating to complexion of the patient,
a parameter relating to an eye of the patient,
a parameter relating to the mouth of the patient,
a parameter expressing a result of near-infrared analysis of water content of the face of the patient, and
a parameter expressing three-dimensional shape data of the face of the patient.

**5.** The heart failure estimation device according to claim 4, wherein the parameter relating to shape of the face of the patient includes at least one of

a parameter expressing an area of the face,
a parameter expressing a horizontal direction width of the face,
a parameter expressing an inclination of the face, and
a parameter expressing a change in a face vector expressing features of the face.

**6.** The heart failure estimation device according to claim 4, wherein the parameter relating to complexion of the patient includes at least one of

a parameter expressing hue of the face of the patient, and
a parameter expressing greenness of the face of the patient.

**7.** The heart failure estimation device according to claim 4, wherein the parameter relating to an eye of the patient includes at least one of

a parameter expressing an aspect ratio of the eye of the patient,
a parameter expressing a difference between aspect ratios of the left and right eyes of the patient,
a parameter expressing a duration the eye is closed in a single blink,
a parameter expressing a proportion of a unit time that the eye is closed,
a parameter expressing an interval between blinks,
a parameter expressing a number of blinks per unit time,
a parameter expressing a speed when the eye closes,
a parameter expressing a speed when the eye opens,
a parameter expressing a length of time the eye is closed,
a parameter expressing an average speed during opening and closing of the eye, and
a parameter expressing a vertical direction inclination of the face.

**8.** The heart failure estimation device according to claim 4, wherein the parameter relating to the mouth of the patient includes at least one of

a parameter expressing an aspect ratio of the mouth of the patient,
a parameter expressing a degree of opening of the mouth of the patient,
a parameter expressing the left corner of the mouth, and
a parameter expressing the right corner of the mouth.

**9.** The heart failure estimation device according to claim 2, wherein the trained model is a model trained in advance for patients with respective attributes.

**10.** The heart failure estimation device according to claim 3, wherein the statistical model is a statistical model created in advance for patients with respective attributes.

**11.** The heart failure estimation device according to claim 2 or claim 3, wherein the estimation unit estimates a degree of progression of heart failure of the patient on the basis of chronological data of the score or chronological data of the at least one feature parameter.

**12.** The heart failure estimation device according to claim 2 or claim 3, wherein the extraction unit

extracts feature points indicating the positions of points in the face from the image or video acquired by the acquisition unit, and
extracts the at least one feature parameter on the basis of the feature points.

**13.** heart failure estimation system comprising

a user terminal provided with a camera, and
the heart failure estimation device according to any one of claims 1 to 3,

wherein:

the user terminal acquires the image or video with the camera and transmits the image or video to the heart failure estimation device;
the acquisition unit of the heart failure estimation device acquires the image or video transmitted from the user terminal; and
the estimation unit of the heart failure estimation device estimates the degree of heart failure of the patient on the basis of the image or video.

**14.** The heart failure estimation device according to any one of claims 1 to 3, wherein the at least one feature parameter includes a parameter relating to shape of the face of the patient.

**15.** The heart failure estimation device according to any one of claims 1 to 3, wherein the at least one feature parameter includes a parameter relating to complexion of the patient.

**16.** The heart failure estimation device according to any one of claims 1 to 3, wherein the at least one feature parameter includes a parameter relating to an eye of the patient.

**17.** The heart failure estimation device according to any one of claims 1 to 3, wherein the at least one feature parameter includes a parameter relating to the mouth of the patient.

**18.** The heart failure estimation device according to any one of claims 1 to 3, wherein the at least one feature parameter includes a parameter expressing a result of near-infrared analysis of water content of the face of the patient.

**19.** The heart failure estimation device according to any one of claims 1 to 3, wherein the at least one feature parameter includes a parameter expressing three-dimensional shape data of the face of the patient.

**20.** heart failure estimation method comprising a computer executing processing including:

acquiring an image or video obtained by imaging the face of a patient;
extracting at least one feature parameter from the acquired image or video; and
estimating a degree of heart failure of the patient on the basis of the extracted at least one feature parameter.

**21.** A heart failure estimation program for causing a computer to execute processing comprising:

acquiring an image or video obtained by imaging the face of a patient;
extracting at least one feature parameter from the acquired image or video; and
estimating a degree of heart failure of the patient on the basis of the extracted at least one feature parameter.

FIG.1

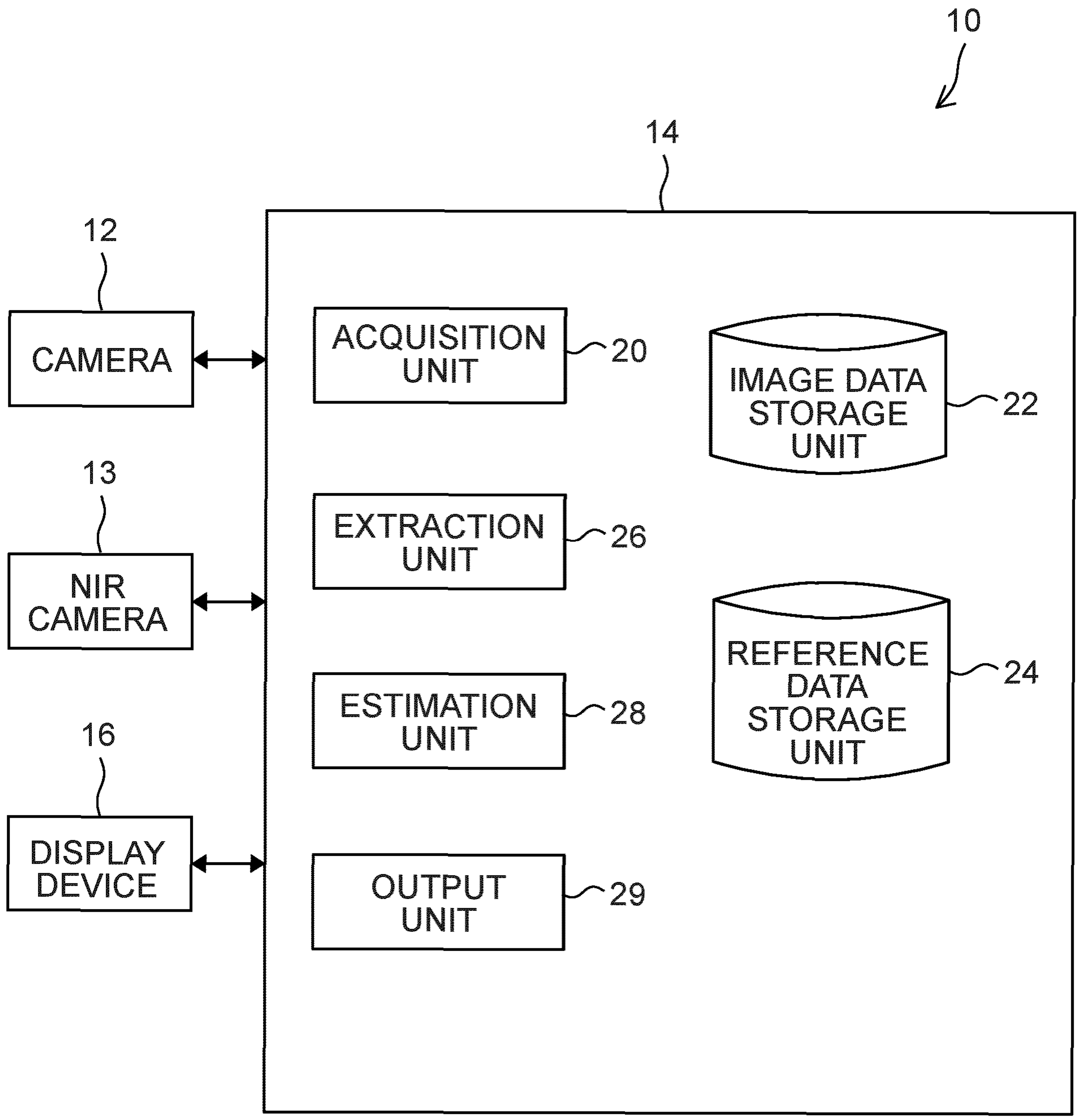
10
14
12
CAMERA
13
NIR CAMERA
16
DISPLAY DEVICE
ACQUISITION UNIT
20
EXTRACTION UNIT
26
ESTIMATION UNIT
28
OUTPUT UNIT
29
IMAGE DATA STORAGE UNIT
22
REFERENCE DATA STORAGE UNIT
24

FIG.2

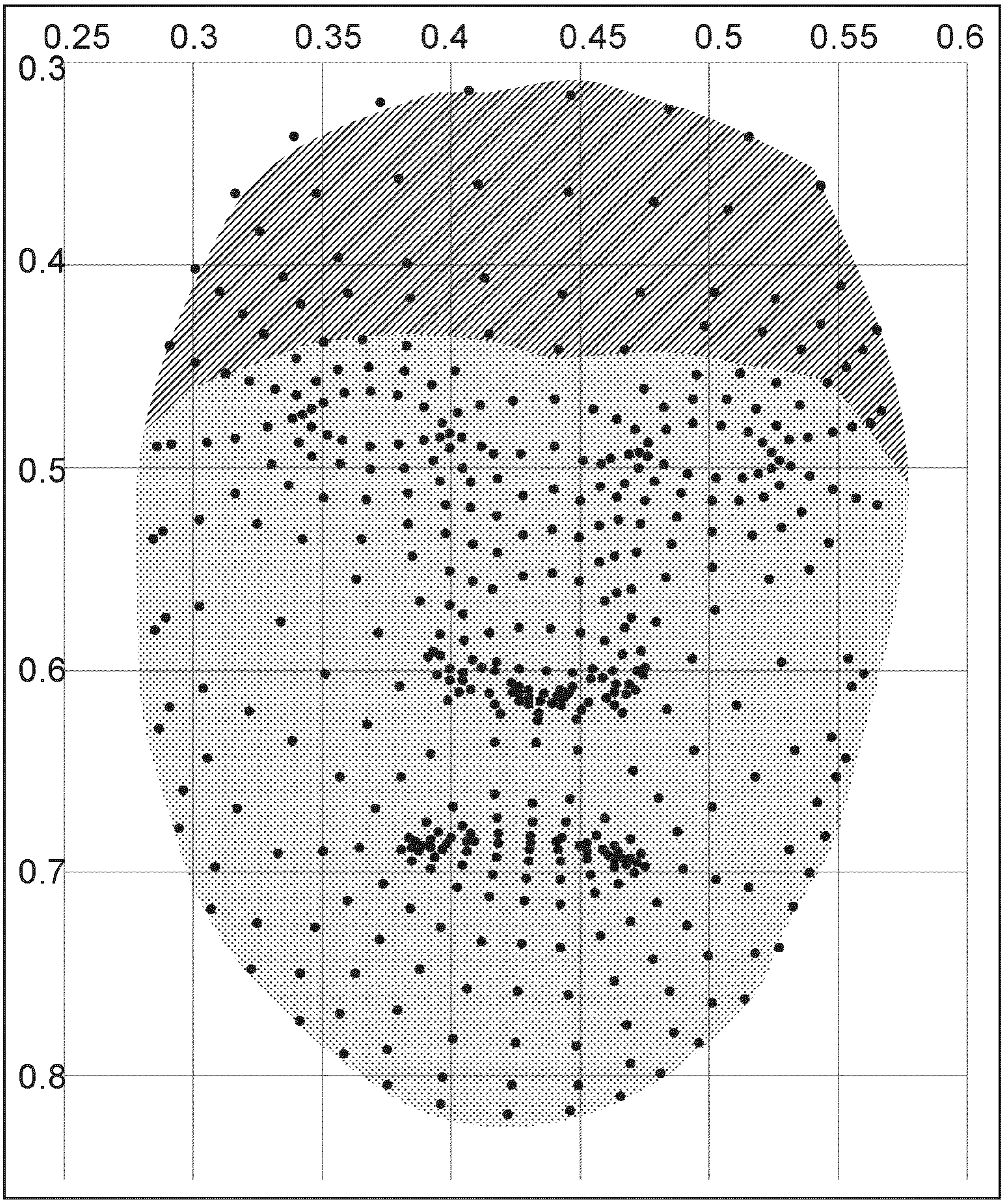

FIG.3

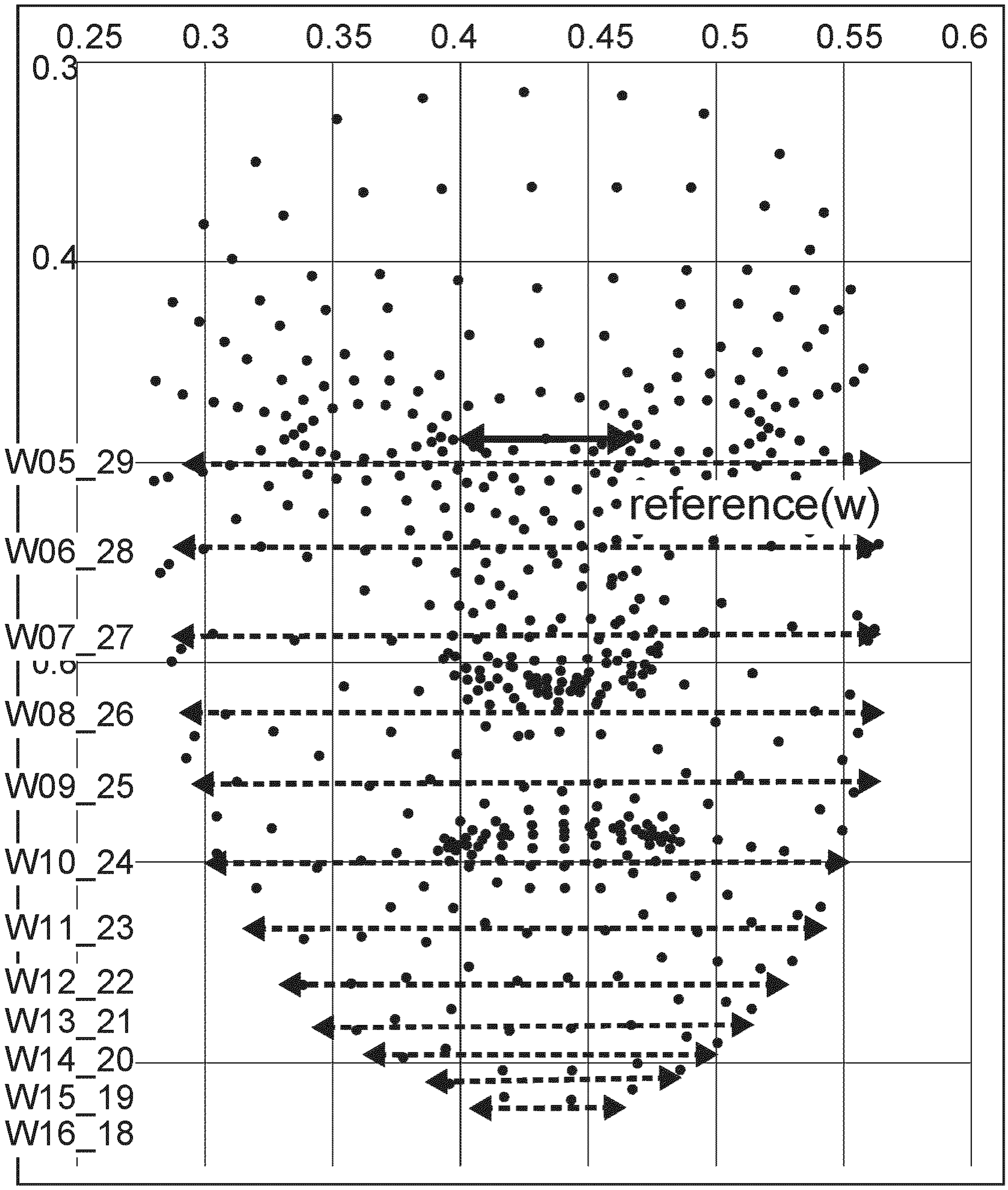
0.25
0.3
0.35
0.4
0.45
0.5
0.55
0.6
0.3
0.4
W05_29
reference(w)
W06_28
W07_27
W08_26
W09_25
W10_24
W11_23
W12_22
W13_21
W14_20
W15_19
W16_18

FIG.4

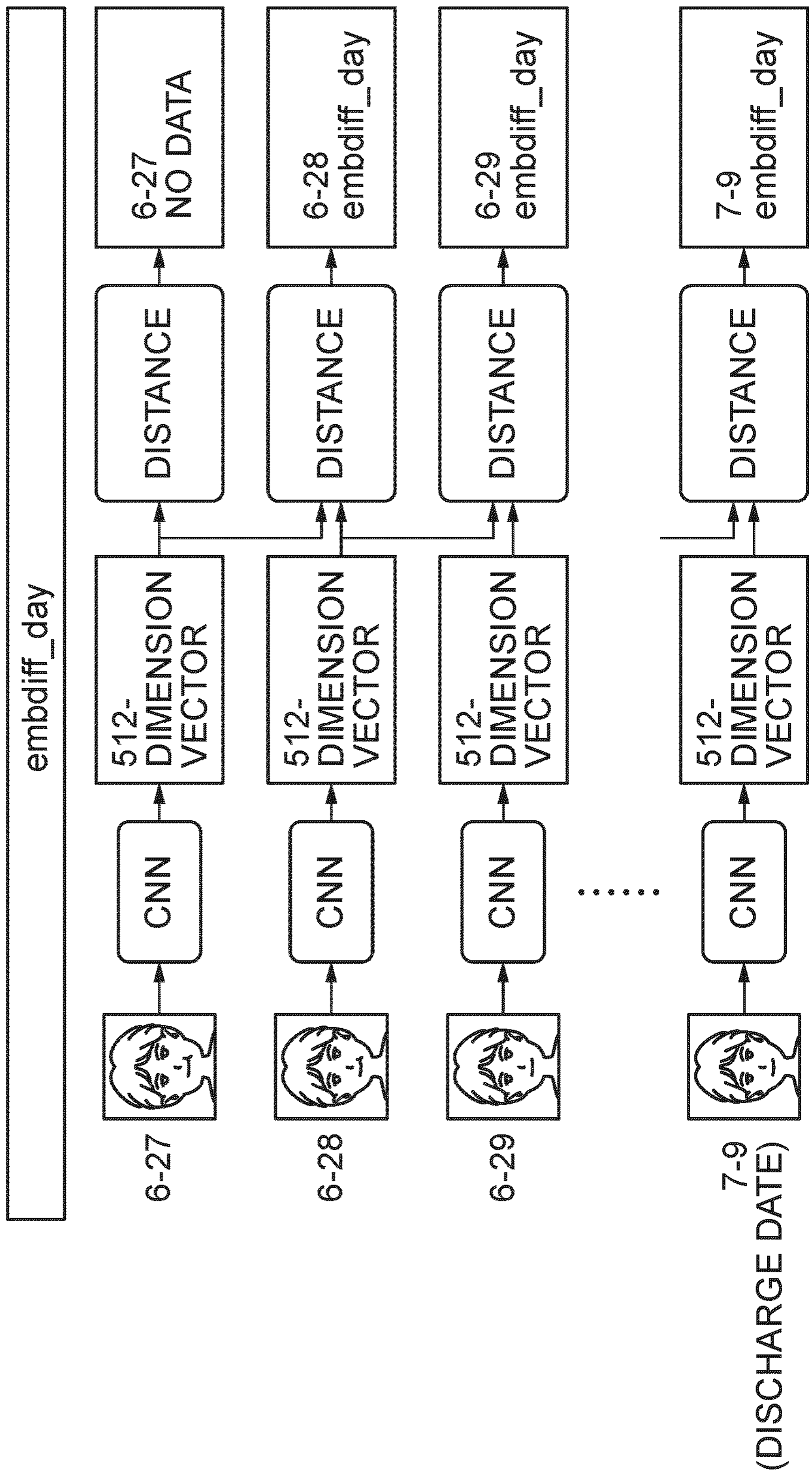
embdiff_day
6-27
6-28
6-29
7-9
(DISCHARGE DATE)
CNN
CNN
CNN
CNN
512-
DIMENSION
VECTOR
512-
DIMENSION
VECTOR
512-
DIMENSION
VECTOR
512-
DIMENSION
VECTOR
DISTANCE
DISTANCE
DISTANCE
DISTANCE
6-27
NO DATA
6-28
embdiff_day
6-29
embdiff_day
7-9
embdiff_day

FIG.5

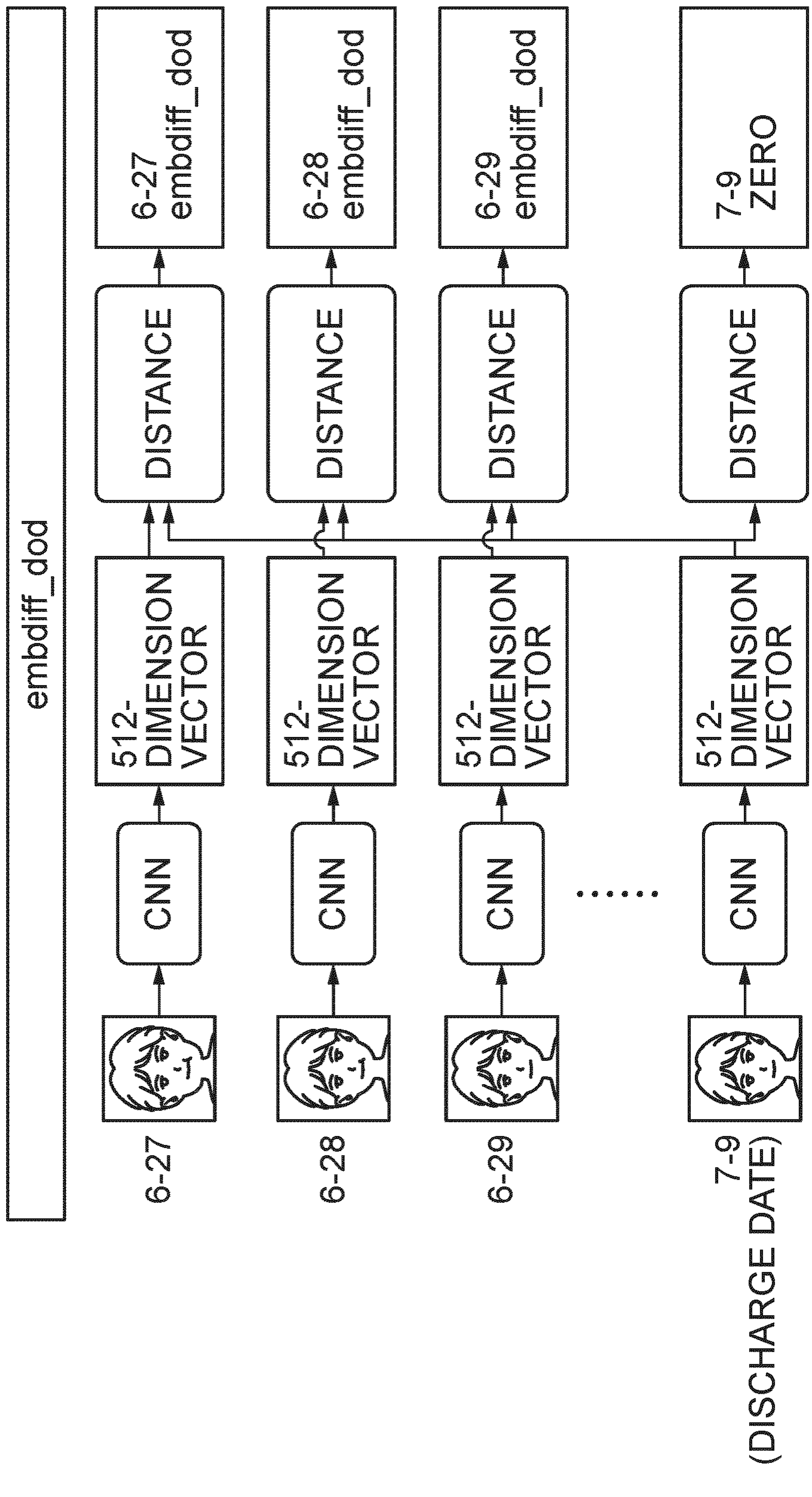
embdiff_dod
6-27
6-28
6-29
7-9
(DISCHARGE DATE)
CNN
512-
DIMENSION
VECTOR
DISTANCE
6-27
embdiff_dod
6-28
embdiff_dod
6-29
embdiff_dod
7-9
ZERO

FIG.6-1A

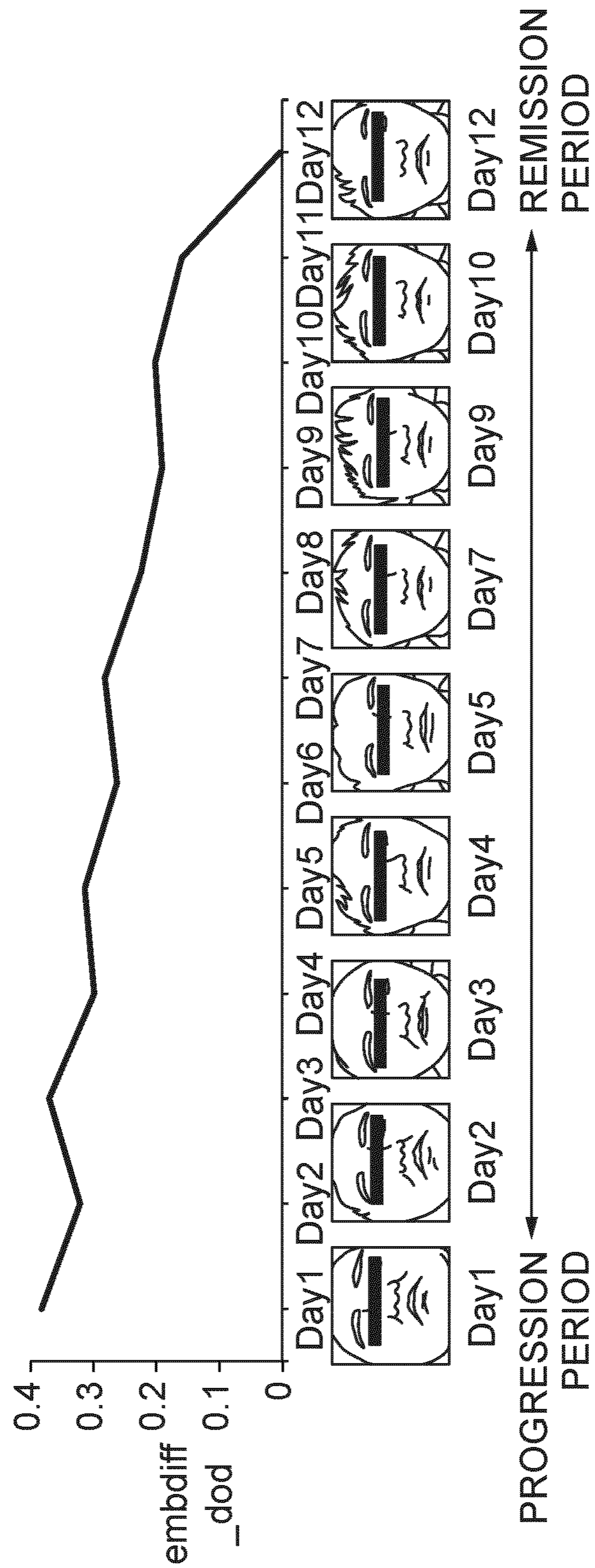
embdiff
_dod
0.4
0.3
0.2
0.1
0
Day1
Day2
Day3
Day4
Day5
Day6
Day7
Day8
Day9
Day10
Day11
Day12
Day1
Day2
Day3
Day4
Day5
Day7
Day9
Day10
Day12
PROGRESSION
PERIOD
REMISSION
PERIOD

FIG.6-1B

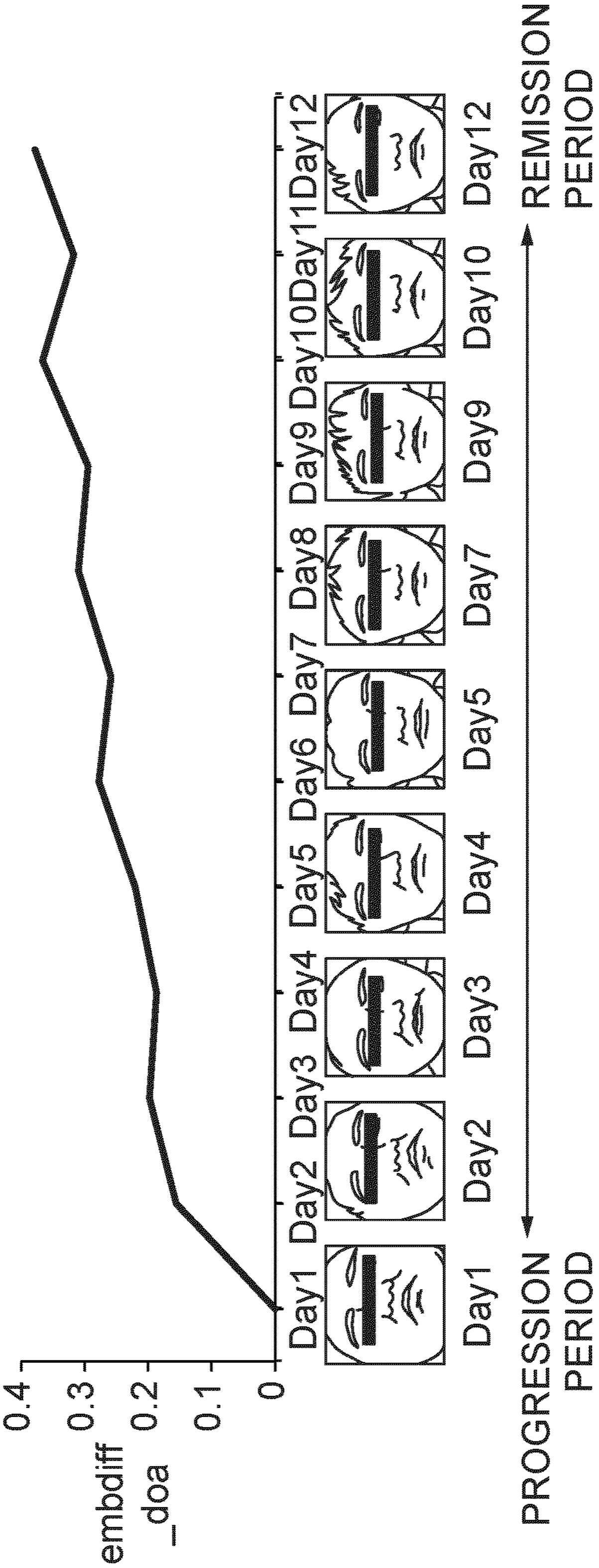
embdiff
_doa
0.4
0.3
0.2
0.1
0
Day1
Day2
Day3
Day4
Day5
Day6
Day7
Day8
Day9
Day10
Day11
Day12
Day1
Day2
Day3
Day4
Day5
Day7
Day9
Day10
Day12
PROGRESSION
PERIOD
REMISSION
PERIOD

FIG.6-2

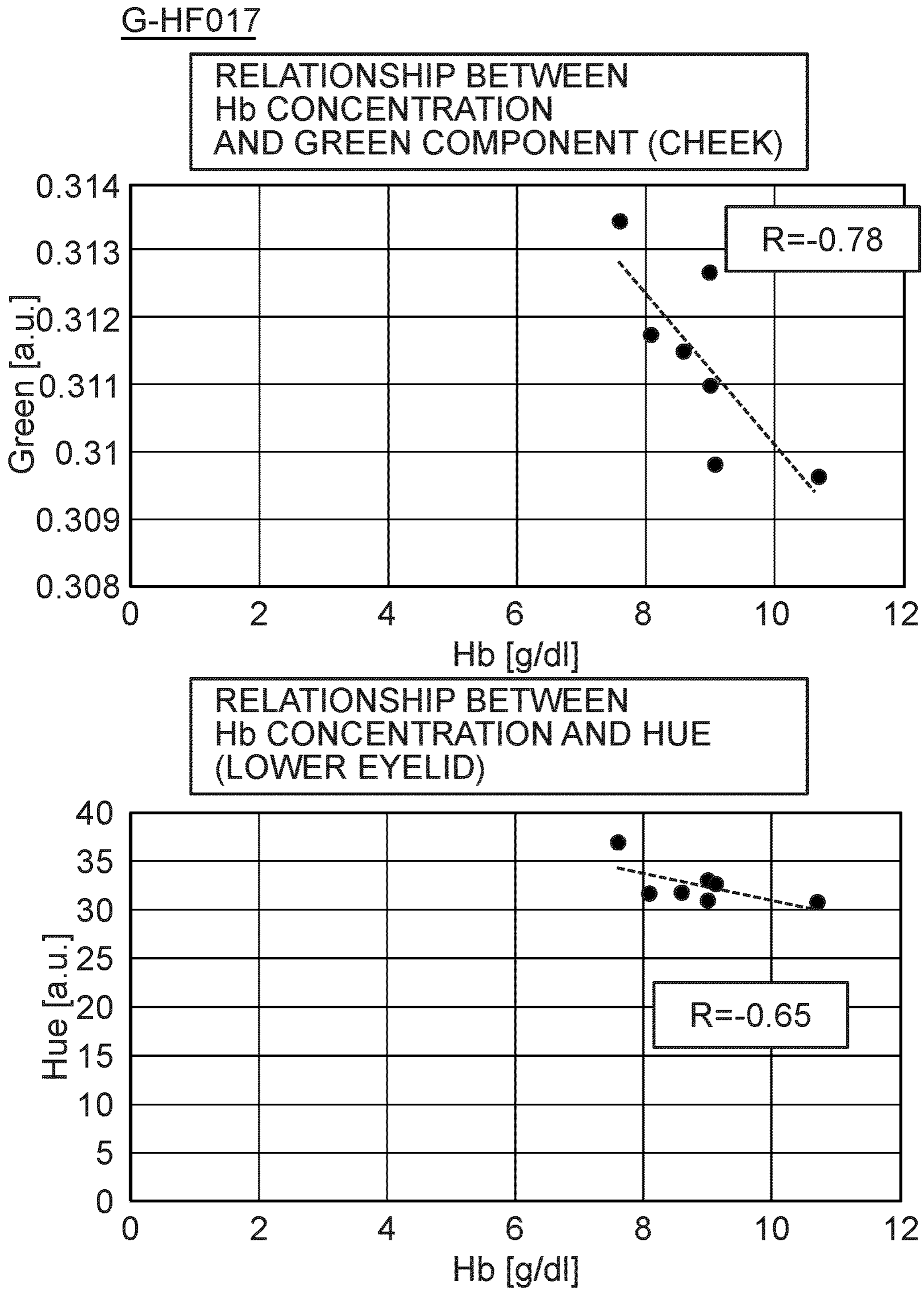
G-HF017
RELATIONSHIP BETWEEN Hb CONCENTRATION AND GREEN COMPONENT (CHEEK)
R=-0.78
Green [a.u.]
0.314
0.313
0.312
0.311
0.31
0.309
0.308
0
2
4
6
8
10
12
Hb [g/dl]
RELATIONSHIP BETWEEN Hb CONCENTRATION AND HUE (LOWER EYELID)
R=-0.65
Hue [a.u.]
40
35
30
25
20
15
10
5
0
Hb [g/dl]

FIG.6-3

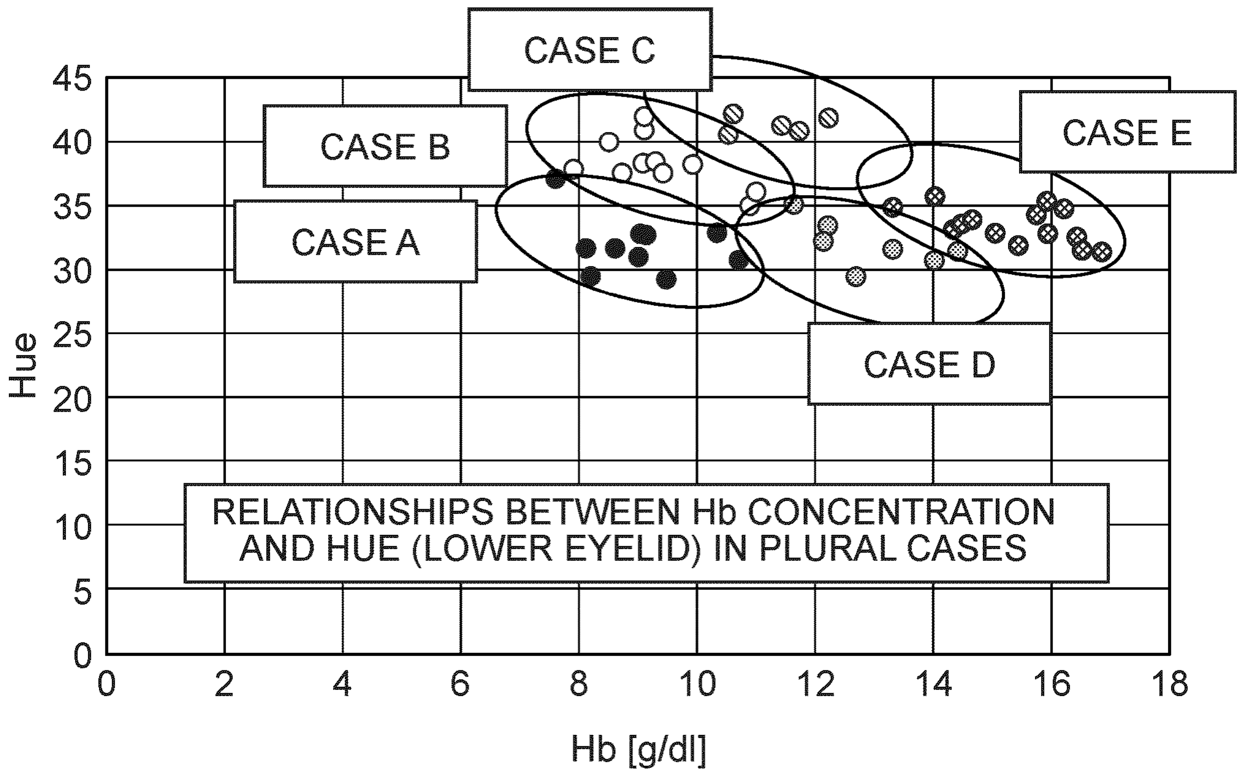
CASE C
CASE B
CASE A
CASE E
CASE D
45
40
35
30
25
20
15
10
5
0
Hue
0
2
4
6
8
10
12
14
16
18
Hb [g/dl]
RELATIONSHIPS BETWEEN Hb CONCENTRATION AND HUE (LOWER EYELID) IN PLURAL CASES

FIG.6-4

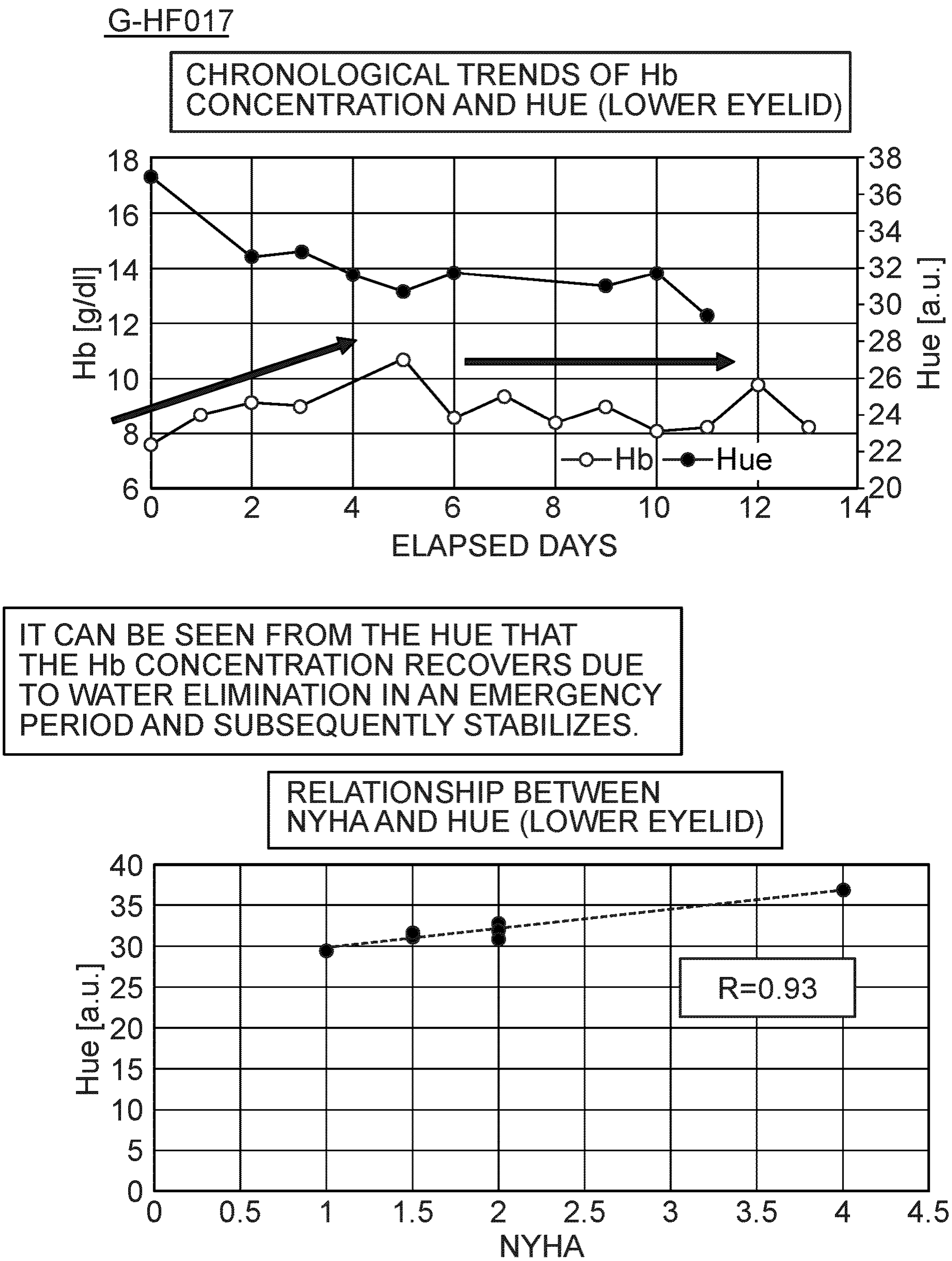
G-HF017
CHRONOLOGICAL TRENDS OF Hb CONCENTRATION AND HUE (LOWER EYELID)
Hb [g/dl]
Hue [a.u.]
ELAPSED DAYS
Hb
Hue
18
16
14
12
10
8
6
38
36
34
32
30
28
26
24
22
20
0
2
4
6
8
10
12
14
IT CAN BE SEEN FROM THE HUE THAT THE Hb CONCENTRATION RECOVERS DUE TO WATER ELIMINATION IN AN EMERGENCY PERIOD AND SUBSEQUENTLY STABILIZES.
RELATIONSHIP BETWEEN NYHA AND HUE (LOWER EYELID)
Hue [a.u.]
NYHA
R=0.93
40
35
30
25
20
15
10
5
0
0
0.5
1
1.5
2
2.5
3
3.5
4
4.5

FIG.7

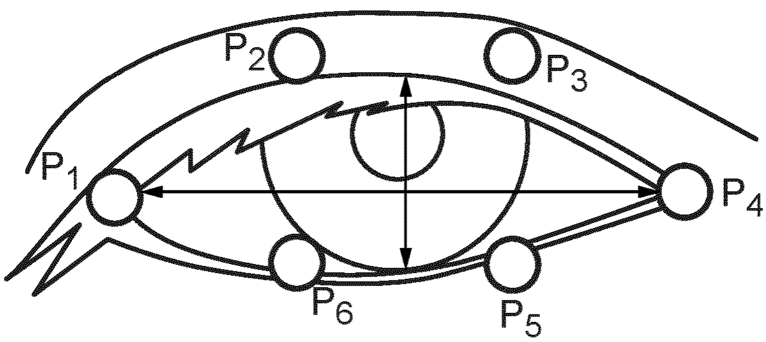

FIG.8

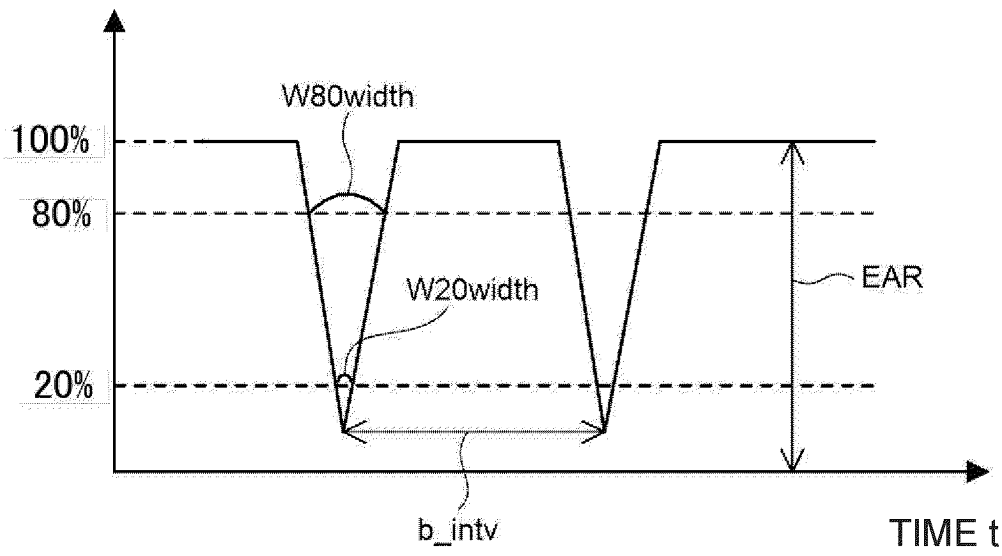

FIG.9

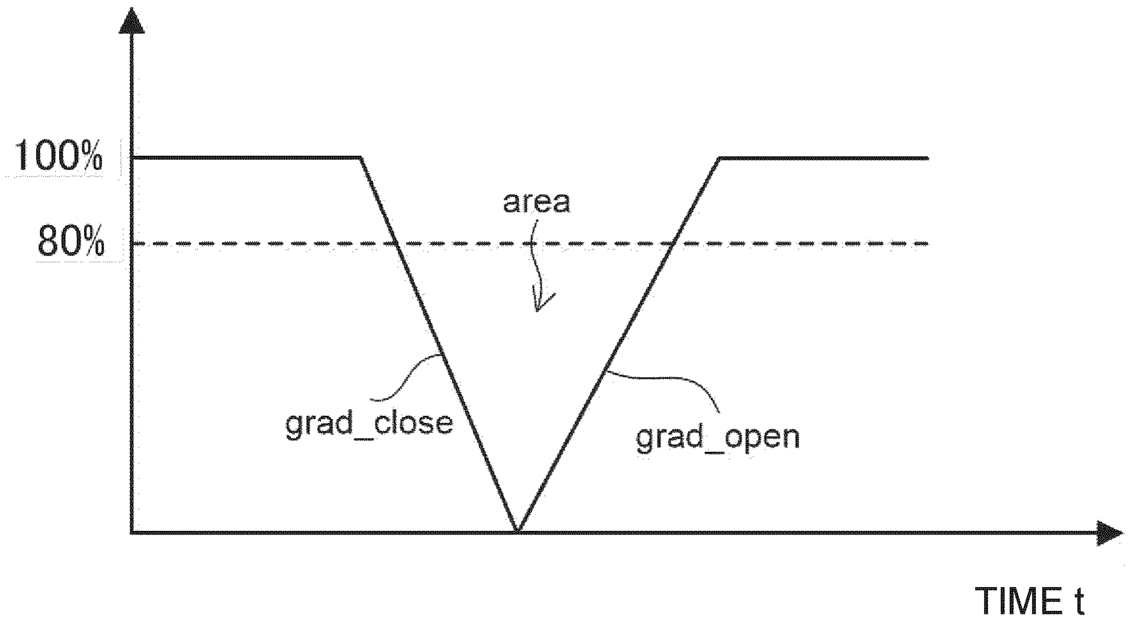

FIG.10

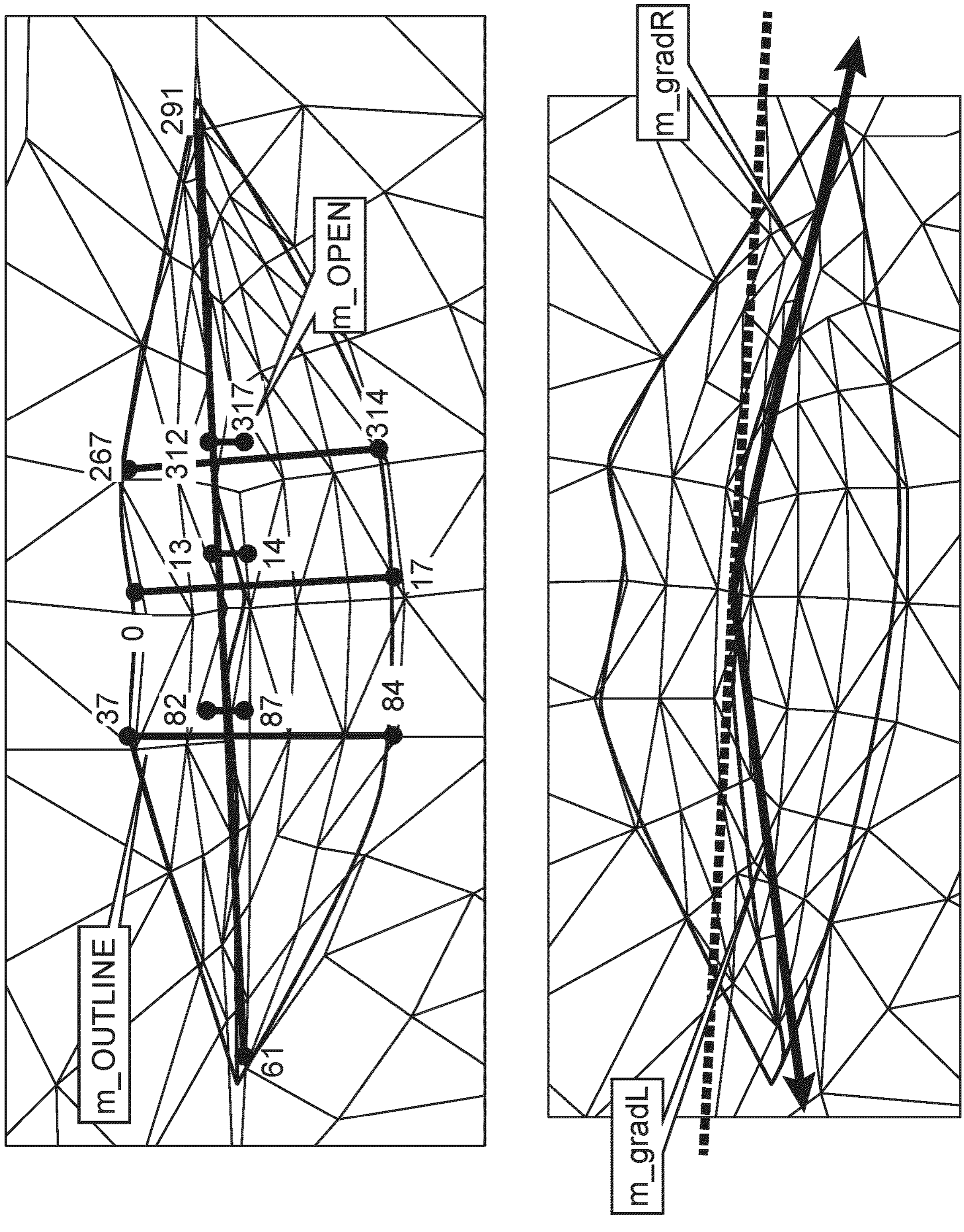
291
m_OPEN
267
312
317
314
13
14
17
0
37
82
87
84
m_OUTLINE
61
m_gradR
m_gradL

FIG.11-1

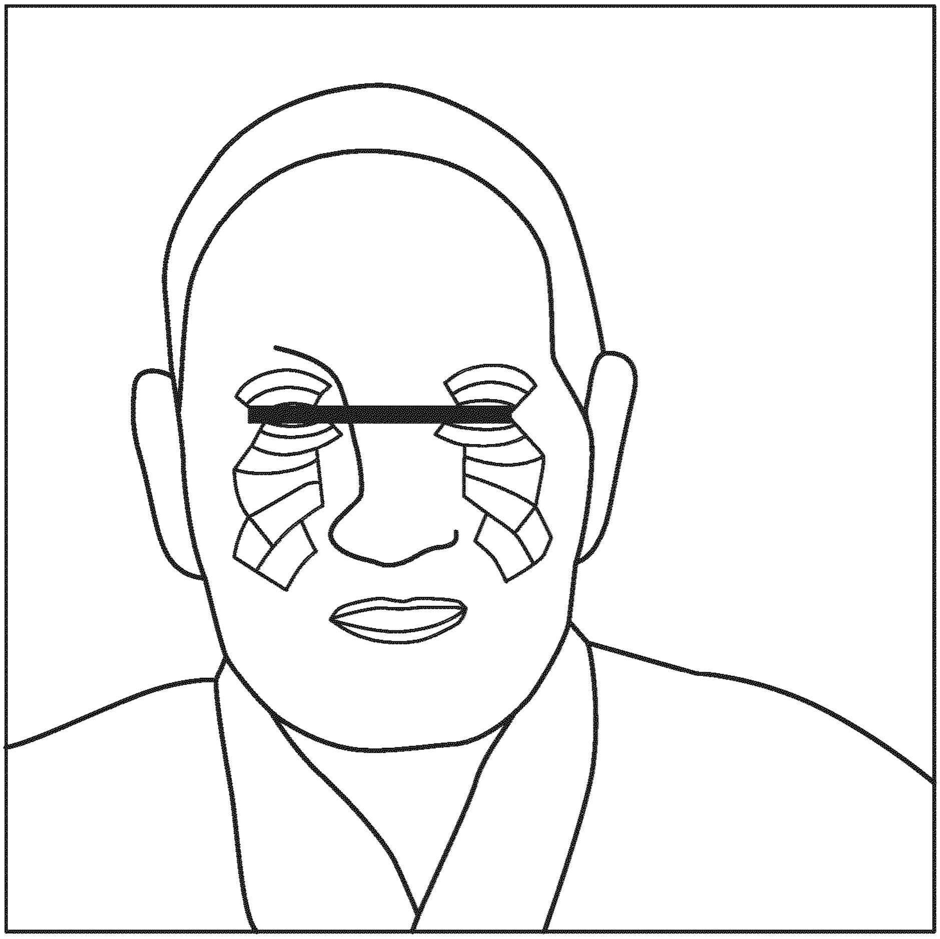

FIG.11-2

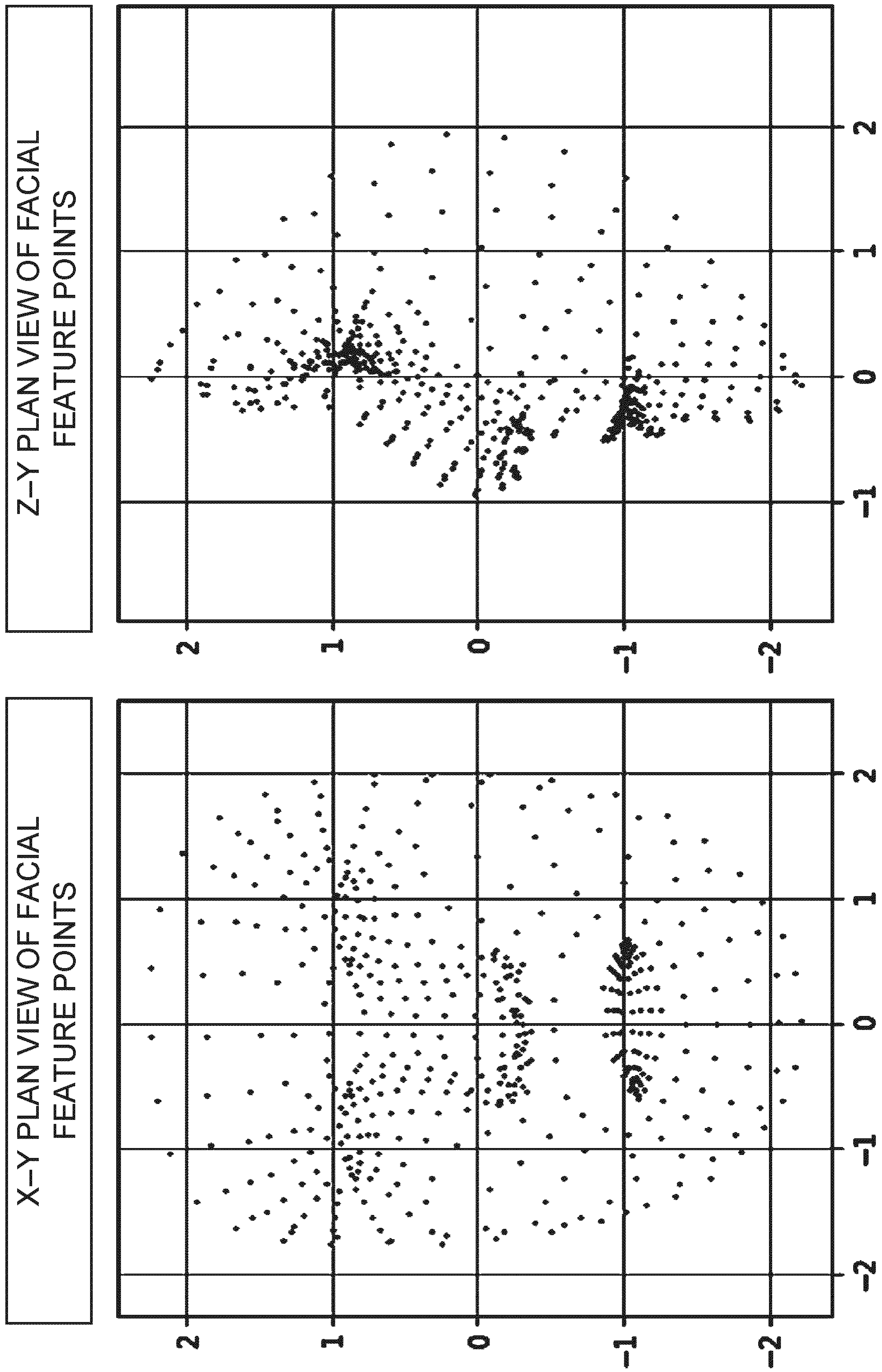
X–Y PLAN VIEW OF FACIAL FEATURE POINTS
Z–Y PLAN VIEW OF FACIAL FEATURE POINTS

FIG.12

| Feature parameters | More specific feature parameter | Labelr | Object information | Data | Area of interest | Related symptoms and features |
|---|---|---|---|---|---|---|
| Parameters relating to shape of the face of the patient | Parameter expressing area of the face | Area_all, Area_brow | 2-dimensional shape | RGB still image or video | Whole face | Edema |
| | Parameter expressing horizontal direction width of the face | — | 2-dimensional shape | RGB still image or video | Whole face, cheek or jaw | Edema |
| | Parameter expressing vertical direction | — | 2-dimensional shape | RGB still image or video | Whole face | Edema and expression |
| | Parameter expressing a face vector | — | 2-dimensional shape | RGB still image or video | Whole face | Edema and expression |
| | Feature parameter expressing distance between a face vector obtained on the estimation date and a face vector obtained on a previous date | embdiff_day | 2-dimensional shape | RGB still image or video | Whole face | Edema and expression |
| | Feature parameter expressing distance between a face vector obtained on the estimation date and a face vector in a period of remission | embdiff_dod | 2-dimensional shape | RGB still image or video | Whole face | Edema and expression |
| | Feature parameter expressing distance between a face vector obtained on the estimation date and a face vector in a period of progression | embdiff_doa | 2-dimensional shape | RGB still image or video | Whole face | Edema and expression |
| Parameters relating to complexion of the patient | Parameter expressing a hue of the face | — | Color | RGB still image or video | Whole face | Peripheral circulation and degree of anemia |
| | Parameter expressing greenness of the face | Green | Color | RGB still image or video | Cheek, between eyebrows or nose | Peripheral circulation and degree of anemia |

FIG.13

| Feature parameters | More specific feature parameter | Labelr | Object information | Data | Area of interest | Related symptoms and features |
|---|---|---|---|---|---|---|
| Parameters relating to eyes of the patient | Parameter expressing aspect ratio of an eye of the patient | EAR | 2-dimensional shape | RGB still image or video | Eye | Edema, weariness and expression |
| | Parameter expressing difference between the aspect ratios EAR of the left and right eyes of the patient | diffLR | 2-dimensional shape | RGB still image or video | Eyes | Edema, weariness and expression |
| | Parameter expressing duration an eye is closed in a single blink | width, W80width, W20width | 2-dimensional shape | Video | Eyes | Edema and weariness |
| | Parameter expressing proportion of a unit time that an eye is closed | fcnt, W80fcnt, W20fcnt | 2-dimensional shape | Video | Eye | Edema and weariness |
| | Parameter expressing interval between blinks | b_intv, b_intv_avg | 2-dimensional shape | Video | Eye | Edema and weariness |
| | Parameter expressing number of blinks per unit time | Close | 2-dimensional shape | Video | Eye | Edema and weariness |
| | Parameter expressing speed when an eye closes | grad_close, grad_close_avg | 2-dimensional shape | Video | Eye | Edema and weariness |
| | Parameter expressing speed when an eye opens | grad_open, grad_open_avg | 2-dimensional shape | Video | Eye | Edema and weariness |
| | Parameter expressing length of time an eye is closed | area | 2-dimensional shape | Video | Eye | Edema and weariness |
| | Parameter expressing average speed during opening and closing of an eye | grad_avg | 2-dimensional shape | Video | Eye | Edema and weariness |
| | Parameter expressing vertical direction inclination of the face | Pitch | 2-dimensional shape | RGB still image or video | Eyes | Edema and weariness |
| Parameters relating to the mouth of the patient | Parameter expressing aspect ratio of the mouth of the patient | m_OUTLINE | 2-dimensional shape | RGB still image or video | Mouth | Edema, gritted teeth and expression |
| | Parameter expressing opening state of the mouth of the patient | m_OPEN | 2-dimensional shape | RGB still image or video | Mouth | Edema, gritted teeth and expression |
| | Parameter expressing the left corner of the mouth | m_gradL | 2-dimensional shape | RGB still image or video | Mouth | Edema, gritted teeth and expression |
| | Parameter expressing the right corner of the mouth | m_gradR | 2-dimensional shape | RGB still image or video | Mouth | Edema, gritted teeth and expression |
| Parameter relating to water content of the face | Parameter relating to water content of the face | Adiff | Light of specific near-infrared wavelengths | NIR still image | Upper and lower eyelids and cheeks | Edema |
| Parameter relating to three-dimensional shape of the face | Parameter relating to three-dimensional shape of the face | — | 3-dimensional shape | Depth map | Whole face, eyelids and cheeks | Edema |

FIG.14

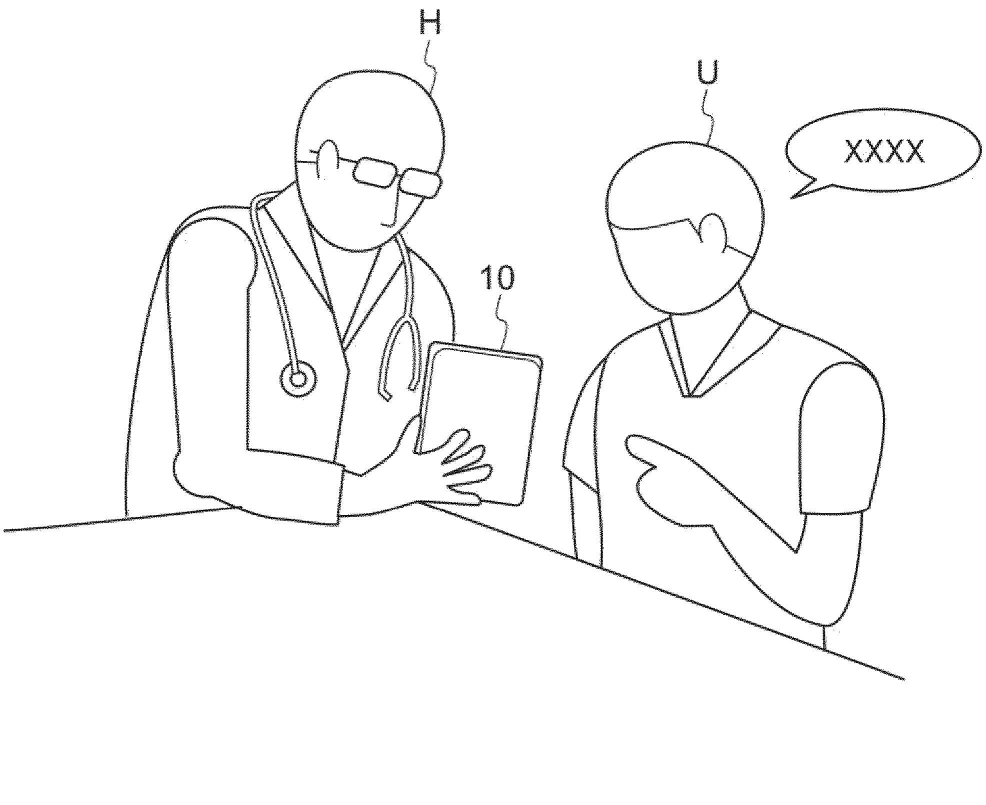

FIG.15

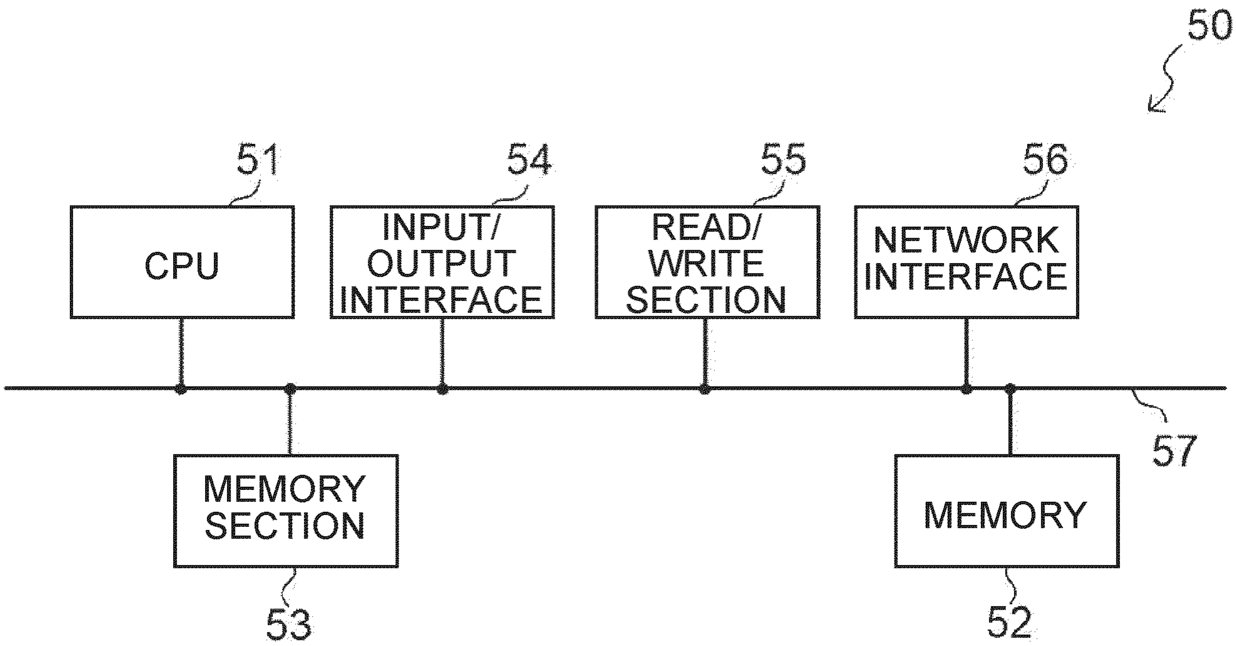
50
51
CPU
54
INPUT/
OUTPUT
INTERFACE
55
READ/
WRITE
SECTION
56
NETWORK
INTERFACE
57
MEMORY
SECTION
53
MEMORY
52

FIG.16

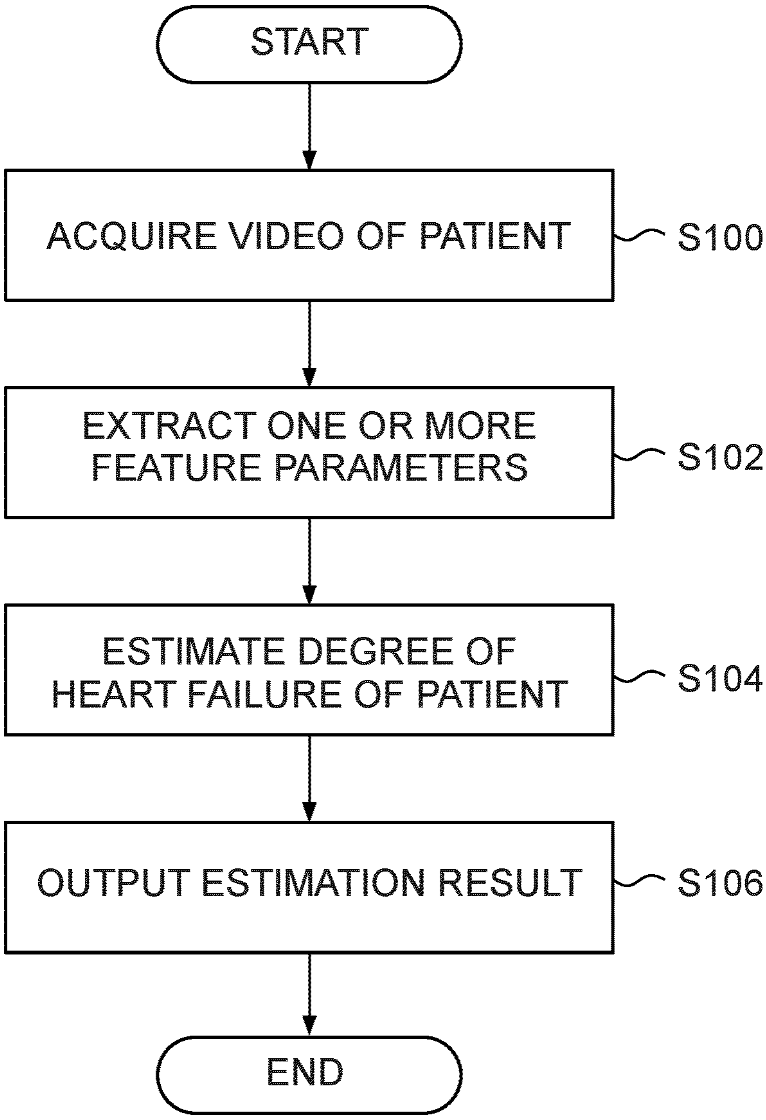
START
ACQUIRE VIDEO OF PATIENT
S100
EXTRACT ONE OR MORE FEATURE PARAMETERS
S102
ESTIMATE DEGREE OF HEART FAILURE OF PATIENT
S104
OUTPUT ESTIMATION RESULT
S106
END

FIG.17

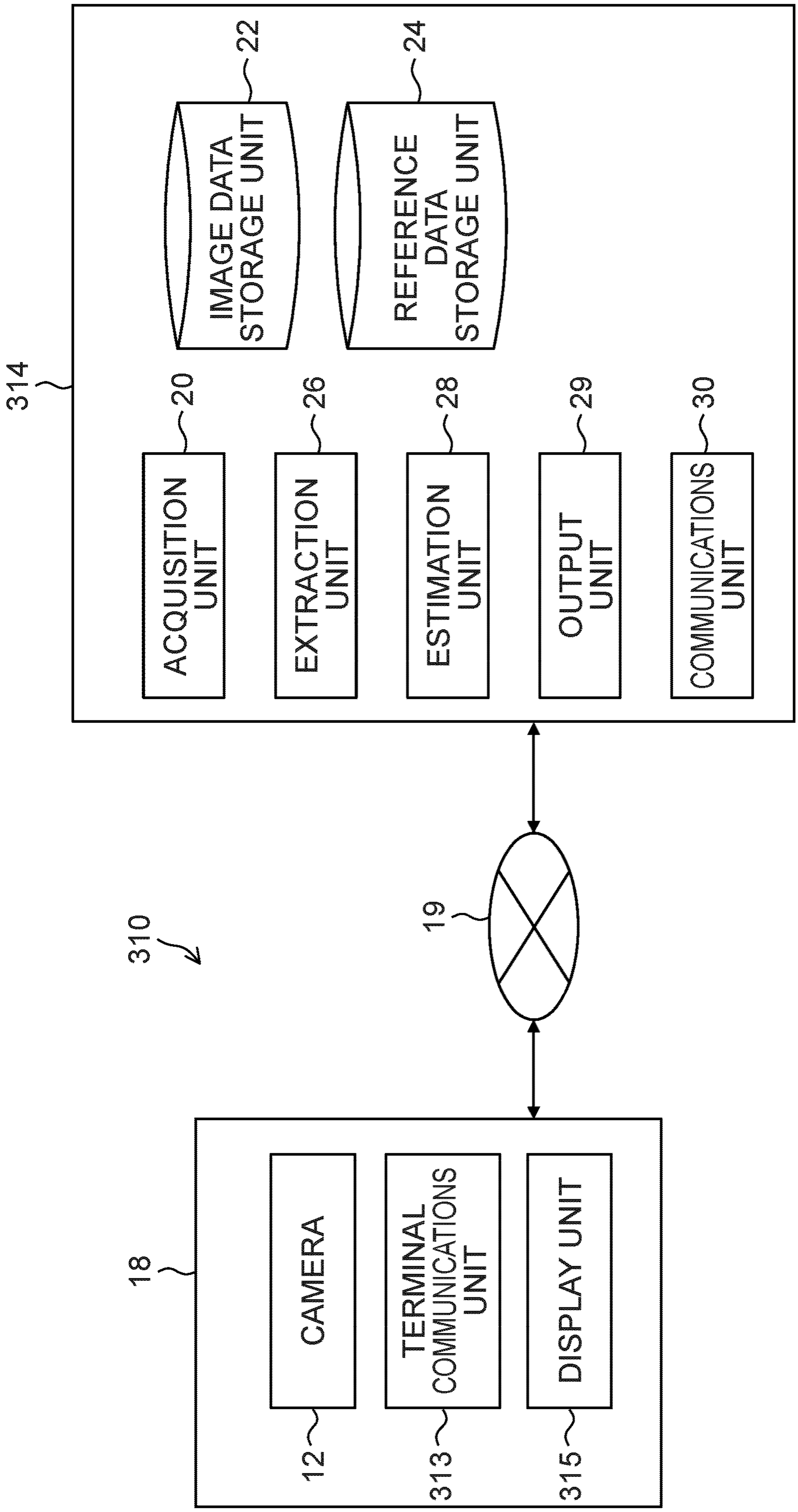
310
314
ACQUISITION UNIT
20
EXTRACTION UNIT
26
ESTIMATION UNIT
28
OUTPUT UNIT
29
COMMUNICATIONS UNIT
30
IMAGE DATA STORAGE UNIT
22
REFERENCE DATA STORAGE UNIT
24
19
18
CAMERA
12
TERMINAL COMMUNICATIONS UNIT
313
DISPLAY UNIT
315

FIG.18

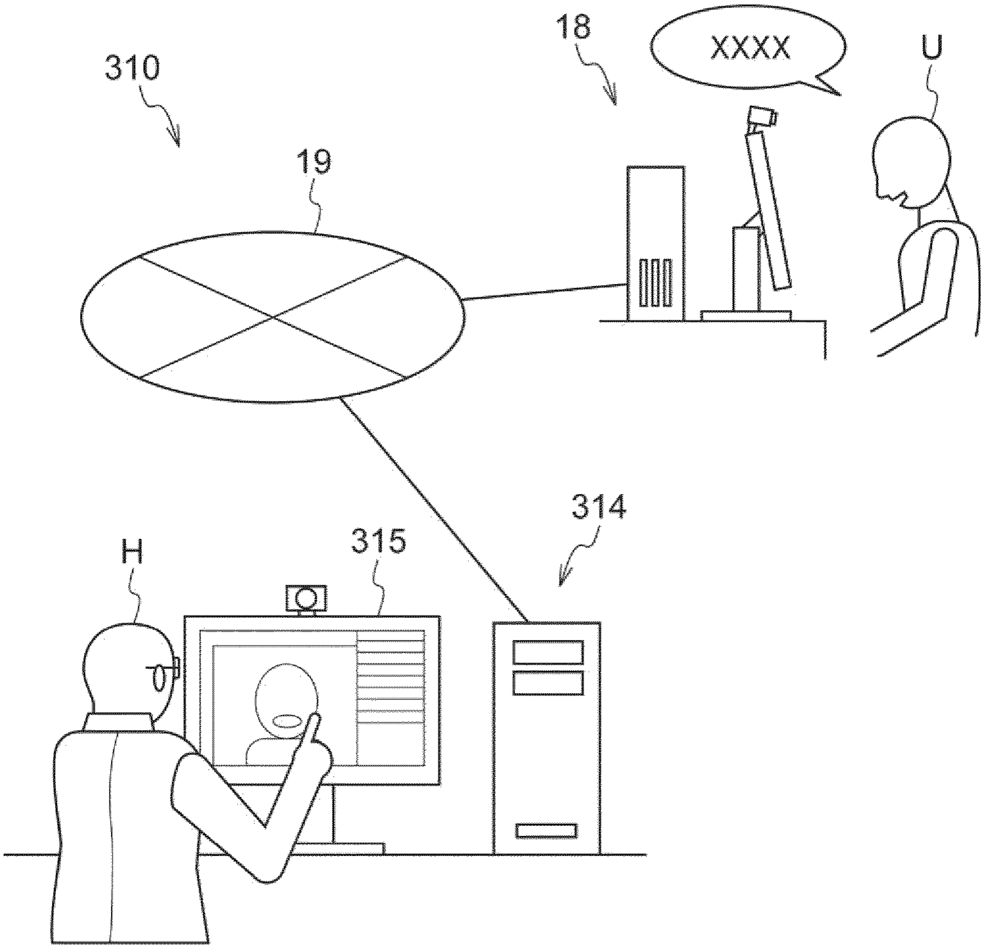

FIG.19

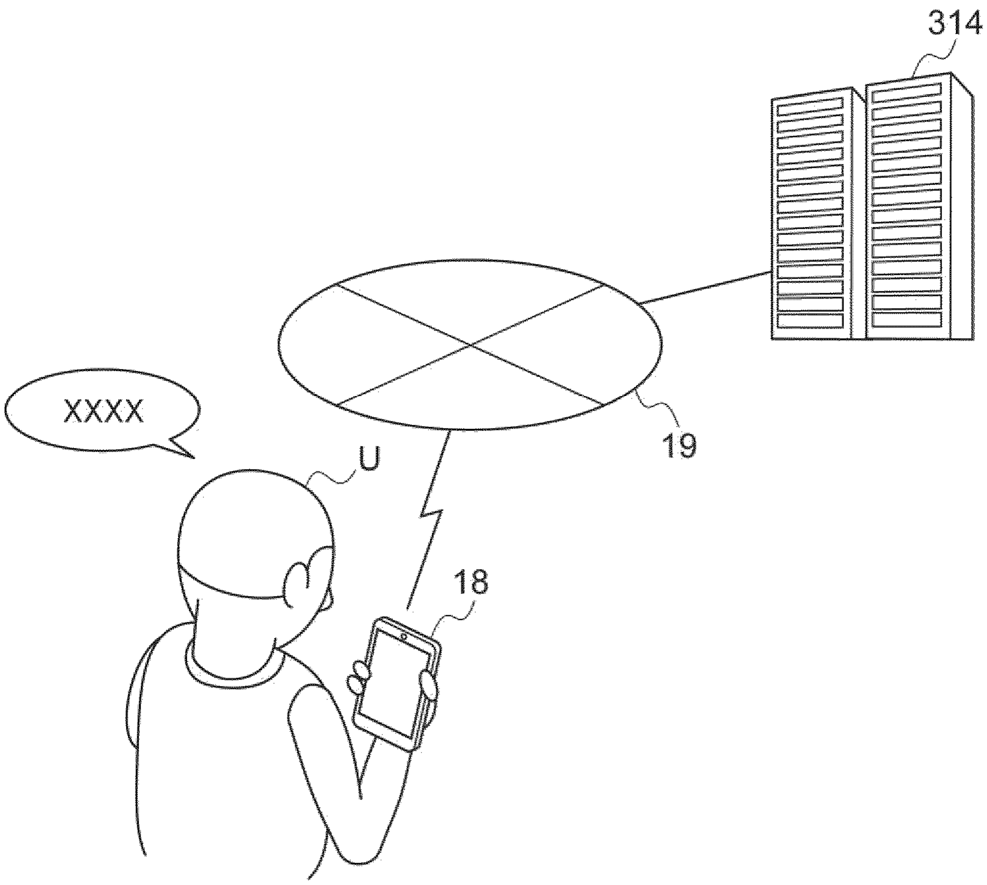

FIG.20

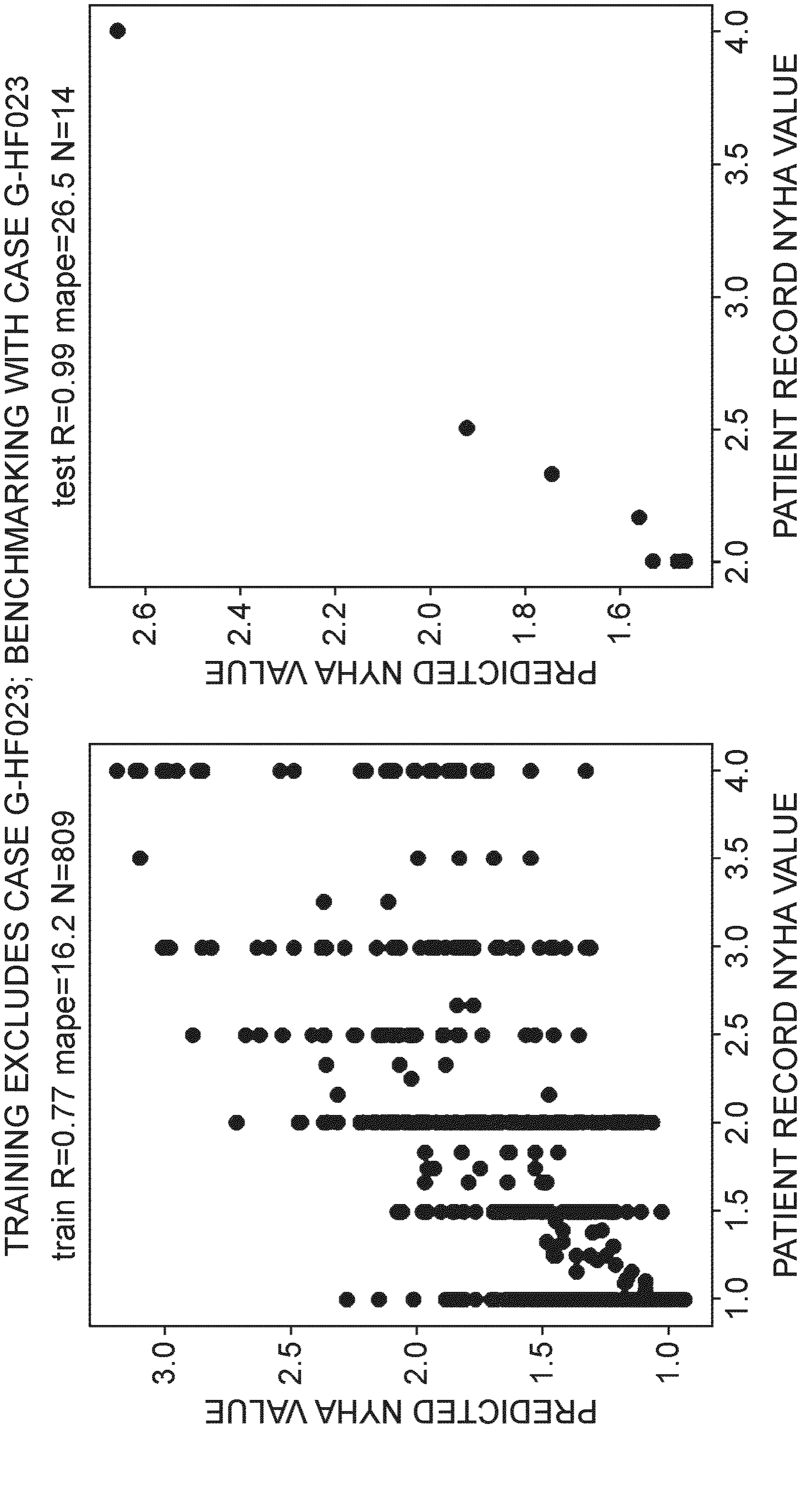
TRAINING EXCLUDES CASE G-HF023; BENCHMARKING WITH CASE G-HF023
train R=0.77 mape=16.2 N=809
test R=0.99 mape=26.5 N=14
PREDICTED NYHA VALUE
PATIENT RECORD NYHA VALUE
3.0
2.5
2.0
1.5
1.0
4.0
3.5
2.6
2.4
2.2
1.8
1.6

FIG.21

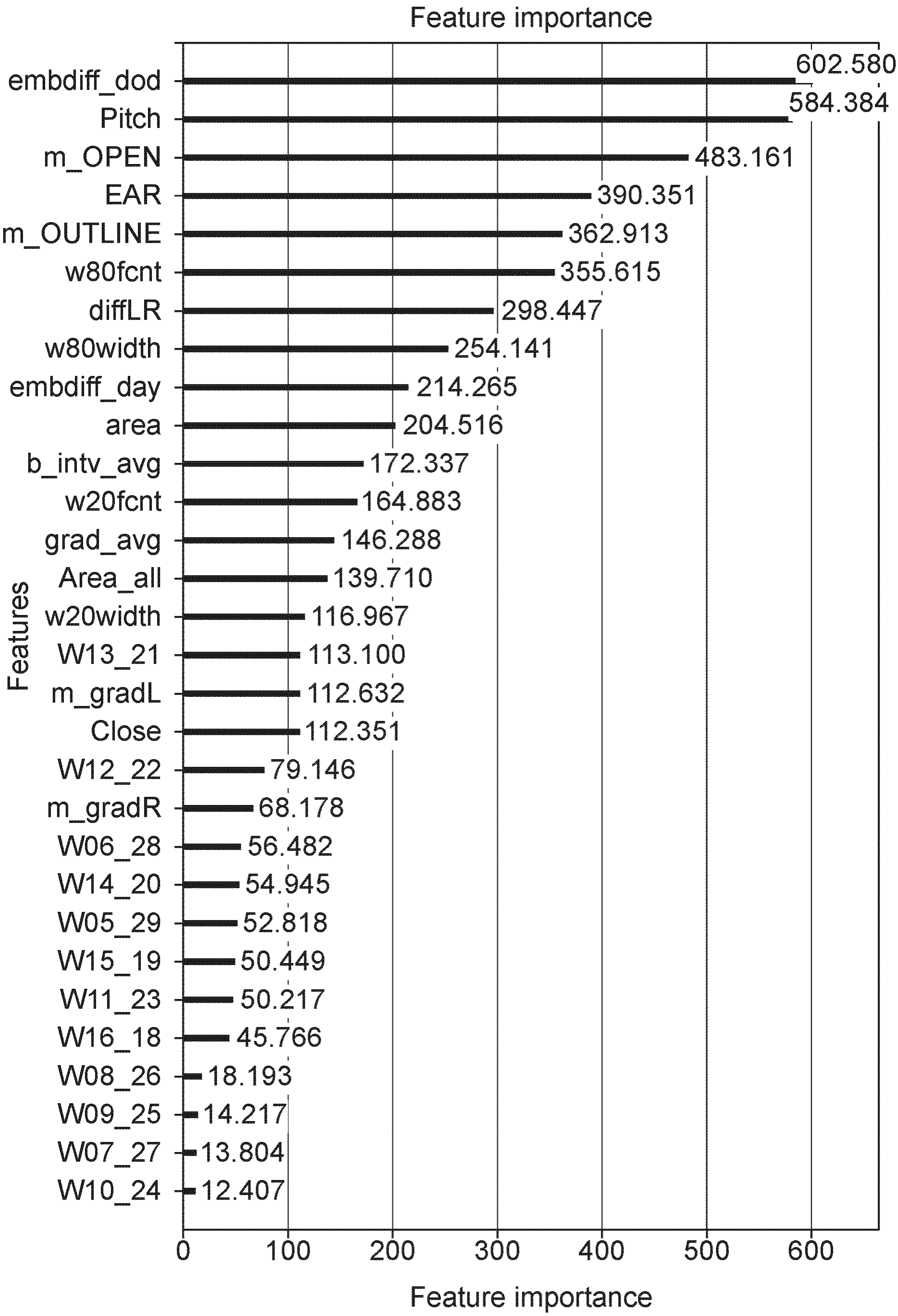
Feature importance
embdiff_dod 602.580
Pitch 584.384
m_OPEN 483.161
EAR 390.351
m_OUTLINE 362.913
w80fcnt 355.615
diffLR 298.447
w80width 254.141
embdiff_day 214.265
area 204.516
b_intv_avg 172.337
w20fcnt 164.883
grad_avg 146.288
Area_all 139.710
w20width 116.967
W13_21 113.100
m_gradL 112.632
Close 112.351
W12_22 79.146
m_gradR 68.178
W06_28 56.482
W14_20 54.945
W05_29 52.818
W15_19 50.449
W11_23 50.217
W16_18 45.766
W08_26 18.193
W09_25 14.217
W07_27 13.804
W10_24 12.407
Features
0
100
200
300
400
500
600
Feature importance

# FIG.22-1

NYHA PREDICTION EXPRESSION = -10.89 + 1.29*embdiff_dod + 2.07*eye W80fcnt + 0.16*Pitch - 0.15+Area_all
(PREDICTION EXPRESSION CREATED FROM DATA OBTAINED FROM CASES G-HF002 TO G-HF038.
THE GRAPHS BELOW SHOW NYHA PREDICTION RESULTS OF THE INDICATORS.)

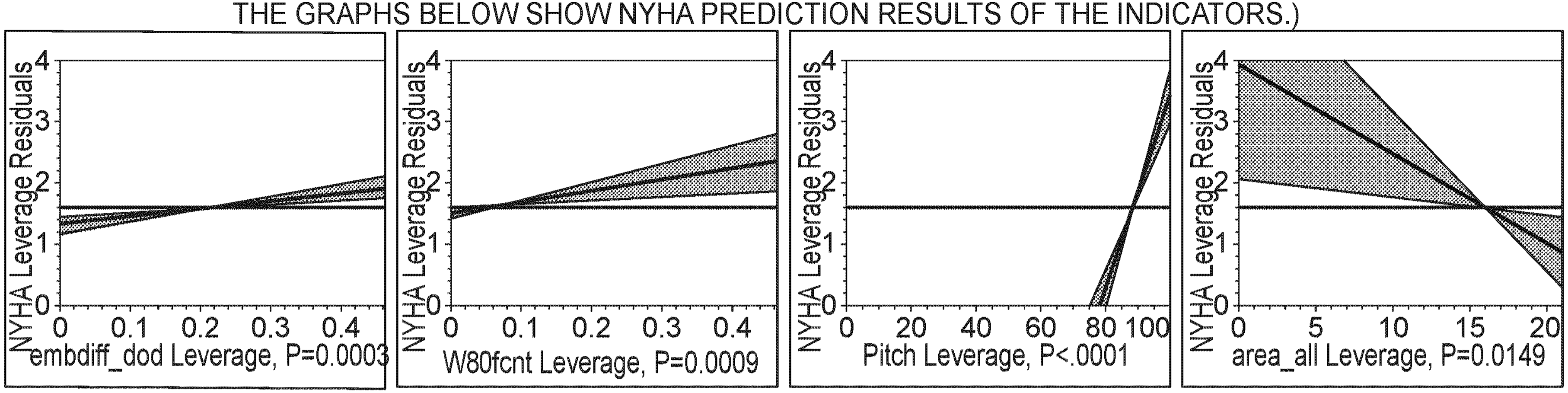


embdiff_dod W80fcnt(EYE) Pitch Area_all

CORRELATION BETWEEN ACTUAL NYHA VALUES (X AXIS) AND PREDICTED NYHA VALUES (Y AXIS)

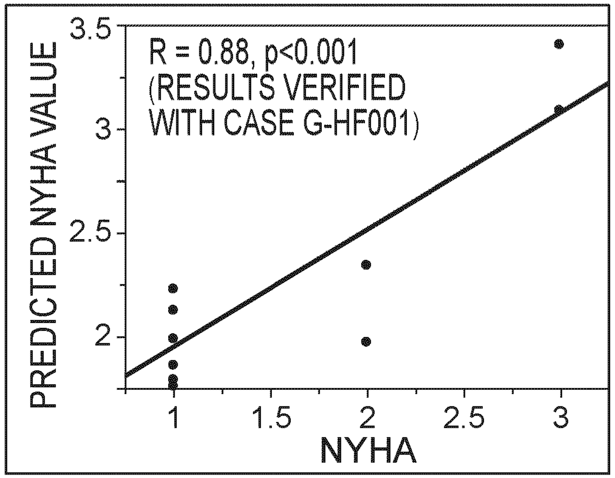


PREDICTION EXPRESSION FOR ESTIMATING NYHA CREATED USING DATA OBTAINED FROM CASES G-HF002 TO G-HF038 (UPPER GRAPHS).
THE RESULTS ARE VERIFIED WITH CASE G-HF001, CONFIRMING A STRONG CORRELATION (CORRELATION COEFFICIENT 0.88) BETWEEN NYHA ACTUAL VALUES AND PREDICTED VALUES (LOWER GRAPH).

FIG.22-2

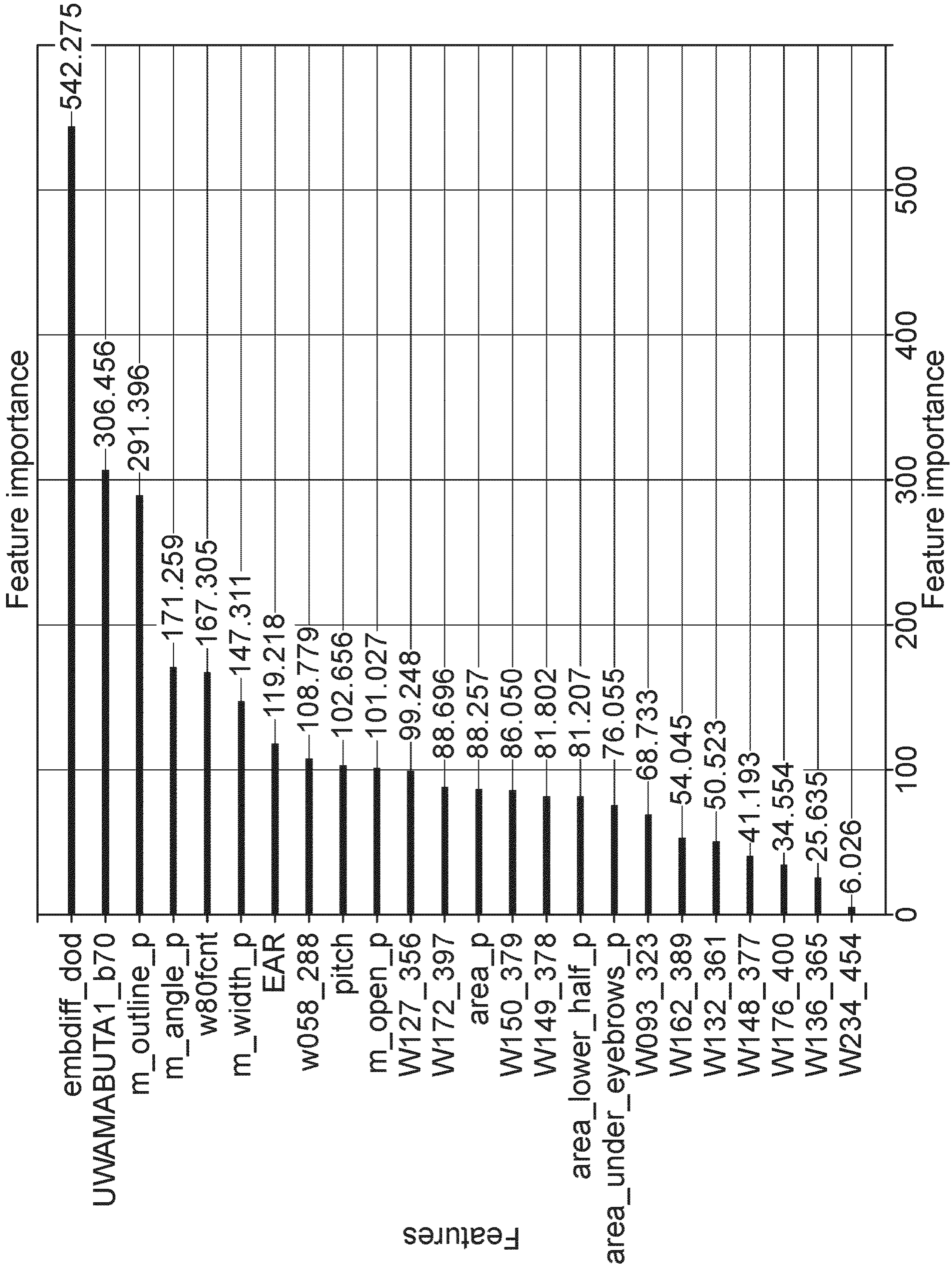
Feature importance
Features
embdiff_dod 542.275
UWAMABUTA1_b70 306.456
m_outline_p 291.396
m_angle_p 171.259
w80fcnt 167.305
m_width_p 147.311
EAR 119.218
w058_288 108.779
pitch 102.656
m_open_p 101.027
W127_356 99.248
W172_397 88.696
area_p 88.257
W150_379 86.050
W149_378 81.802
area_lower_half_p 81.207
area_under_eyebrows_p 76.055
W093_323 68.733
W162_389 54.045
W132_361 50.523
W148_377 41.193
W176_400 34.554
W136_365 25.635
W234_454 6.026
0
100
200
300
400
500
Feature importance

FIG.22-3

TRAINING EXCLUDES CASE G-HF017; BENCHMARKING WITH CASE G-HF017

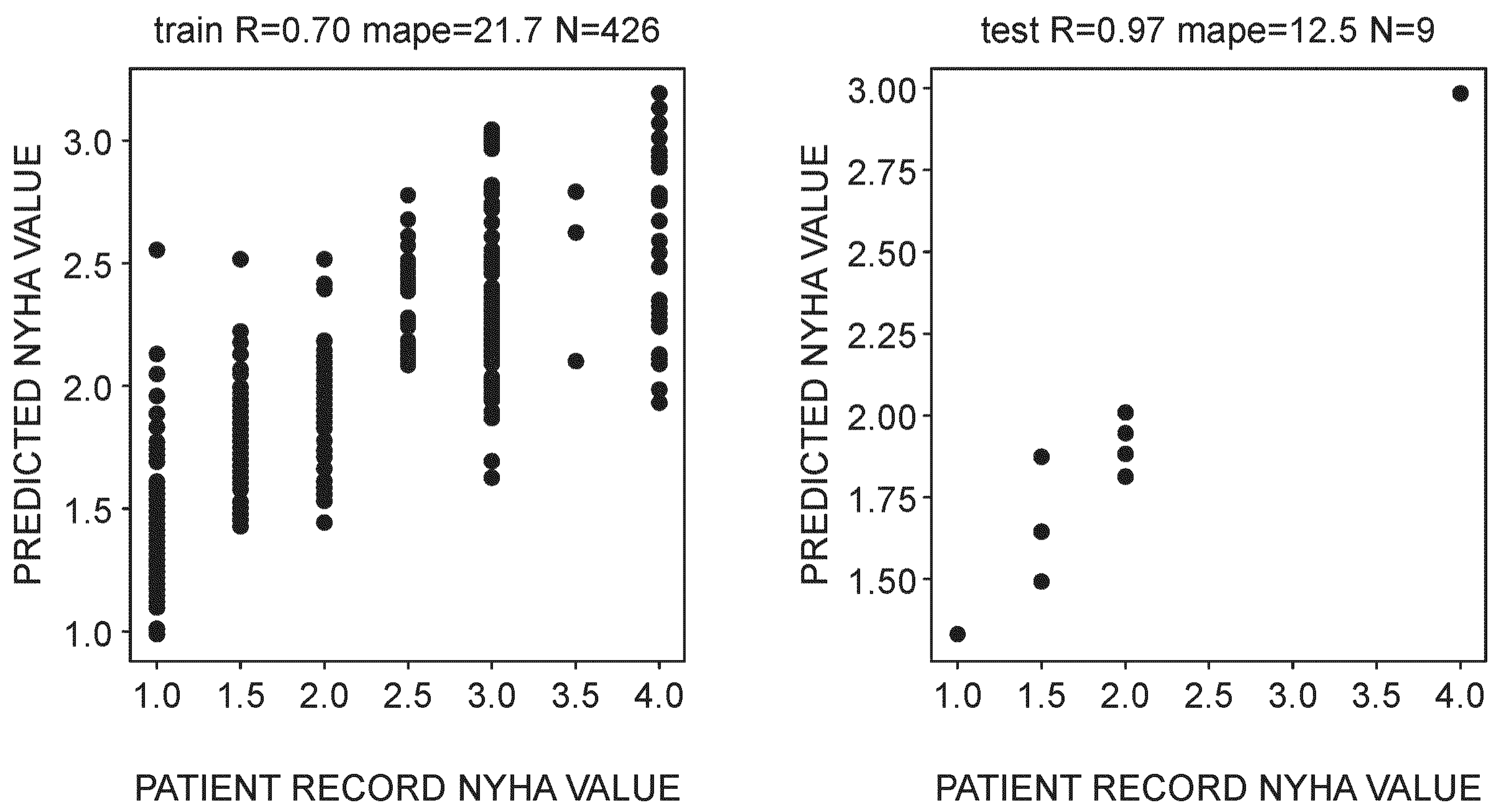

FIG.22-4

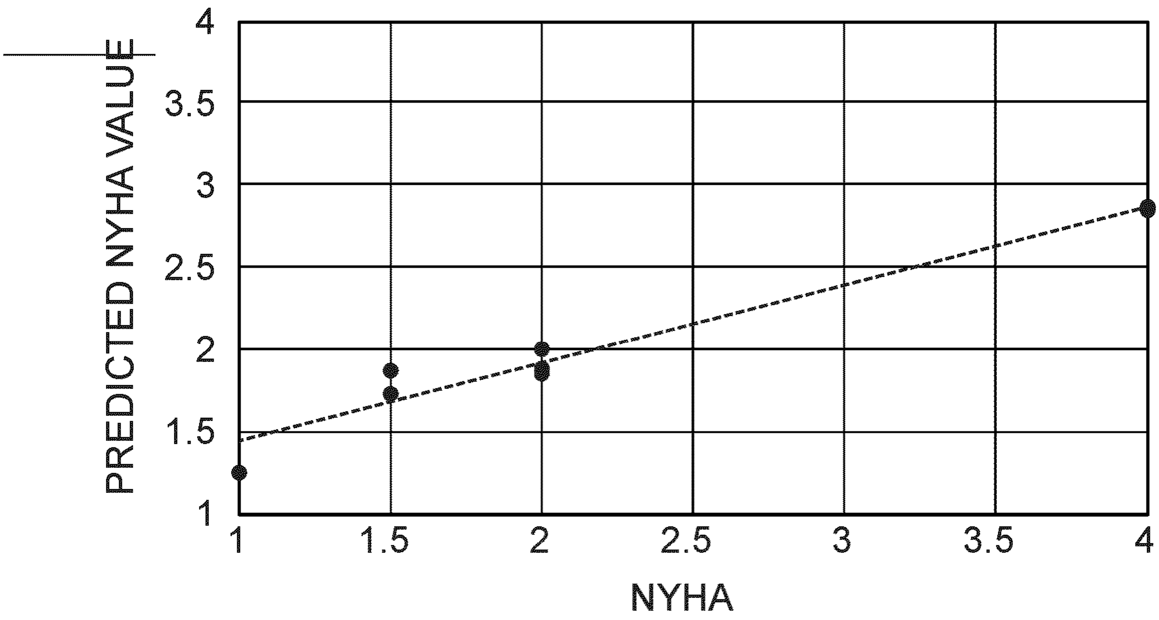

FIG.23

| Indicator | Subjective values | | | | Area_all Threshold | | embdiff_dod Threshold | | EAR | Adiff relation (body weight) | Adiff relation (BNP) | Detection confidence |
|---|---|---|---|---|---|---|---|---|---|---|---|---|
| Case | Face outline | Eyes | Mouth | Logical OR | Change | 0.06 | embdiff_dod | 0.29 | 1 for increasing | r ≥ 0.6 | r ≥ 0.6 | Total of indicators |
| G-HF001 | 1 | 1 | 1 | 1 | 0.16 | 1 | 0.4 | 1 | 1 | | | 3 |
| G-HF002 | 0 | 0 | 0 | 0 | 0.13 | 1 | 0.33 | 1 | 1 | | | 3 |
| G-HF003 | 0 | 0 | 1 | 1 | 0.08 | 1 | 0.3 | 1 | 1 | | | 3 |
| G-HF004 | 0 | 0 | 0 | 0 | 0.05 | 0 | 0.39 | 1 | 1 | | | 2 |
| G-HF005 | 0 | 0 | 1 | 1 | 0.02 | 0 | 0.26 | 0 | 1 | | | 1 |
| G-HF006 | 0 | 0 | 0 | 0 | 0.04 | 0 | 0.19 | 0 | 0 | | | 0 |
| G-HF007 | 1 | 0 | 0 | 1 | 0.06 | 1 | 0.22 | 0 | 1 | | | 2 |
| G-HF008 | 0 | 0 | 1 | 1 | 0.05 | 0 | 0.18 | 0 | 0 | | | 0 |
| G-HF009 | 1 | 1 | 1 | 1 | 0.09 | 1 | 0.3 | 1 | 1 | | | 3 |
| G-HF010 | 0 | 0 | 0 | 0 | 0.06 | 1 | 0.26 | 0 | 0 | | | 1 |
| G-HF011 | 0 | 0 | 0 | 0 | 0.09 | 1 | 0.33 | 1 | 0 | | | 2 |
| G-HF012 | 1 | 1 | 0 | 1 | 0.06 | 1 | 0.39 | 1 | 1 | 0 | 0 | 3 |
| G-HF013 | 1 | 1 | 0 | 1 | 0.04 | 0 | 0.43 | 1 | 1 | 1 | 1 | 4 |
| G-HF014 | 0 | 0 | 0 | 0 | 0.03 | 0 | 0.19 | 0 | 0 | 1 | 1 | 2 |
| G-HF015 | 0 | 0 | 1 | 1 | 0.05 | 0 | 0.38 | 1 | 0 | 1 | 1 | 3 |
| G-HF016 | 0 | 1 | 0 | 1 | 0.07 | 1 | 0.24 | 0 | 1 | 0 | 1 | 3 |
| G-HF017 | 1 | 1 | 1 | 1 | 0.11 | 1 | 0.41 | 1 | 1 | 1 | 1 | 5 |
| G-HF018 | 0 | 1 | 0 | 1 | 0.04 | 0 | 0.23 | 0 | 0 | 1 | 1 | 2 |
| G-HF019 | 0 | 0 | 0 | 0 | 0.03 | 0 | 0.16 | 0 | 1 | 1 | 0 | 2 |
| G-HF020 | 0 | 0 | 0 | 0 | 0.05 | 0 | 0.27 | 0 | 0 | 1 | 1 | 2 |
| G-HF021 | 0 | 1 | 0 | 1 | 0.11 | 1 | 0.29 | 1 | 1 | 1 | 1 | 5 |
| G-HF022 | 0 | 0 | 0 | 0 | 0.02 | 0 | 0.26 | 0 | 0 | 1 | 1 | 2 |
| G-HF023 | 0 | 1 | 0 | 1 | 0.04 | 0 | 0.44 | 1 | 1 | | | 2 |
| G-HF024 | 0 | 0 | 0 | 0 | 0.01 | 0 | 0.29 | 1 | 1 | 1 | 1 | 4 |
| G-HF025 | 0 | 0 | 0 | 0 | 0.07 | 1 | 0.23 | 0 | 0 | 1 | 1 | 3 |
| G-HF026 | 0 | 0 | 0 | 0 | 0.04 | 0 | 0.27 | 0 | 0 | 0 | 0 | 0 |
| G-HF027 | 0 | 1 | 1 | 1 | 0.09 | 1 | 0.22 | 0 | 1 | 1 | 0 | 3 |
| G-HF028 | 1 | 1 | 1 | 1 | 0.06 | 1 | 0.4 | 1 | 0 | 1 | 1 | 4 |
| G-HF029 | 1 | 1 | 1 | 1 | 0.08 | 1 | 0.48 | 1 | 1 | 0 | 0 | 3 |
| G-HF030 | 1 | 1 | 1 | 1 | 0.06 | 1 | 0.25 | 0 | 1 | | | 2 |

FIG.24

LISTS OF CORRELATION COEFFICIENTS (r) OF THE FEATURE PARAMETERS WITH NYHA

| NYHA | score | Face vector embdiff_dod | Mouth m_gradl | Eye EAR | Eye W80fcnt | Face pitch Pitch | NIR Adiff | Face width W10_24 | Face width W15_19 | Face area Area_all |
|---|---|---|---|---|---|---|---|---|---|---|
| G-HF001 | 8 | 0.687358873 | -0.76079204 | -0.79570645 | 0.722793713 | 0.831138764 | | 0.836746968 | 0.800610627 | 0.731610155 |
| G-HF002 | 7 | 0.727902808 | -0.80827185 | -0.8236351 | 0.509236339 | 0.846469615 | | 0.802538515 | 0.69046526 | 0.897359874 |
| G-HF003 | 0 | 0.385848887 | -0.25057758 | -0.56418508 | 0.213018173 | 0.571065651 | | -0.14264767 | 0.215868449 | -0.17228208 |
| G-HF004 | 2 | 0.731153406 | -0.10507966 | -0.32906233 | 0.757612665 | 0.331345884 | | 0.023588705 | -0.44448568 | -0.48122987 |
| G-HF005 | 6 | 0.743489205 | 0.13084053 | -0.79339853 | 0.843857728 | 0.804083569 | | 0.885243029 | 0.782106369 | 0.49595576 |
| G-HF006 | 0 | 0.421056035 | -0.2491671 | -0.18634625 | -0.10680895 | 0.067598627 | | -0.41430362 | -0.37294585 | -0.55771812 |
| G-HF007 | 2 | 0.803289067 | -0.33849574 | -0.41899148 | 0.608304462 | -0.05534459 | | 0.340210064 | -0.03343119 | -0.00092865 |
| G-HF008 | 1 | 0.56619959 | 0.505242419 | 0.359982675 | -0.21768086 | -0.57066448 | | 0.612245775 | -0.07577404 | -0.15406247 |
| G-HF009 | 7 | 0.318213421 | -0.64462146 | -0.73614178 | 0.748630745 | 0.678133551 | | 0.912631984 | 0.866694818 | 0.828833385 |
| G-HF010 | 1 | -0.18112604 | 0.567474996 | -0.04582559 | 0.681206124 | -0.47973525 | | -0.42872076 | -0.48323808 | -0.35337754 |
| G-HF011 | 0 | 0.523324421 | 0.008811914 | -0.40117929 | 0.546745703 | 0.028466022 | | -0.08070129 | -0.26514124 | -0.25225285 |
| G-HF012 | 5 | 0.875920478 | -0.15522405 | -0.76554922 | -0.5158362 | 0.761389838 | 0.601535299 | 0.519622459 | 0.753623254 | 0.508775243 |
| G-HF013 | 1 | 0.993589201 | 0.006856328 | -0.48026585 | 0.163108699 | 0.307545788 | 0.537513635 | 0.557983534 | -0.07326967 | 0.386353014 |
| G-HF014 | 3 | 0.547145564 | -0.77095951 | -0.23390602 | -0.41214215 | 0.613410392 | 0.850447526 | -0.58551299 | -0.66417858 | 0.141285292 |
| G-HF015 | 4 | 0.63923294 | -0.44501067 | 0.504740771 | 0.209108968 | 0.892392609 | 0.865026219 | 0.400465043 | 0.566986958 | 0.765171134 |
| G-HF016 | 4 | 0.744642212 | -0.65518128 | -0.73381069 | 0.858487947 | 0.49361294 | 0.203287352 | -0.4196309 | 0.267705129 | -0.63109 |
| G-HF017 | 7 | 0.988762734 | -0.89327286 | -0.80447363 | 0.124521371 | 0.936038902 | 0.782949818 | 0.596399484 | 0.909984133 | 0.900628407 |
| G-HF018 | 4 | 0.745099978 | -0.91705188 | 0.986112108 | 0.819039088 | -0.05898087 | 0.783544772 | 0.427403949 | -0.7713356 | 0.527148324 |
| G-HF019 | 3 | 0.386915862 | -0.55327626 | -0.62510011 | 0.508731559 | 0.612480198 | 0.614269468 | -0.31308781 | -0.30108547 | -0.12831269 |
| G-HF020 | 2 | 0.699843247 | -0.32828397 | -0.5907205 | 0.911533165 | 0.368719044 | 0.439740373 | 0.126426728 | 0.34391796 | -0.0914722 |
| G-HF021 | 7 | 0.923244112 | -0.87078119 | -0.88980649 | 0.873662327 | 0.912671481 | 0.801630813 | 0.097537695 | 0.385001579 | 0.82652158 |
| G-HF022 | 2 | 0.940022475 | 0.386629981 | 0.217355556 | 0.135104922 | -0.34876913 | 0.71984119 | -0.29958863 | 0.14553147 | -0.3467065 |
| G-HF023 | 7 | 0.913429364 | -0.9513533 | -0.80805007 | -0.32528761 | 0.991150928 | 0.727948512 | -0.35170293 | 0.929808257 | 0.659201817 |
| G-HF024 | 6 | 0.109999033 | -0.93608135 | -0.97822413 | -0.96279444 | 0.668169655 | 0.843202141 | 0.733683063 | 0.872096494 | 0.571080906 |
| G-HF025 | 4 | 0.697583467 | 0.176849871 | 0.322095959 | -0.02110902 | 0.10086876 | 0.39253132 | 0.840889045 | 0.791560409 | 0.692985264 |
| G-HF026 | 1 | 0.288917274 | 0.450801896 | -0.04512727 | 0.828579599 | -0.51595366 | 0.399609669 | 0.008975389 | -0.37626838 | -0.19857112 |
| G-HF027 | 6 | 0.794908034 | -0.6414681 | -0.60211362 | -0.89810733 | 0.636065128 | 0.780310679 | -0.07447812 | -0.31091323 | 0.661327185 |
| G-HF028 | 2 | 0.888071057 | 0.121592513 | -0.27931078 | -0.42563091 | -0.15494093 | 0.930814076 | -0.06156289 | -0.79490175 | -0.1583424 |
| G-HF029 | 6 | 0.704680857 | -0.82178561 | -0.50497016 | -0.43044376 | 0.883887696 | 0.335892802 | 0.904541785 | 0.927605452 | 0.879385831 |
| G-HF030 | 2 | 0.555316926 | -0.27567262 | -0.34205373 | -0.04043887 | 0.706164857 | 0.833691889 | 0.360896057 | 0.171851961 | 0.438739474 |
| G-HF031 | 1 | -0.14557814 | -0.28013493 | -0.09203141 | -0.64539605 | 0.387210617 | -0.062888383 | 0.016434744 | 0.667257429 | 0.152780058 |
| G-HF032 | 3 | 0.739158369 | -0.47979449 | -0.68907179 | -0.30029311 | 0.168343605 | 0.83029235 | -0.44829995 | 0.493221961 | -0.37684007 |
| G-HF033 | 6 | 0.925283674 | -0.8230237 | -0.84730331 | 0.814677217 | 0.839150802 | 0.705511818 | 0.283244159 | 0.494400181 | 0.494521363 |
| G-HF034 | 0 | 0.336921009 | -0.27442354 | -0.46443741 | -0.39177578 | 0.227521908 | 0.340810923 | 0.11507577 | 0.37149865 | 0.131913535 |
| G-HF035 | 1 | 0.251955947 | -0.27622431 | -0.53692085 | 0.146223468 | -0.09361159 | 0.687312916 | -0.64088732 | -0.73119333 | -0.27073819 |
| G-HF036 | 1 | 0.50392321 | -0.2542189 | -0.49979453 | 0.373804242 | 0.320429784 | 0.830910472 | -0.53403395 | -0.23208018 | -0.10088817 |
| G-HF037 | 4 | -0.44956692 | -0.53648608 | -0.80670033 | -0.16701766 | 0.577048066 | 0.468718695 | 0.747588908 | 0.827836571 | 0.69254698 |
| G-HF038 | 2 | -0.07736757 | -0.78351222 | -0.98460645 | 0.125658189 | 0.454447702 | | -0.3537202 | 0.410497441 | 0.597724367 |
| score addition condition | | r≧0.6 | r≦−0.6 | r≦−0.6 | r≧0.6 | r≧0.6 | r≧0.6 | r≧0.6 | r≧0.6 | r≧0.6 |

FIG.25

| Weight | score | Face Vector | Mouth | Eye | Eye | Face Pitch | NIR | Face Width | Face width | Face Area |
|---|---|---|---|---|---|---|---|---|---|---|
| | | embdiff_dod | m_gradl | EAR | W80fcnt | Pitch | Adiff | W10_24 | W15_19 | Area_all |
| G-HF001 | 4 | 0.54508853 | -0.87572176 | -0.87757816 | 0.225822268 | 0.838825844 | | 0.674222001 | 0.560928853 | 0.51918917 |
| G-HF002 | 7 | 0.545274567 | -0.70943718 | -0.88904578 | 0.721493108 | 0.70469349 | | 0.600295206 | 0.714729592 | 0.653049249 |
| G-HF003 | 0 | 0.486652689 | -0.14196442 | -0.59374688 | 0.250355001 | 0.50065712 | | 0.03319805 | 0.200542988 | -0.23107216 |
| G-HF004 | 2 | 0.719157481 | -0.24669973 | -0.25302638 | 0.749811867 | 0.450297718 | | -0.24383804 | -0.56905198 | -0.4683502 |
| G-HF005 | 7 | 0.654809498 | 0.15647616 | -0.816291 | 0.74925079 | 0.831468703 | | 0.891387644 | 0.814615906 | 0.668009279 |
| G-HF006 | 0 | 0.528683817 | -0.08972512 | -0.07648537 | -0.35538631 | 0.016427034 | | -0.74986353 | -0.76322279 | -0.70221915 |
| G-HF007 | 3 | 0.854673264 | 0.448979421 | -0.75918364 | 0.987315026 | -0.02100261 | | 0.050420281 | -0.3042233 | 0.033892308 |
| G-HF008 | 0 | 0.56795521 | 0.414205419 | 0.448428112 | -0.11640504 | -0.57276516 | | 0.526095705 | -0.34816602 | -0.19423673 |
| G-HF009 | 6 | -0.11621938 | -0.85802544 | -0.9239896 | 0.53729081 | 0.887585851 | | 0.938045065 | 0.932319384 | 0.909417299 |
| G-HF010 | 0 | -0.46268946 | 0.116514964 | -0.43782247 | 0.28319651 | 0.095229789 | | 0.538632946 | 0.527128142 | 0.541233805 |
| G-HF011 | 1 | 0.822138473 | -0.06432768 | 0.200788391 | -0.09289548 | -0.13810393 | | -0.42862907 | -0.78828729 | -0.59656175 |
| G-HF012 | 6 | 0.863912206 | -0.31805529 | -0.86310667 | -0.29717927 | 0.908092913 | 0.583556052 | 0.75732203 | 0.732597142 | 0.62167819 |
| G-HF013 | 2 | 0.963881109 | 0.235564299 | -0.71328907 | 0.429021722 | 0.252823636 | 0.434130199 | 0.44633152 | -0.11403732 | 0.239925875 |
| G-HF014 | 5 | 0.865735631 | -0.91887089 | -0.72855844 | 0.488668629 | 0.870415275 | 0.844432263 | -0.6357878 | -0.7434328 | 0.470846721 |
| G-HF015 | 4 | 0.923703885 | -0.32626138 | 0.382846823 | 0.500381279 | 0.909721214 | 0.916247653 | 0.256334269 | 0.489339143 | 0.637908725 |
| G-HF016 | 2 | 0.591639555 | -0.58746659 | -0.71359597 | 0.705399835 | 0.487176446 | 0.302241317 | -0.22084627 | 0.45046676 | -0.425305 |
| G-HF017 | 7 | 0.898563903 | -0.71347936 | -0.77815533 | 0.064021981 | 0.769416046 | 0.90937547 | 0.453859374 | 0.741093663 | 0.6569153 |
| G-HF018 | 3 | 0.906111577 | -0.89739355 | 0.752486359 | 0.570412107 | 0.259905396 | 0.842885037 | 0.197167102 | -0.76358877 | 0.3960141 |
| G-HF019 | 6 | 0.857258989 | -0.92617268 | -0.9889677 | 0.999733049 | 0.990482033 | 0.999174941 | -0.96938246 | -0.68713375 | -0.633832 |
| G-HF020 | 2 | 0.622298736 | -0.12351009 | -0.29092614 | 0.799321457 | 0.137320307 | 0.258703698 | 0.057683835 | 0.314466313 | -0.137361 |
| G-HF021 | 6 | 0.870573236 | -0.90567416 | -0.80194331 | 0.592788182 | 0.906898326 | 0.870198356 | -0.02454626 | 0.316050114 | 0.721117 |
| G-HF022 | 3 | 0.824766905 | 0.660674537 | -0.11184613 | 0.908556136 | -0.58444806 | 0.966609764 | 0.015764072 | 0.37540765 | -0.25202 |
| G-HF023 | 7 | 0.990569954 | -0.93187233 | -0.87434293 | -0.27023526 | 0.977361807 | 0.978231371 | -0.36280884 | 0.929461687 | 0.6818667 |
| G-HF024 | 3 | -0.31617158 | -0.78456011 | -0.91945796 | -0.95305302 | 0.435442619 | 0.49416718 | 0.505773867 | 0.686726311 | 0.5311776 |
| G-HF025 | 2 | 0.801554662 | -0.09924561 | 0.456137046 | 0.279044944 | 0.357382248 | 0.324220535 | 0.564831159 | 0.554701948 | 0.7986624 |
| G-HF026 | 2 | 0.258599752 | 0.424784749 | -0.07236809 | 0.966195022 | -0.44320097 | 0.624571958 | -0.03721927 | -0.46743247 | -0.051859 |
| G-HF027 | 6 | 0.868131551 | -0.76931684 | -0.82465318 | -0.90330407 | 0.768043748 | 0.953889269 | 0.155121359 | -0.22833964 | 0.742502228 |
| G-HF028 | 2 | 0.953045649 | -0.2884291 | -0.36591108 | -0.603469 | 0.287363931 | 0.863219741 | 0.064717225 | -0.52599653 | 0.223810943 |
| G-HF029 | 6 | 0.753636202 | -0.79658681 | -0.56943504 | -0.51274114 | 0.843079636 | 0.447672084 | 0.85986207 | 0.882625766 | 0.861416299 |
| G-HF030 | 6 | 0.994714787 | -0.87046774 | -0.75758484 | 0.932981079 | 0.916527078 | 0.861932274 | 0.247448236 | 0.116446355 | 0.291152077 |
| G-HF031 | 5 | 0.566640228 | -0.86346798 | -0.6255976 | -1 | 0.91079516 | -0.676329763 | -0.01160154 | 0.968131942 | 0.666971479 |
| G-HF032 | 3 | 0.769664393 | -0.47119918 | -0.82191825 | -0.07106436 | 0.31549476 | 0.887248561 | -0.53363872 | 0.404463744 | -0.38724125 |
| G-HF033 | 5 | 0.842284354 | -0.72660107 | -0.72058165 | 0.504160678 | 0.696025226 | 0.840115918 | 0.117583842 | 0.272832156 | 0.418060457 |
| G-HF034 | 2 | 0.323872382 | -0.40399309 | -0.43711728 | -0.43798297 | 0.383839865 | 0.826710612 | 0.296090608 | 0.880964171 | 0.467299345 |
| G-HF035 | 1 | 0.161592169 | -0.35600915 | -0.44237729 | 0.449223404 | 0.019269799 | 0.851289209 | -0.62829755 | -0.71456723 | -0.19006551 |
| G-HF036 | 1 | 0.568946946 | -0.37549728 | -0.46402374 | 0.151445324 | 0.371085743 | 0.863229579 | -0.4901964 | 0.014911765 | -0.05190088 |
| G-HF037 | 4 | -0.53784268 | -0.4447529 | -0.79981496 | -0.05671808 | 0.518431592 | 0.487450275 | 0.668066197 | 0.813200095 | 0.724965176 |
| G-HF038 | 2 | -0.05939854 | -0.79153733 | -0.99825029 | 0.165120118 | 0.468032264 | | -0.33816988 | 0.445527328 | 0.597380677 |
| score addition condition | | r≧0.6 | r≦-0.6 | r≦-0.6 | r≧0.6 | r≧0.6 | r≧0.6 | r≧0.6 | r≧0.6 | r≧0.6 |

FIG.26

LISTS OF CORRELATION COEFFICIENTS (r) OF THE FEATURE PARAMETERS WITH BNP

| | | Face Vector | Mouth | Eye | Eye | Face Pitch | NIR | Face Width | FACE WIDTH | Face Area |
|---|---|---|---|---|---|---|---|---|---|---|
| | score | embdiff_dod | m_gradl | EAR | W80FCNT | Pitch | Adiff | W10_24 | W15_19 | Area_all |
| G-HF001 | 5 | 0.371869292 | -0.86225681 | -0.87384677 | 0.273094407 | 0.835714249 | | 0.784227156 | 0.585390373 | 0.666533475 |
| G-HF002 | 3 | 0.463213432 | -0.58196324 | -0.72015661 | 0.718606695 | 0.563439499 | | 0.392088655 | 0.83155807 | 0.331398015 |
| G-HF003 | 4 | 0.396108436 | -0.31621977 | -0.8691745 | -0.4355498 | 0.957290586 | | 0.727857725 | 0.724234431 | 0.073217772 |
| G-HF004 | 1 | -0.26303381 | 0.710855085 | 0.432928226 | -0.71756777 | -0.81979183 | | 0.332615947 | 0.313998781 | 0.753064471 |
| G-HF005 | 3 | 0.863489302 | 0.012520242 | -0.49012508 | 0.992520134 | 0.490806113 | | 0.705455663 | 0.38538382 | 0.303899318 |
| G-HF006 | 1 | 0.996401249 | 0.743826781 | 0.962519803 | -1 | -0.79670251 | | -0.97374508 | -0.91639402 | -0.97565104 |
| G-HF007 | 3 | 0.700551133 | 0.106965007 | -0.68711745 | 0.944140534 | -0.29128981 | | 0.103005115 | -0.27137164 | -0.19951918 |
| G-HF008 | 2 | 0.753927964 | 0.373134949 | 0.915202694 | -0.50199197 | -0.3121827 | | 0.774708881 | -0.31624174 | 0.174926583 |
| G-HF009 | 6 | 0.0386733 | -0.72652944 | -0.7477566 | 0.522631803 | 0.705785448 | | 0.715491553 | 0.742206513 | 0.619203045 |
| G-HF010 | 1 | -0.34489642 | 0.20234488 | -0.25979212 | 0.614100266 | -0.14602869 | | -0.16117073 | -0.1993898 | -0.00689408 |
| G-HF011 | 5 | 0.306035977 | -0.98173902 | 0.136214209 | 0.107485415 | 0.99350947 | | 0.759755824 | 0.609025791 | 0.948604678 |
| G-HF012 | 3 | 0.567896034 | 0.202911274 | -0.62831978 | -0.82598385 | 0.228429438 | -0.227318059 | 0.106676441 | 0.810307162 | 0.931169453 |
| G-HF013 | 6 | 0.819777527 | -0.75308889 | -0.04613029 | -0.34534232 | 0.601013441 | 0.900461827 | 0.833576449 | 0.052743516 | 0.728235734 |
| G-HF014 | 3 | 0.339307522 | -0.81360504 | -0.12741164 | -0.24320947 | 0.709877423 | 0.829576888 | -0.5969582 | -0.66358818 | -0.79153001 |
| G-HF015 | 5 | 0.781478838 | -0.60782272 | 0.609619294 | 0.190041405 | 0.99754157 | 0.998788592 | 0.58584304 | 0.579494177 | 0.841667793 |
| G-HF016 | 4 | 0.577813114 | -0.90078466 | -0.98725427 | 0.897023305 | 0.79484176 | 0.345030933 | -0.74599026 | -0.29349239 | -0.61190026 |
| G-HF017 | 8 | 0.939306493 | -0.81425605 | -0.75494224 | 0.275480816 | 0.856855495 | 0.961904301 | 0.83582631 | 0.86407861 | 0.812908462 |
| G-HF018 | 3 | 0.63590354 | -0.77344263 | 0.909014286 | 0.880681408 | -0.09911223 | 0.54358424 | 0.598532736 | -0.78171096 | 0.55848511 |
| G-HF019 | 6 | 0.74050716 | -0.88890521 | -0.97056335 | 0.871731517 | 0.937852906 | 0.800169151 | -0.64156296 | -0.55225026 | -0.32293416 |
| G-HF020 | 4 | 0.820257589 | -0.46879992 | -0.55559404 | 0.864232682 | 0.6280103 | 0.481438606 | 0.515385162 | 0.744637649 | 0.521380308 |
| G-HF021 | 7 | 0.946622149 | -0.85747445 | -0.67720757 | -1 | 0.8443235 | 0.888641137 | 0.026431686 | 0.746503122 | 0.975358305 |
| G-HF022 | 2 | 0.869677719 | 0.933624003 | -0.13196429 | -1 | -0.99194011 | 0.967069378 | -0.063959 | 0.410013734 | -0.23380793 |
| G-HF023 | 7 | 0.953147028 | -0.84049528 | -0.68499757 | -0.79400195 | 0.903212661 | 0.88092448 | -0.44701371 | 0.90991924 | 0.853896235 |
| G-HF024 | 6 | 0.401599144 | -0.8737829 | -0.87744349 | -0.87008477 | 0.648091774 | 0.966366431 | 0.709492544 | 0.82390798 | 0.434872458 |
| G-HF025 | 0 | 0.449242441 | 0.134700177 | 0.722428989 | 0.501064606 | 0.153233982 | 0.38438652 | 0.105133769 | 0.066066663 | 0.57513833 |
| G-HF026 | 6 | -0.99981657 | -0.62046511 | -0.99133918 | -0.06339101 | 0.746530034 | 0.562893579 | 0.854027444 | 0.915147205 | 0.686120905 |
| G-HF027 | 2 | -0.36989454 | 0.391671412 | 0.757804277 | 0.810980179 | -0.41732949 | 0.276836205 | -0.1478329 | 0.8165855 | -0.19273018 |
| G-HF028 | 3 | 0.778417991 | 0.499432205 | -0.83059222 | -0.54624321 | -0.4219352 | 0.817440032 | -0.1897931 | -0.72917159 | -0.26652524 |
| G-HF029 | 1 | 0.342762314 | -0.54598572 | -0.42010435 | 0.755952309 | 0.531808917 | -0.818191176 | -0.07529145 | 0.015513235 | -0.08682914 |
| G-HF030 | 7 | 0.994406009 | -0.8166997 | -0.86101883 | -1 | 0.999977089 | 0.986238667 | 0.653128714 | 0.470616545 | 0.721290614 |
| G-HF031 | 2 | 0.327776019 | -0.59387388 | -0.23264646 | -1 | 0.747026372 | -0.300164099 | -0.29455423 | 0.788993071 | 0.286273871 |
| G-HF032 | 3 | 0.700543352 | -0.32683359 | -0.75497624 | -0.07606158 | 0.221207817 | 0.888483524 | -0.58000508 | 0.427965778 | -0.37475552 |
| G-HF033 | 0 | -0.07513299 | 0.00162082 | 0.038309505 | -0.22675418 | -0.00818906 | 0.097280877 | -0.07924364 | 0.091472198 | 0.146276491 |
| G-HF034 | 1 | 0.119651602 | 0.342828498 | -0.78823088 | -0.99971031 | 0.055963846 | -0.103487005 | 0.560241337 | 0.153673583 | 0.366412419 |
| G-HF035 | 3 | 0.776215223 | -0.7923917 | -0.25254089 | 0.088466913 | 0.529338294 | 0.889173573 | -0.29829839 | -0.46890175 | 0.066166357 |
| G-HF036 | 1 | 0.137440456 | 0.488787037 | -0.0128059 | 0.021479974 | -0.61704442 | 0.617053921 | -0.9453206 | -0.85255893 | -0.81375695 |
| G-HF037 | 1 | -0.31997169 | -0.14473 | -0.17866619 | -0.66799759 | 0.174077548 | 0.307818791 | 0.72720156 | 0.371309033 | 0.442021677 |
| G-HF038 | 0 | 0.396854679 | -0.22879273 | -0.5717689 | 0.321033253 | -0.03147963 | | -0.57022513 | -0.01206104 | -0.0700021 |
| score addition condition | | r≧0.6 | r≦−0.6 | r≦−0.6 | r≧0.6 | r≧0.6 | r≧0.6 | r≧0.6 | r≧0.6 | r≧0.6 |

# FIG.27-1

| | | Face vector | Mouth | Eye | Eye | Face pitch | NIR | Face width | Face width | Face area |
|---|---|---|---|---|---|---|---|---|---|---|
| NYHA | score | embdiff_dod | m_gradl | EAR | W80fcnt | Pitch | Adiff | W10_24 | W15_19 | Area_all |
| G-HF001 | 7 | 0.82 | 0.60 | -0.80 | 0.72 | 0.82 | | 0.92 | 0.84 | 0.95 |
| G-HF002 | 6 | 0.78 | 0.59 | -0.87 | 0.42 | 0.83 | | 0.93 | 0.70 | 0.92 |
| G-HF003 | 1 | 0.60 | 0.50 | -0.62 | 0.36 | 0.49 | | -0.49 | -0.80 | -0.72 |
| G-HF004 | 2 | 0.50 | 0.08 | -0.53 | 0.78 | 0.68 | | 0.14 | -0.35 | 0.18 |
| G-HF005 | 4 | 0.61 | -0.40 | 0.02 | -0.13 | 0.58 | | 0.68 | 0.83 | 0.83 |
| G-HF006 | 0 | 0.22 | -0.32 | 0.13 | -0.38 | 0.26 | | -0.14 | -0.14 | 0.40 |
| G-HF007 | 3 | 0.75 | 0.16 | -0.60 | 0.39 | 0.63 | | 0.39 | -0.19 | -0.14 |
| G-HF008 | 0 | 0.54 | -0.27 | 0.46 | -0.36 | -0.65 | | 0.11 | -0.14 | 0.14 |
| G-HF009 | 6 | 0.36 | 0.44 | -0.78 | 0.71 | 0.82 | | 0.78 | 0.86 | 0.80 |
| G-HF010 | 2 | 0.43 | -0.27 | -0.68 | 0.87 | -0.01 | | 0.21 | -0.14 | -0.30 |
| G-HF011 | 2 | 0.61 | -0.13 | -0.28 | 0.54 | 0.21 | | 0.64 | 0.36 | 0.30 |
| G-HF012 | 5 | 0.84 | 0.63 | -0.72 | -0.54 | 0.71 | 0.63 | -0.01 | -0.05 | -0.19 |
| G-HF013 | 3 | 0.99 | -0.28 | -0.67 | 0.20 | 0.72 | 0.56 | 0.00 | 0.26 | -0.31 |
| G-HF014 | 1 | 0.60 | 0.43 | -0.44 | 0.47 | 0.14 | 0.86 | -0.49 | -0.51 | -0.32 |
| G-HF015 | 4 | 0.64 | -0.33 | 0.31 | 0.23 | 0.91 | 0.87 | -0.16 | 0.72 | 0.50 |
| G-HF016 | 3 | 0.75 | 0.36 | -0.78 | 0.94 | 0.25 | 0.20 | -0.81 | 0.51 | -0.86 |
| G-HF017 | 6 | 0.96 | 0.89 | -0.81 | 0.40 | 0.86 | 0.78 | 0.31 | 0.73 | 0.24 |
| G-HF018 | 3 | 0.75 | 0.41 | 0.98 | 0.83 | -0.05 | 0.78 | 0.57 | -0.17 | 0.25 |
| G-HF019 | 3 | 0.39 | 0.55 | -0.63 | 0.51 | 0.61 | 0.61 | -0.31 | -0.30 | -0.13 |
| G-HF020 | 3 | 0.70 | 0.76 | -0.60 | 0.95 | 0.55 | 0.44 | 0.16 | 0.29 | -0.10 |
| G-HF021 | 5 | 0.88 | 0.27 | -0.86 | 0.82 | 0.76 | 0.70 | -0.17 | 0.00 | -0.18 |
| G-HF022 | 2 | 0.92 | 0.40 | 0.24 | 0.22 | -0.13 | 0.72 | -0.16 | 0.16 | -0.46 |
| G-HF023 | 3 | 0.92 | -0.40 | -0.26 | -0.03 | 0.85 | 0.73 | -0.94 | -0.68 | -0.96 |
| G-HF024 | 6 | 0.09 | 0.74 | -0.98 | -0.28 | 0.57 | 0.84 | 0.62 | 0.88 | 0.84 |
| G-HF025 | 3 | 0.68 | -0.04 | 0.06 | 0.04 | 0.87 | 0.39 | 0.40 | 0.75 | 0.24 |
| G-HF026 | 0 | 0.45 | 0.11 | 0.10 | -0.41 | -0.71 | 0.40 | -0.67 | -0.71 | -0.72 |
| G-HF027 | 4 | 0.80 | 0.49 | -0.61 | -0.22 | 0.66 | 0.78 | 0.11 | 0.31 | 0.36 |
| G-HF028 | 2 | 0.90 | -0.32 | -0.21 | -0.39 | -0.26 | 0.93 | -0.10 | -0.42 | -0.45 |
| G-HF029 | 6 | 0.70 | 0.83 | -0.48 | -0.30 | 0.85 | 0.34 | 0.79 | 0.84 | 0.69 |
| G-HF030 | 4 | 0.56 | -0.08 | -0.45 | 0.53 | 0.86 | 0.83 | 0.70 | 0.61 | -0.36 |
| G-HF031 | 1 | -0.16 | -0.37 | -0.47 | 1.00 | 0.33 | -0.06 | -0.92 | -0.63 | -1.00 |
| G-HF032 | 5 | 0.76 | 0.68 | -0.64 | 0.38 | -0.20 | 0.83 | 0.52 | 0.63 | 0.47 |
| G-HF033 | 5 | 0.92 | -0.76 | -0.88 | 0.86 | 0.92 | 0.71 | 0.47 | 0.46 | 0.49 |
| G-HF034 | 0 | 0.35 | 0.15 | -0.36 | -0.29 | -0.42 | 0.34 | 0.08 | 0.35 | 0.20 |
| G-HF035 | 1 | 0.47 | -0.08 | -0.24 | -0.38 | -0.61 | 0.74 | -0.51 | -0.50 | -0.41 |
| G-HF036 | 1 | 0.49 | 0.28 | -0.49 | 0.56 | 0.06 | 0.83 | 0.06 | 0.41 | 0.35 |
| G-HF037 | 2 | -0.36 | -0.04 | -0.82 | 0.37 | 0.79 | 0.35 | 0.14 | 0.27 | 0.10 |
| G-HF038 | 3 | -0.08 | 0.69 | -0.72 | 0.45 | 0.19 | 0.67 | 0.08 | 0.15 | -0.10 |
| G-HF039 | 4 | 0.83 | 0.71 | -0.87 | -0.32 | 0.69 | -0.79 | -0.19 | 0.32 | -0.29 |
| G-HF040 | 1 | 0.62 | 0.20 | 0.59 | -0.69 | 0.21 | 0.30 | 0.16 | 0.07 | 0.32 |
| G-HF041 | 5 | 0.80 | -0.03 | -0.57 | 0.33 | 0.84 | -0.13 | 0.75 | 0.74 | 0.70 |
| G-HF042 | | | | | | | | | | |
| G-HF043 | 7 | 0.83 | 0.81 | -0.67 | 0.51 | 0.42 | 0.69 | 0.95 | 0.98 | 0.99 |
| G-HF044 | 2 | 0.75 | -0.47 | 0.88 | 0.64 | -0.63 | 0.46 | -0.53 | -0.48 | -0.34 |
| G-HF045 | 2 | 0.61 | -0.49 | -0.31 | -0.32 | 0.64 | 0.46 | 0.35 | -0.29 | -0.05 |
| G-HF046 | 3 | 0.86 | 0.62 | -0.79 | -0.17 | -0.55 | 0.55 | 0.46 | 0.47 | 0.37 |
| G-HF047 | 3 | 0.19 | 0.43 | 0.17 | 0.11 | 0.11 | -0.08 | 0.74 | 0.96 | 0.94 |
| G-HF048 | 1 | 0.25 | -0.94 | 0.23 | -0.71 | -0.76 | 0.88 | -0.69 | -0.82 | -0.81 |
| G-HF049 | 4 | 0.54 | 0.96 | -0.49 | 0.07 | 0.18 | 0.69 | 0.58 | 0.86 | 0.90 |
| G-HF050 | 2 | 0.94 | 0.11 | 0.07 | -0.22 | 0.07 | 0.68 | -0.81 | -0.61 | -0.72 |
| G-HF051 | | | | | | | | | | |
| G-HF052 | 5 | 0.97 | 0.95 | -0.94 | 0.24 | 0.93 | -0.85 | 0.39 | 0.93 | 0.29 |
| G-HF053 | | | | | | | | | | |
| G-HF054 | 8 | 0.71 | 0.94 | -0.86 | -0.68 | 0.79 | 1.00 | 0.80 | 0.72 | 0.87 |
| G-HF055 | | | | | | | | | | |
| G-HF056 | 4 | 0.82 | -0.70 | 0.96 | -0.83 | 0.52 | -0.22 | 0.69 | 0.74 | 0.74 |
| G-HF057 | 3 | 0.76 | -0.17 | 0.20 | -0.44 | 0.81 | 0.44 | 0.94 | -0.68 | 0.58 |
| G-HF058 | 2 | 0.36 | -0.28 | -0.77 | -0.03 | -0.12 | 0.81 | 0.36 | -0.55 | 0.05 |
| G-HF059 | 4 | -0.39 | -0.99 | -0.46 | 0.46 | -0.99 | 0.90 | 0.97 | 1.00 | 0.93 |
| G-HF060 | 6 | 0.69 | 0.91 | -0.59 | 0.03 | 0.90 | 0.27 | 0.77 | 0.93 | 0.97 |
| score addition condition | | r≧0.6 | r≧0.6 | r≦-0.6 | r≧0.6 | r≧0.6 | r≧0.6 | r≧0.6 | r≧0.6 | r≧0.6 |

THE GRAY CELLS WITH NO NUMBERS LISTED ARE NOT CALCULATED BECAUSE OF INSUFFICIENT USEFUL DATA.

FIG.27-2

| | | Face vector | Mouth | Eye | Eye | Face pitch | NIR | Face width | Face width | Face area |
|---|---|---|---|---|---|---|---|---|---|---|
| Weight | score | embdiff_dod | m_gradl | EAR | W80fcnt | Pitch | Adiff | W10_24 | W15_19 | Area_all |
| G-HF001 | 5 | 0.85 | 0.84 | -0.86 | 0.27 | 0.82 | | 0.53 | 0.67 | 0.49 |
| G-HF002 | 2 | 0.08 | 0.50 | -0.69 | 0.47 | 0.58 | | 0.48 | 0.75 | 0.37 |
| G-HF003 | 4 | 0.44 | 0.14 | -0.88 | 0.68 | 1.00 | | 0.99 | 0.15 | -0.99 |
| G-HF004 | 0 | -0.33 | -0.63 | 0.52 | -0.71 | -0.55 | | 0.03 | 0.44 | -0.28 |
| G-HF005 | 5 | 0.96 | -0.80 | -0.59 | 0.98 | 0.53 | | 0.66 | 0.79 | 0.61 |
| G-HF006 | 2 | -1.00 | -0.97 | 0.98 | -0.99 | 0.97 | | -0.99 | -0.97 | 0.88 |
| G-HF007 | 4 | 0.71 | -0.38 | -0.83 | 0.82 | 0.94 | | 0.39 | 0.09 | -0.18 |
| G-HF008 | 1 | 0.99 | -0.20 | 0.92 | -0.39 | -0.69 | | 0.50 | 0.01 | 0.09 |
| G-HF009 | 4 | -0.07 | 0.54 | -0.74 | 0.89 | 0.72 | | 0.51 | 0.71 | 0.54 |
| G-HF010 | 0 | 0.18 | -0.33 | 0.06 | 0.52 | -0.14 | | 0.45 | -0.22 | 0.42 |
| G-HF011 | 6 | 1.00 | 0.96 | 0.12 | 0.26 | 1.00 | | 1.00 | 1.00 | 1.00 |
| G-HF012 | 3 | -0.45 | 0.05 | -0.47 | 0.38 | -0.17 | -0.68 | 0.65 | 0.80 | 0.82 |
| G-HF013 | 2 | 0.91 | -0.24 | -0.44 | -0.16 | 0.58 | 0.91 | 0.47 | 0.54 | 0.08 |
| G-HF014 | 4 | 0.37 | 0.77 | -0.34 | 0.81 | 0.60 | 0.83 | -0.52 | -0.63 | -0.30 |
| G-HF015 | 4 | 0.78 | -0.37 | 0.30 | 0.24 | 0.99 | 1.00 | -0.08 | 0.70 | 0.49 |
| G-HF016 | 3 | 0.56 | 0.79 | -0.99 | 0.88 | 0.50 | 0.35 | -0.75 | 0.56 | -0.83 |
| G-HF017 | 8 | 0.89 | 0.88 | -0.79 | 0.42 | 0.83 | 0.97 | 0.79 | 0.91 | 0.67 |
| G-HF018 | 4 | 0.64 | -0.02 | 0.91 | 0.87 | -0.48 | 0.54 | 0.89 | 0.43 | 0.73 |
| G-HF019 | | | | | | | | | | |
| G-HF020 | 4 | 0.82 | 0.82 | -0.56 | 0.93 | 0.63 | 0.48 | -0.07 | 0.14 | 0.01 |
| G-HF021 | 5 | 0.83 | 0.47 | -0.95 | 0.93 | 0.95 | 0.80 | -0.70 | -0.40 | -0.80 |
| G-HF022 | 4 | 0.86 | 0.99 | -0.03 | -0.13 | -0.89 | 0.97 | 0.27 | 0.82 | -0.06 |
| G-HF023 | 3 | 0.95 | -0.50 | -0.03 | -0.47 | 0.88 | 0.88 | -0.98 | -0.85 | -0.98 |
| G-HF024 | 7 | 0.39 | 0.62 | -0.89 | -0.39 | 0.62 | 0.97 | 0.82 | 0.98 | 0.97 |
| G-HF025 | 2 | 0.46 | -0.02 | 0.45 | 0.69 | 0.84 | 0.38 | -0.35 | -0.07 | -0.32 |
| G-HF026 | 0 | 0.26 | 0.47 | -0.24 | -0.55 | -0.94 | 0.56 | -0.52 | -0.83 | -0.78 |
| G-HF027 | 1 | -0.35 | -0.84 | 0.70 | 0.63 | -0.59 | 0.28 | 0.52 | 0.30 | 0.26 |
| G-HF028 | 3 | 0.81 | -0.54 | -0.66 | -0.55 | -0.49 | 0.82 | -0.42 | -0.63 | -0.67 |
| G-HF029 | 2 | 0.35 | 0.22 | -0.47 | 0.69 | 0.63 | -0.82 | 0.29 | 0.32 | 0.25 |
| G-HF030 | 8 | 1.00 | 0.67 | -0.95 | 0.99 | 1.00 | 0.99 | 0.84 | 1.00 | -0.48 |
| G-HF031 | 1 | 0.31 | -0.24 | -0.58 | 0.98 | 0.45 | -0.30 | -0.97 | -0.52 | -0.98 |
| G-HF032 | 2 | 0.57 | 0.48 | -0.64 | 0.53 | -0.33 | 0.89 | 0.14 | 0.40 | 0.06 |
| G-HF033 | 0 | -0.10 | -0.15 | 0.02 | -0.26 | 0.00 | 0.10 | 0.26 | 0.49 | 0.27 |
| G-HF034 | 1 | 0.12 | -0.32 | -0.74 | -0.36 | -0.66 | -0.10 | -0.62 | -0.13 | -0.34 |
| G-HF035 | 2 | 0.61 | 0.43 | 0.92 | -0.42 | -0.13 | 0.83 | 0.22 | 0.21 | 0.26 |
| G-HF036 | 1 | 0.12 | -0.37 | 0.07 | 0.22 | -0.62 | 0.62 | -0.42 | -0.36 | 0.04 |
| G-HF037 | 1 | -0.26 | -0.24 | -0.46 | 0.22 | 0.68 | 0.37 | 0.09 | 0.00 | -0.05 |
| G-HF038 | 0 | -0.96 | 0.26 | -0.44 | 0.40 | 0.56 | 0.38 | -0.97 | 0.14 | -0.83 |
| G-HF039 | 1 | 0.32 | 0.75 | -0.51 | -0.51 | 0.24 | -0.86 | 0.53 | 0.53 | 0.24 |
| G-HF040 | 1 | 0.81 | 0.29 | 0.29 | -0.25 | -0.05 | -0.78 | 0.22 | -0.18 | 0.19 |
| G-HF041 | 5 | 0.96 | 0.19 | -0.59 | 0.27 | 0.69 | -0.31 | 0.74 | 0.83 | 0.91 |
| G-HF042 | | | | | | | | | | |
| G-HF043 | 7 | 0.95 | 0.87 | -0.68 | -0.03 | 0.56 | 0.94 | 0.83 | 0.91 | 0.81 |
| G-HF044 | 0 | 0.53 | 0.30 | 0.97 | 0.14 | -0.94 | 0.42 | 0.07 | -0.04 | 0.36 |
| G-HF045 | 2 | 0.07 | -0.47 | -0.98 | 0.70 | 0.22 | -0.21 | -0.23 | -0.24 | -0.31 |
| G-HF046 | 3 | 0.51 | 0.73 | -0.72 | -0.01 | -0.10 | 0.63 | 0.20 | 0.40 | 0.12 |
| G-HF047 | 0 | -0.74 | -0.27 | 0.26 | -0.25 | -0.96 | 0.57 | -0.97 | -0.97 | -0.96 |
| G-HF048 | 1 | 0.05 | -0.84 | -0.27 | -0.42 | -0.42 | 0.91 | -0.53 | -0.74 | -0.71 |
| G-HF049 | 7 | 0.73 | 0.84 | -0.68 | 0.32 | 0.40 | 0.67 | 0.79 | 0.97 | 0.97 |
| G-HF050 | 2 | 0.88 | 0.50 | -0.31 | -0.29 | -0.34 | 0.75 | -0.70 | -0.29 | -0.43 |
| G-HF051 | | | | | | | | | | |
| G-HF052 | 5 | 0.89 | 0.87 | -0.99 | 0.13 | 0.82 | -0.86 | 0.25 | 0.87 | 0.15 |
| G-HF053 | 1 | 0.98 | -0.93 | -0.15 | -0.99 | -0.72 | -0.91 | -0.97 | -0.93 | -0.99 |
| G-HF054 | 1 | -0.86 | 0.10 | -0.28 | 0.88 | 0.41 | -0.21 | -0.78 | -0.85 | -0.69 |
| G-HF055 | 4 | 0.79 | 0.50 | -0.13 | 0.29 | 0.89 | 0.83 | 0.36 | 0.64 | 0.51 |
| G-HF056 | 3 | 0.62 | -0.38 | 1.00 | -1.00 | 0.64 | 0.33 | 0.09 | 0.35 | 1.00 |
| G-HF057 | 2 | 0.29 | -0.66 | 0.69 | -0.85 | 0.37 | 0.85 | 0.63 | -0.97 | 0.06 |
| G-HF058 | 2 | 0.13 | 0.25 | -0.49 | 0.29 | 0.43 | 0.97 | 0.72 | -0.10 | 0.42 |
| G-HF059 | 3 | -0.88 | -0.72 | -0.55 | 0.77 | -0.54 | 0.82 | 0.55 | 0.70 | 0.47 |
| G-HF060 | 5 | 0.08 | 0.53 | -0.69 | -0.56 | 0.90 | 0.72 | 0.43 | 0.70 | 0.75 |
| score addition condition | | r≧0.6 | r≧0.6 | r≦-0.6 | r≧0.6 | r≧0.6 | r≧0.6 | r≧0.6 | r≧0.6 | r≧0.6 |

FIG.27-3

| | | Face vector | Mouth | Eye | Eye | Face pitch | NIR | Face width | Face width | Face area |
|---|---|---|---|---|---|---|---|---|---|---|
| BNP | score | embdiff_dod | m_gradl | EAR | W80fcnt | Pitch | Adiff | W10_24 | W15_19 | Area_all |
| G-HF001 | 4 | 0.16 | 0.60 | -0.78 | 0.13 | 0.73 | | 0.49 | 0.60 | 0.37 |
| G-HF002 | 5 | 0.53 | 0.50 | -0.88 | 0.52 | 0.69 | | 0.87 | 0.86 | 0.75 |
| G-HF003 | 1 | 0.44 | 0.25 | -0.69 | 0.37 | 0.54 | | -0.67 | -0.47 | -0.86 |
| G-HF004 | 1 | 0.78 | 0.15 | -0.55 | 0.60 | 0.58 | | 0.09 | -0.27 | 0.13 |
| G-HF005 | 3 | 0.48 | -0.26 | -0.07 | -0.06 | 0.50 | | 0.67 | 0.72 | 0.83 |
| G-HF006 | 2 | 0.65 | -0.46 | 0.27 | -0.53 | 0.42 | | -0.23 | -0.26 | 0.62 |
| G-HF007 | 4 | 0.90 | -0.41 | -0.89 | 0.98 | 0.96 | | 0.45 | 0.44 | 0.12 |
| G-HF008 | 0 | 0.47 | -0.13 | 0.56 | -0.30 | -0.70 | | 0.16 | -0.17 | 0.06 |
| G-HF009 | 7 | 0.03 | 0.69 | -0.95 | 0.94 | 0.91 | | 0.70 | 0.83 | 0.63 |
| G-HF010 | 1 | -0.13 | 0.07 | -0.30 | 0.58 | 0.21 | | 0.84 | 0.31 | 0.60 |
| G-HF011 | 2 | 0.82 | -0.15 | 0.50 | 0.25 | 0.21 | | 0.75 | 0.19 | 0.09 |
| G-HF012 | 4 | 0.84 | 0.76 | -0.86 | -0.30 | 0.81 | 0.47 | 0.35 | 0.15 | 0.08 |
| G-HF013 | 3 | 0.94 | -0.47 | -0.85 | 0.43 | 0.74 | 0.49 | -0.26 | 0.01 | -0.55 |
| G-HF014 | 3 | 0.34 | 0.81 | -0.13 | -0.24 | 0.71 | 0.93 | -0.60 | -0.66 | -0.79 |
| G-HF015 | 4 | 0.92 | -0.40 | 0.23 | 0.47 | 0.90 | 0.92 | -0.31 | 0.68 | 0.47 |
| G-HF016 | 4 | 0.60 | 0.35 | -0.75 | 0.83 | 0.32 | 0.30 | -0.73 | 0.63 | -0.80 |
| G-HF017 | 6 | 0.88 | 0.81 | -0.79 | 0.31 | 0.73 | 0.90 | 0.38 | 0.70 | 0.36 |
| G-HF018 | 3 | 0.91 | 0.72 | 0.73 | 0.51 | -0.03 | 0.84 | 0.46 | -0.30 | 0.18 |
| G-HF019 | 8 | 0.86 | -0.43 | -0.99 | 1.00 | 0.99 | 1.00 | 1.00 | 0.97 | 0.69 |
| G-HF020 | 2 | 0.61 | 0.52 | -0.30 | 0.89 | 0.46 | 0.26 | -0.06 | 0.04 | -0.38 |
| G-HF021 | 5 | 0.83 | 0.48 | -0.86 | 0.79 | 0.89 | 0.79 | -0.36 | -0.22 | -0.36 |
| G-HF022 | 2 | 0.83 | 0.44 | -0.03 | 0.18 | -0.41 | 0.97 | 0.28 | 0.46 | -0.07 |
| G-HF023 | 7 | 0.99 | 0.94 | -0.78 | 0.49 | -0.99 | 0.98 | 0.91 | 0.66 | 0.98 |
| G-HF024 | 2 | -0.35 | 0.66 | -0.91 | -0.22 | 0.25 | 0.49 | 0.17 | 0.55 | 0.49 |
| G-HF025 | 1 | 0.37 | 0.14 | 0.08 | 0.38 | 0.93 | 0.32 | 0.16 | 0.45 | 0.13 |
| G-HF026 | 1 | 0.28 | -0.11 | 0.01 | -0.13 | -0.72 | 0.62 | -0.56 | -0.73 | -0.76 |
| G-HF027 | 5 | 0.87 | 0.73 | -0.82 | -0.23 | 0.83 | 0.95 | 0.09 | 0.32 | 0.36 |
| G-HF028 | 2 | 0.95 | -0.04 | -0.36 | -0.57 | 0.18 | 0.86 | 0.34 | 0.02 | -0.01 |
| G-HF029 | 6 | 0.75 | 0.80 | -0.55 | -0.31 | 0.83 | 0.45 | 0.89 | 0.90 | 0.84 |
| G-HF030 | 8 | 0.99 | 0.66 | -0.88 | 1.00 | 0.77 | 0.86 | 0.89 | 0.98 | 0.09 |
| G-HF031 | 3 | 0.56 | 0.31 | -0.93 | 0.72 | 0.86 | -0.68 | -0.95 | 0.02 | -0.71 |
| G-HF032 | 5 | 0.74 | 0.67 | -0.80 | 0.60 | 0.01 | 0.89 | 0.47 | 0.64 | 0.40 |
| G-HF033 | 5 | 0.83 | -0.51 | -0.77 | 0.63 | 0.78 | 0.84 | 0.16 | 0.26 | 0.22 |
| G-HF034 | 4 | 0.34 | 0.15 | -0.40 | -0.61 | -0.44 | 0.83 | 0.62 | 0.85 | 0.77 |
| G-HF035 | 1 | 0.47 | -0.09 | -0.05 | -0.07 | -0.57 | 0.88 | -0.38 | -0.44 | -0.34 |
| G-HF036 | 1 | 0.56 | 0.41 | -0.48 | 0.36 | -0.04 | 0.86 | -0.16 | 0.41 | 0.11 |
| G-HF037 | 2 | -0.43 | -0.07 | -0.81 | 0.31 | 0.84 | 0.37 | 0.29 | 0.38 | 0.33 |
| G-HF038 | 4 | -0.07 | 0.77 | -0.91 | 0.65 | 0.25 | 0.69 | -0.26 | 0.03 | -0.42 |
| G-HF039 | 4 | 0.90 | 0.70 | -0.97 | 0.00 | 0.83 | -0.65 | -0.41 | 0.31 | -0.41 |
| G-HF040 | 1 | 0.81 | 0.40 | 0.07 | -0.29 | 0.20 | -0.20 | -0.25 | -0.38 | -0.10 |
| G-HF041 | 2 | 0.58 | 0.11 | -0.95 | 0.33 | 0.85 | 0.48 | 0.41 | 0.58 | 0.47 |
| G-HF042 | | | | | | | | | | |
| G-HF043 | 7 | 0.78 | 0.84 | -0.71 | 0.43 | 0.46 | 0.74 | 0.95 | 0.99 | 0.96 |
| G-HF044 | 2 | 0.55 | -0.64 | 0.84 | 0.73 | -0.88 | 0.78 | -0.60 | -0.43 | -0.40 |
| G-HF045 | 0 | 0.36 | -0.64 | -0.53 | -0.10 | 0.58 | 0.59 | 0.32 | 0.01 | 0.19 |
| G-HF046 | 2 | 0.58 | 0.40 | -0.93 | -0.27 | -0.41 | 0.76 | 0.10 | 0.00 | -0.05 |
| G-HF047 | 4 | 0.79 | 0.18 | -0.34 | 0.17 | 0.46 | -0.65 | 0.93 | 0.90 | 0.72 |
| G-HF048 | 1 | 0.13 | -0.91 | -0.36 | -0.60 | -0.71 | 0.88 | -0.53 | -0.69 | -0.66 |
| G-HF049 | 6 | 0.70 | 0.85 | -0.58 | 0.45 | 0.53 | 0.73 | 0.86 | 0.97 | 0.97 |
| G-HF050 | 2 | 0.90 | 0.36 | -0.20 | -0.07 | -0.27 | 0.73 | -0.77 | -0.55 | -0.65 |
| G-HF051 | | | | | | | | | | |
| G-HF052 | 5 | 0.95 | 0.91 | -0.90 | 0.20 | 0.90 | -0.86 | 0.35 | 0.88 | 0.26 |
| G-HF053 | 0 | 0.37 | 0.20 | -0.16 | -0.19 | -0.15 | 0.19 | 0.00 | 0.02 | -0.32 |
| G-HF054 | 8 | 0.80 | 0.88 | -0.78 | -0.78 | 0.69 | 0.98 | 0.88 | 0.81 | 0.93 |
| G-HF055 | 6 | 0.94 | 0.46 | -0.58 | 0.53 | 0.83 | 0.73 | 0.75 | 0.93 | 0.81 |
| G-HF056 | 0 | 0.48 | -0.31 | 0.77 | -0.96 | 0.35 | 0.26 | 0.36 | 0.37 | 0.46 |
| G-HF057 | 2 | 0.47 | -0.51 | 0.54 | -0.73 | 0.54 | 0.73 | 0.76 | -0.90 | 0.25 |
| G-HF058 | 2 | 0.25 | 0.35 | -0.37 | 0.26 | 0.46 | 0.95 | 0.67 | -0.04 | 0.35 |
| G-HF059 | 5 | -0.63 | -0.94 | -0.29 | 0.71 | -0.93 | 0.99 | 0.97 | 0.97 | 0.95 |
| G-HF060 | 5 | 0.41 | 0.59 | -0.90 | -0.27 | 0.94 | 0.01 | 0.95 | 0.98 | 0.96 |
| score addition condition | | $r \geq 0.6$ | $r \geq 0.6$ | $r \leq -0.6$ | $r \geq 0.6$ | $r \geq 0.6$ | $r \geq 0.6$ | $r \geq 0.6$ | $r \geq 0.6$ | $r \geq 0.6$ |

FIG.27-4

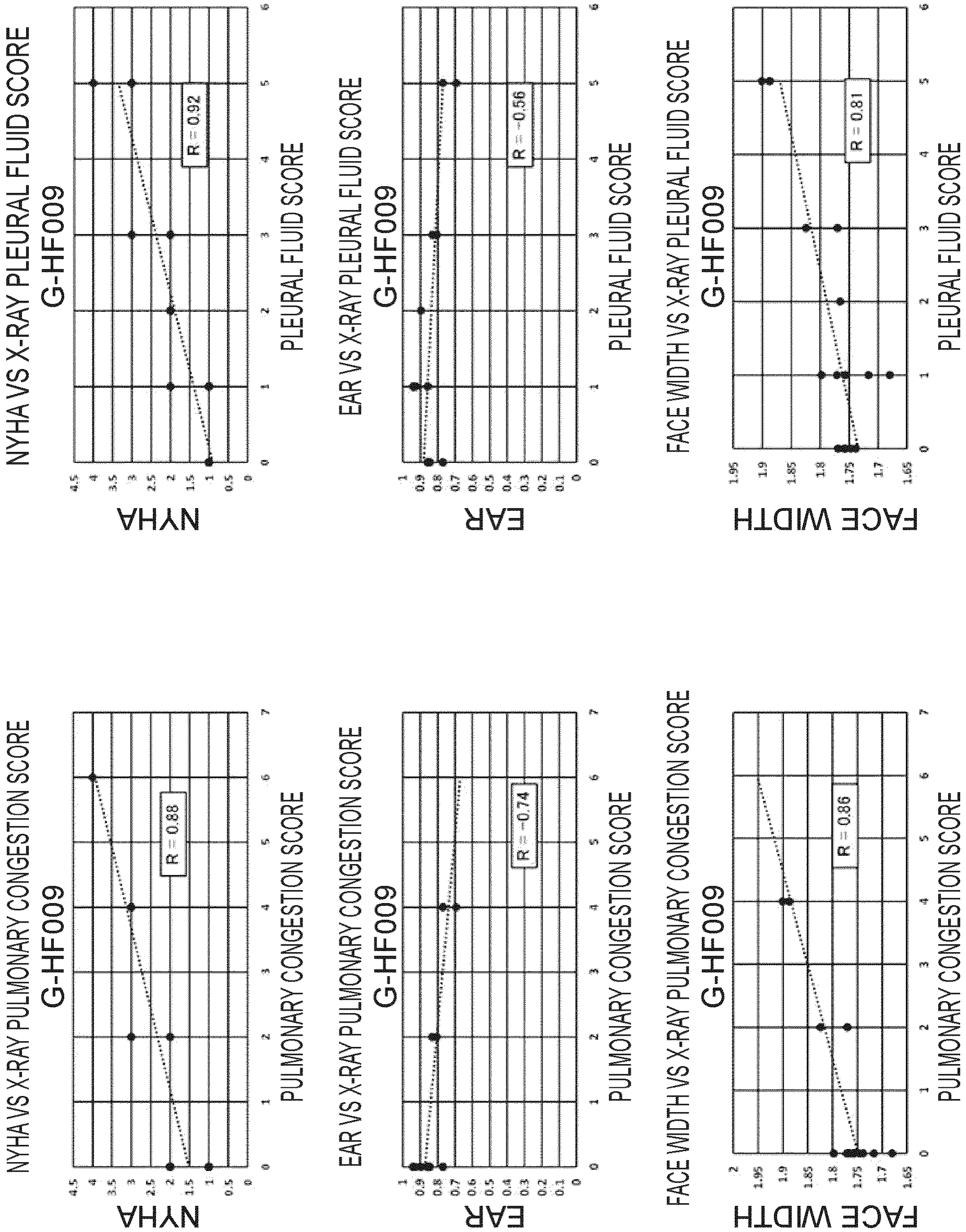
NYHA VS X-RAY PULMONARY CONGESTION SCORE
G-HF009
R = 0.88
NYHA
PULMONARY CONGESTION SCORE
EAR VS X-RAY PULMONARY CONGESTION SCORE
G-HF009
R = -0.74
EAR
PULMONARY CONGESTION SCORE
FACE WIDTH VS X-RAY PULMONARY CONGESTION SCORE
G-HF009
R = 0.86
FACE WIDTH
PULMONARY CONGESTION SCORE
NYHA VS X-RAY PLEURAL FLUID SCORE
G-HF009
R = 0.92
NYHA
PLEURAL FLUID SCORE
EAR VS X-RAY PLEURAL FLUID SCORE
G-HF009
R = -0.56
EAR
PLEURAL FLUID SCORE
FACE WIDTH VS X-RAY PLEURAL FLUID SCORE
G-HF009
R = 0.81
FACE WIDTH
PLEURAL FLUID SCORE

FIG.27-5

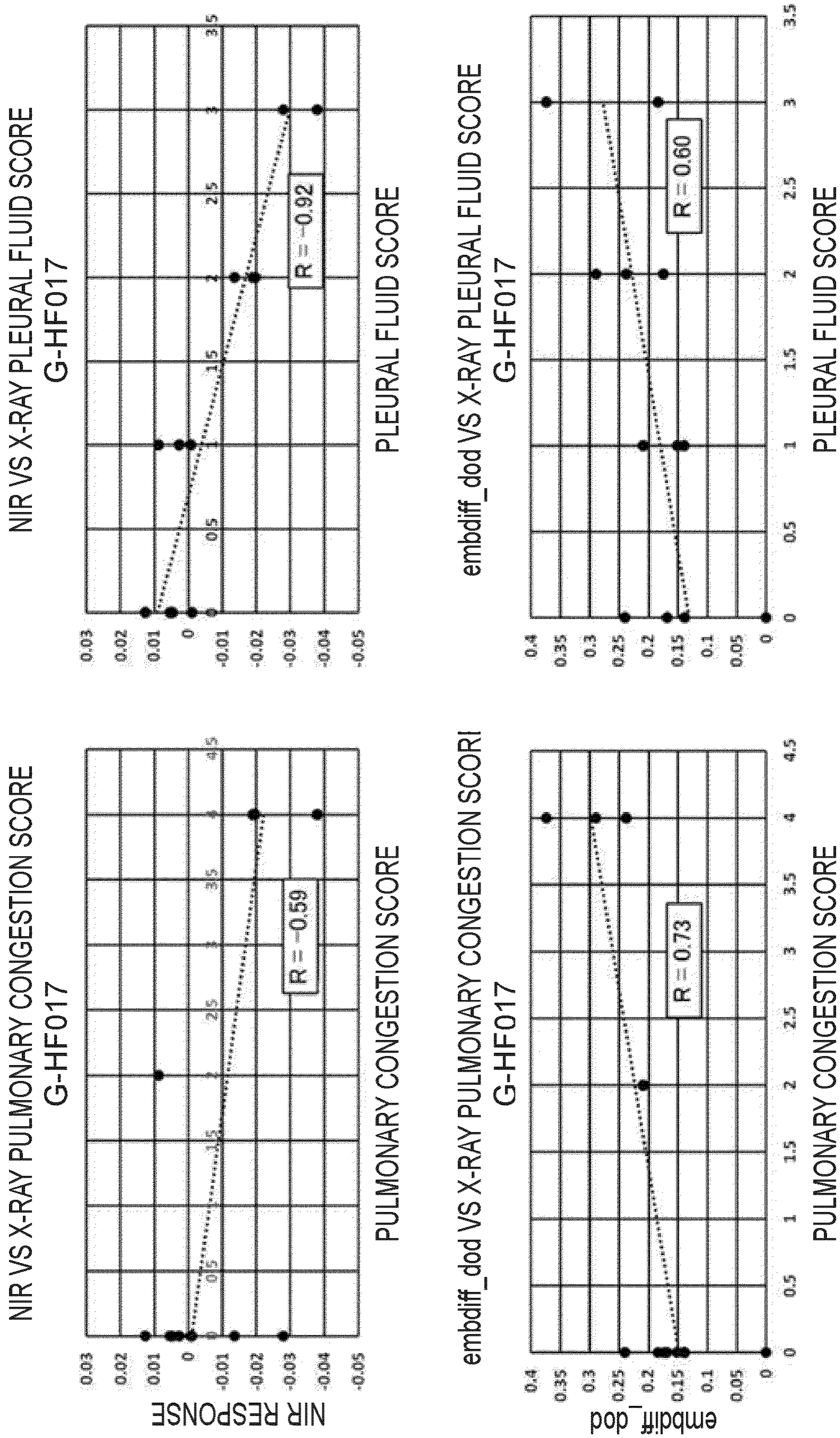
NIR VS X-RAY PULMONARY CONGESTION SCORE
G-HF017
R = −0.59
NIR RESPONSE
PULMONARY CONGESTION SCORE
NIR VS X-RAY PLEURAL FLUID SCORE
G-HF017
R = −0.92
PLEURAL FLUID SCORE
embdiff_dod VS X-RAY PULMONARY CONGESTION SCORI
G-HF017
R = 0.73
embdiff_dod
PULMONARY CONGESTION SCORE
embdiff_dod VS X-RAY PLEURAL FLUID SCORE
G-HF017
R = 0.60
PLEURAL FLUID SCORE

FIG.27-6

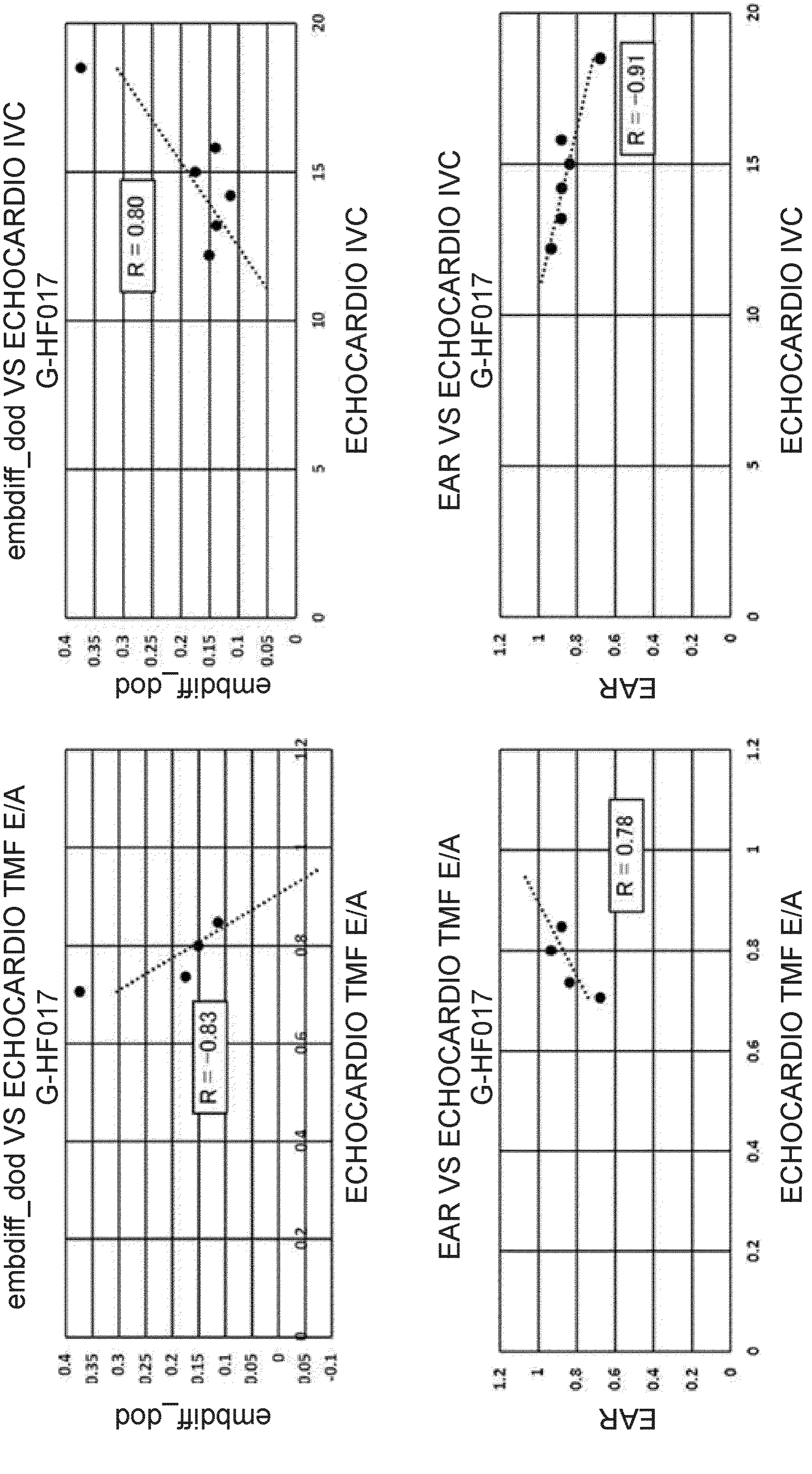
embdiff_dod VS ECHOCARDIO TMF E/A
G-HF017
R = -0.83
embdiff_dod
ECHOCARDIO TMF E/A
embdiff_dod VS ECHOCARDIO IVC
G-HF017
R = 0.80
embdiff_dod
ECHOCARDIO IVC
EAR VS ECHOCARDIO TMF E/A
G-HF017
R = 0.78
EAR
ECHOCARDIO TMF E/A
EAR VS ECHOCARDIO IVC
G-HF017
R = -0.91
EAR
ECHOCARDIO IVC

FIG.27-7

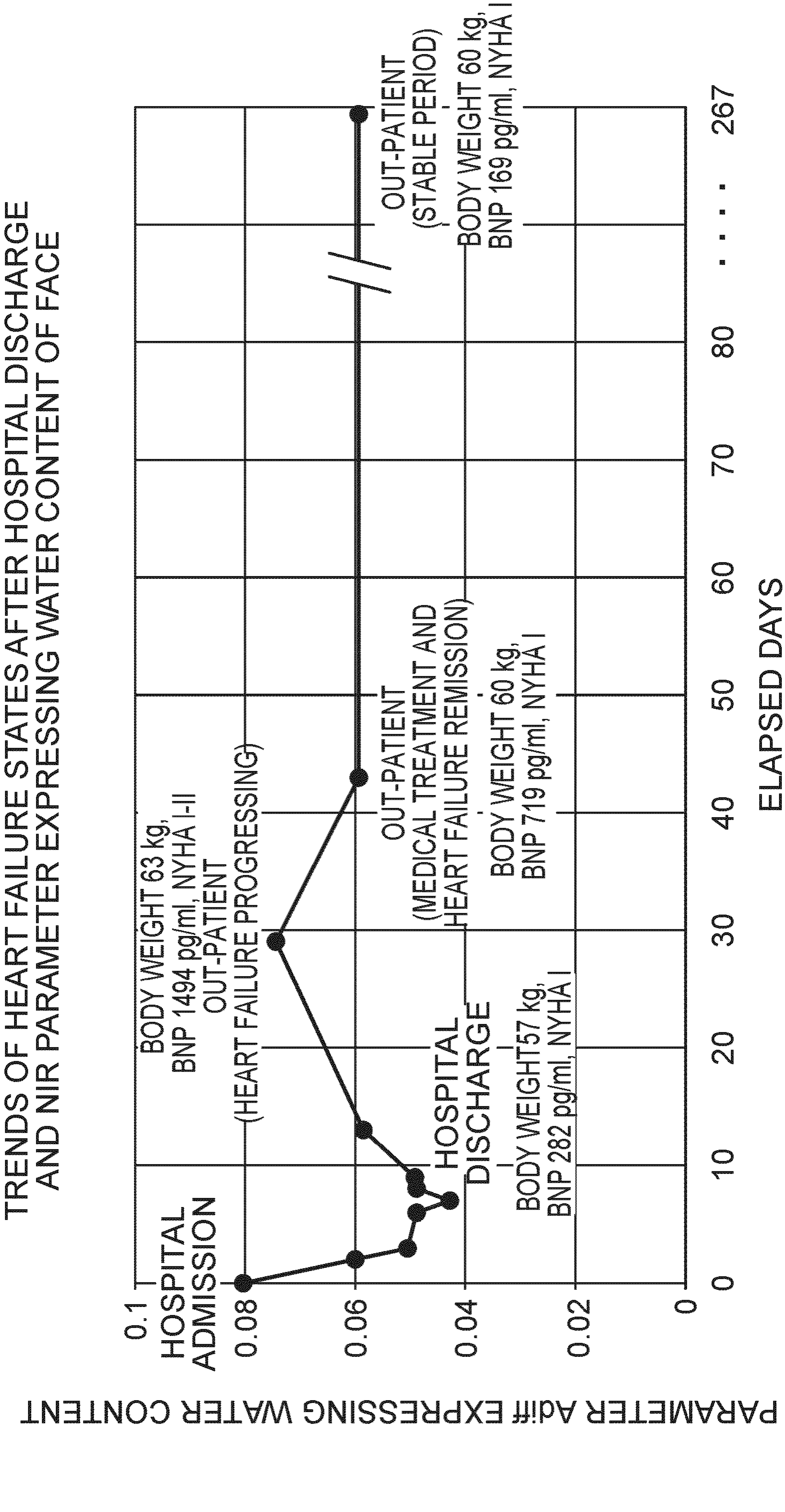
TRENDS OF HEART FAILURE STATES AFTER HOSPITAL DISCHARGE AND NIR PARAMETER EXPRESSING WATER CONTENT OF FACE
PARAMETER Adiff EXPRESSING WATER CONTENT
ELAPSED DAYS
0.1
0.08
0.06
0.04
0.02
0
0
10
20
30
40
50
60
70
80
267
HOSPITAL ADMISSION
HOSPITAL DISCHARGE
BODY WEIGHT57 kg, BNP 282 pg/ml, NYHA I
BODY WEIGHT 63 kg, BNP 1494 pg/ml, NYHA I-II
OUT-PATIENT (HEART FAILURE PROGRESSING)
OUT-PATIENT (MEDICAL TREATMENT AND HEART FAILURE REMISSION)
BODY WEIGHT 60 kg, BNP 719 pg/ml, NYHA I
OUT-PATIENT (STABLE PERIOD)
BODY WEIGHT 60 kg, BNP 169 pg/ml, NYHA I

FIG.27-8

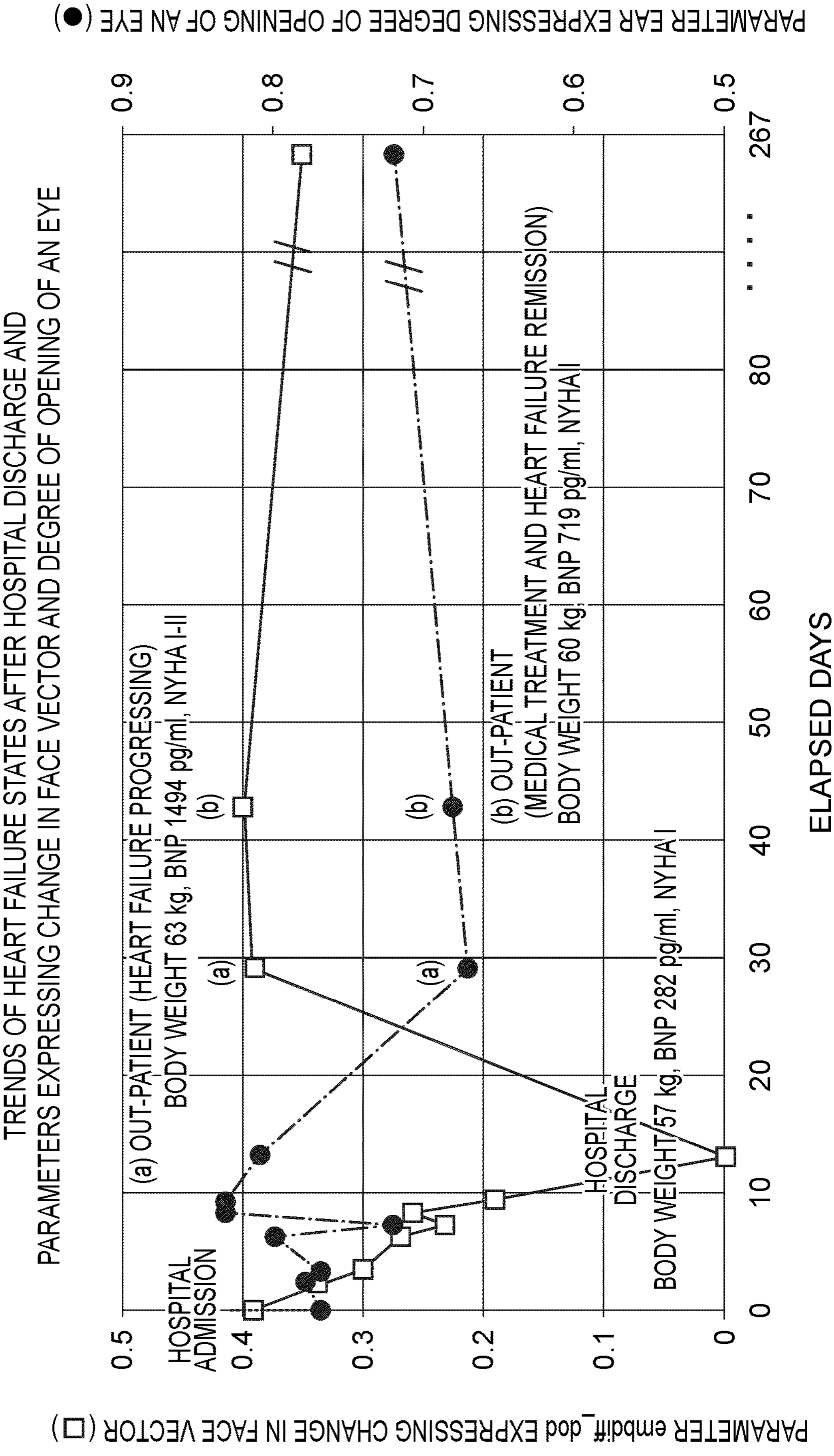
TRENDS OF HEART FAILURE STATES AFTER HOSPITAL DISCHARGE AND
PARAMETERS EXPRESSING CHANGE IN FACE VECTOR AND DEGREE OF OPENING OF AN EYE
PARAMETER embdiff_dod EXPRESSING CHANGE IN FACE VECTOR (□)
PARAMETER EAR EXPRESSING DEGREE OF OPENING OF AN EYE (●)
ELAPSED DAYS
0
0.1
0.2
0.3
0.4
0.5
0.5
0.6
0.7
0.8
0.9
0
10
20
30
40
50
60
70
80
267
HOSPITAL ADMISSION
HOSPITAL DISCHARGE
BODY WEIGHT 57 kg, BNP 282 pg/ml, NYHA I
(a) OUT-PATIENT (HEART FAILURE PROGRESSING)
BODY WEIGHT 63 kg, BNP 1494 pg/ml, NYHA I-II
(b) OUT-PATIENT
(MEDICAL TREATMENT AND HEART FAILURE REMISSION)
BODY WEIGHT 60 kg, BNP 719 pg/ml, NYHA I
(a)
(b)
(a)
(b)

FIG.27-9

TRENDS OF PARAMETER EXPRESSING WATER CONTENT
AND PARAMETER EXPRESSING DEGREE OF OPENING OF AN EYE

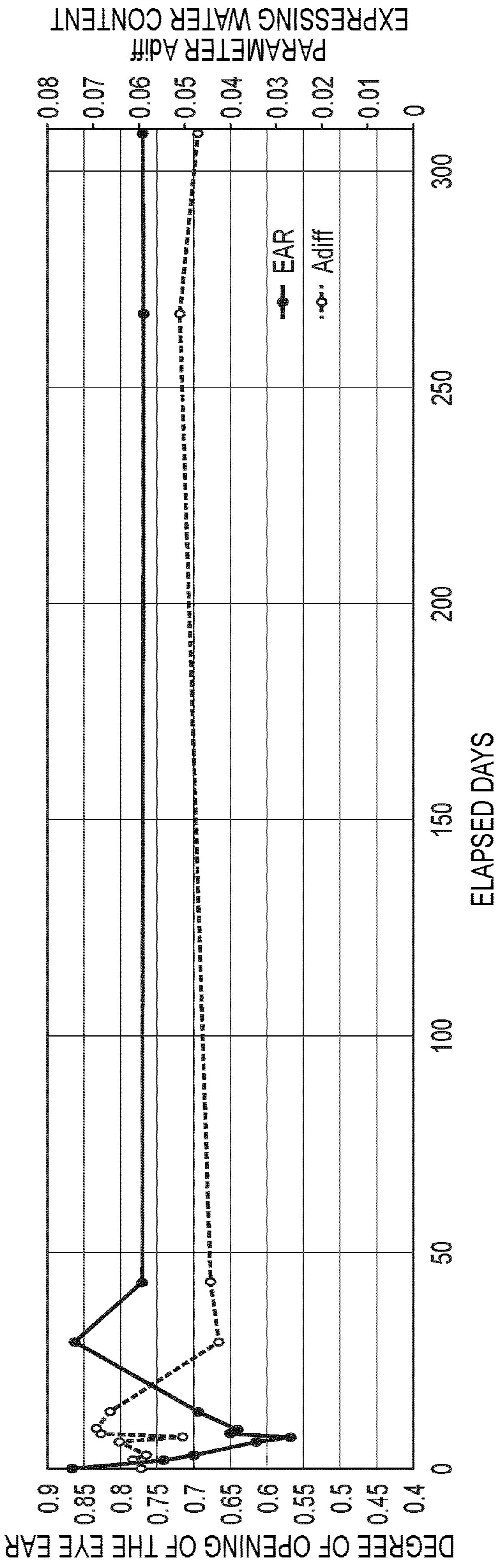

FIG.27-10

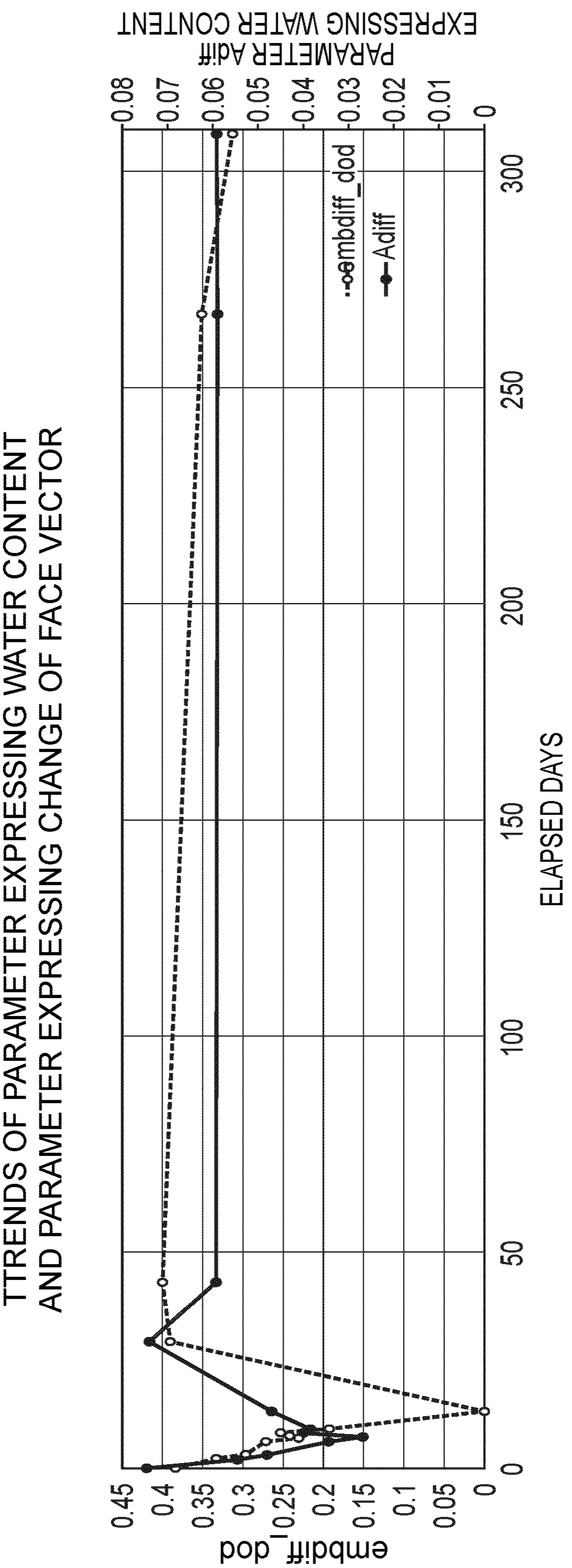
TTRENDS OF PARAMETER EXPRESSING WATER CONTENT
AND PARAMETER EXPRESSING CHANGE OF FACE VECTOR
embdiff_dod
0.45
0.4
0.35
0.3
0.25
0.2
0.15
0.1
0.05
0
PARAMETER Adiff
EXPRESSING WATER CONTENT
-0.08
-0.07
-0.06
-0.05
-0.04
-0.03
-0.02
-0.01
0
embdiff_dod
Adiff
0
50
100
150
200
250
300
ELAPSED DAYS

FIG.27-11

TRENDS OF PARAMETER EXPRESSING WATER CONTENT AND PARAMETER EXPRESSING FACE WIDTH

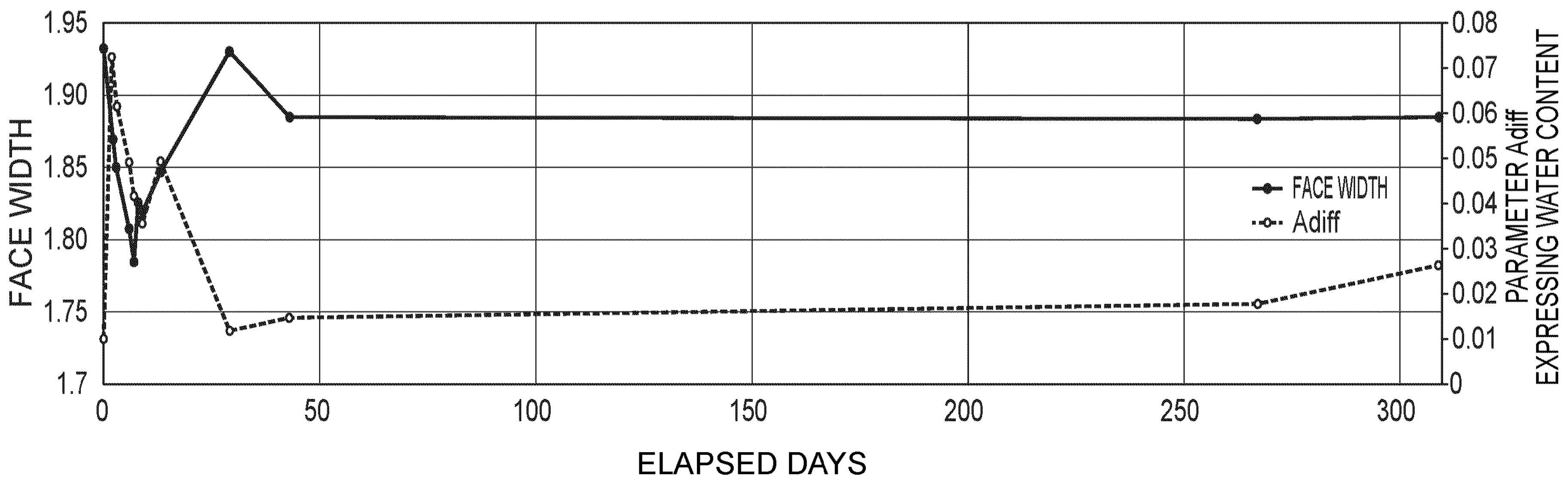

FIG.28

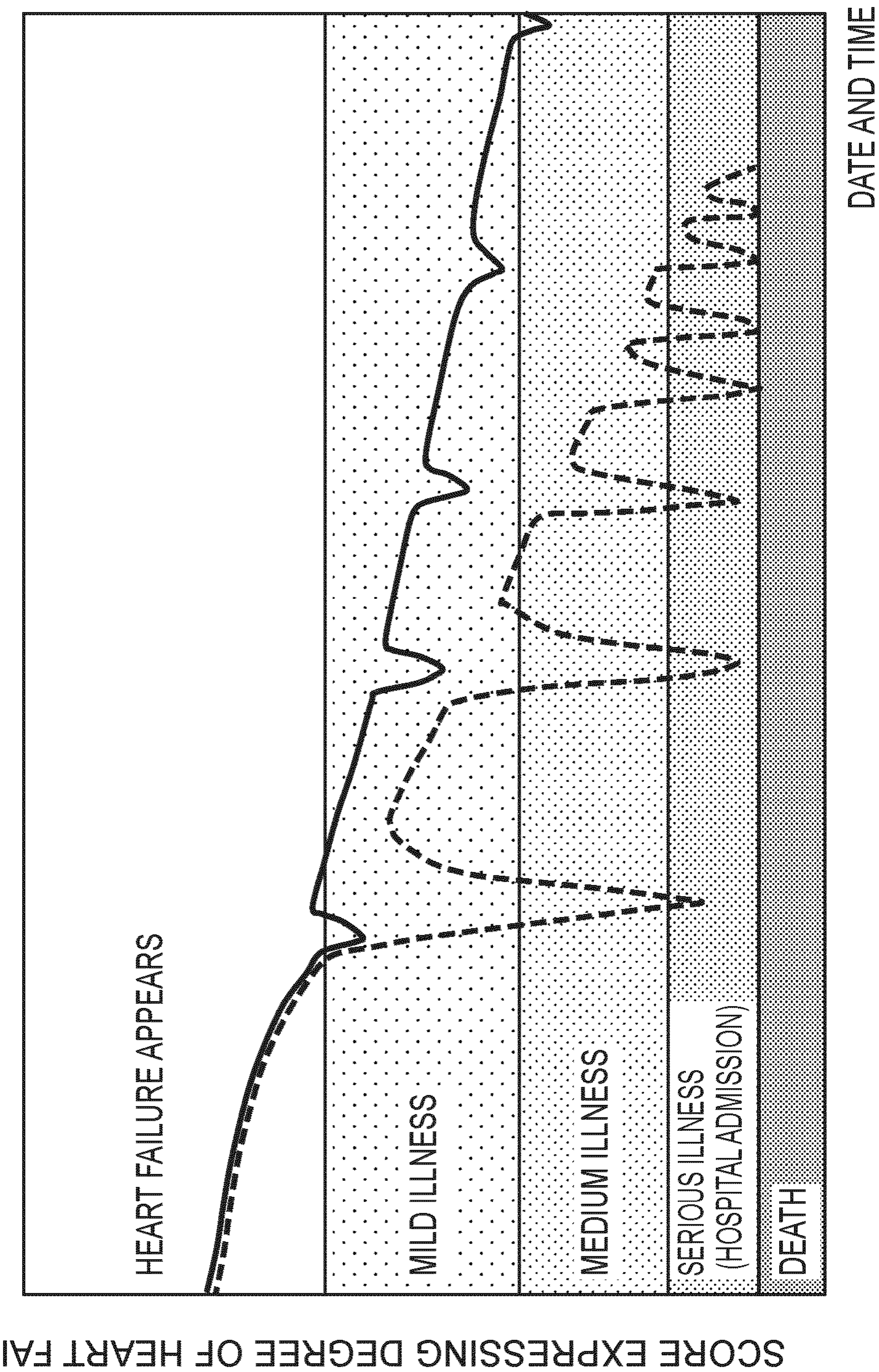
HEART FAILURE APPEARS
MILD ILLNESS
MEDIUM ILLNESS
SERIOUS ILLNESS
(HOSPITAL ADMISSION)
DEATH
DATE AND TIME
SCORE EXPRESSING DEGREE OF HEART FAILURE

## INTERNATIONAL SEARCH REPORT

International application No.
**PCT/JP2024/004673**

### A. CLASSIFICATION OF SUBJECT MATTER

***A61B 10/00***(2006.01)i; ***A61B 3/113***(2006.01)i; ***A61B 5/00***(2006.01)i; ***A61B 5/11***(2006.01)i; ***A61B 5/107***(2006.01)i; ***G06T 7/00***(2017.01)i; ***G16H 50/20***(2018.01)i
FI: A61B10/00 K; A61B3/113; A61B5/00 G; A61B5/00 101A; A61B5/107 110; A61B5/11 320; G06T7/00 612; G16H50/20

According to International Patent Classification (IPC) or to both national classification and IPC

### B. FIELDS SEARCHED

Minimum documentation searched (classification system followed by classification symbols)

A61B10/00; A61B3/113; A61B5/00; A61B5/11; A61B5/107; G06T7/00; G16H50/20

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | KR 10-2020-0099248 A (YKC TECH) 24 August 2020 (2020-08-24) paragraphs [0026]-[0050] | 1-4, 6, 9-10, 13, 15, 20-21 |
| Y | WO 2017/077629 A1 (FUJITSU LIMITED) 11 May 2017 (2017-05-11) paragraph [0013] | 1-4, 6, 9-10, 13, 15, 20-21 |
| Y | JP 2022-37153 A (CARDIO INTELLIGENCE INC.) 08 March 2022 (2022-03-08) paragraphs [0022]-[0045] | 1-4, 6, 9-10, 13, 15, 20-21 |
| A | US 2016/0317041 A1 (THE BOARD OF TRUSTEES OF THE UNIVERSITY OF ILLINOIS) 03 November 2016 (2016-11-03) entire text, all drawings | 1-21 |
| A | WO 2017/085895 A1 (FUJITSU LIMITED) 26 May 2017 (2017-05-26) entire text, all drawings | 1-21 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 April 2024** | **23 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/004673**

| Patent document cited in search report | Publication date (day/month/year) | Patent family member(s) | Publication date (day/month/year) |
|---|---|---|---|
| KR 10-2020-0099248 A | 24 August 2020 | (Family: none) | |
| WO 2017/077629 A1 | 11 May 2017 | EP 3372152 A1<br>paragraph [0014] | |
| JP 2022-37153 A | 08 March 2022 | US 2022/0346647 A1<br>paragraphs [0019]-[0039]<br>EP 4079224 A1<br>paragraphs [0022]-[0042] | |
| US 2016/0317041 A1 | 03 November 2016 | WO 2015/095760 A1<br>entire text, all drawings<br>CA 2934659 A1<br>entire text, all drawings | |
| WO 2017/085895 A1 | 26 May 2017 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5281002 B
- JP 6901042 B
- JP 6893002 B
- JP 2023019491 A